(19) Europäisches Patentamt
European Patent Office
Office européen des brevets

(11) EP 1 253 475 B1

(12) EUROPEAN PATENT SPECIFICATION

(45) Date of publication and mention of the grant of the patent:
**08.08.2007 Bulletin 2007/32**

(51) Int Cl.:
*G03G 9/087* (2006.01)

(21) Application number: **02009673.1**

(22) Date of filing: **29.04.2002**

(54) **Electrostatic charge image developing toner, producing method therefor, and image forming method and image forming apparatus utilizing the toner**

Elektrostatischer Ladungsaufzeichnungstoner, Herstellungsverfahren, Bilderzeugungsverfahren und Bildherstellungsapparat

Révélateur pour le développement d'images électrostatiques, procédé de fabrication, appareil de formation d'images et méthode de formation d'images l'utilisant

(84) Designated Contracting States:
**DE FR GB**

(30) Priority: **27.04.2001 JP 2001133728**
**10.07.2001 JP 2001210021**

(43) Date of publication of application:
**30.10.2002 Bulletin 2002/44**

(73) Proprietor: **CANON KABUSHIKI KAISHA**
**Ohta-ku, Tokyo (JP)**

(72) Inventors:
- **Yano, Tetsuya**
**Tokyo (JP)**
- **Nomoto, Tsuyoshi**
**Tokyo (JP)**
- **Kozaki, Shinya**
**Tokyo (JP)**
- **Honma, Tsutomu**
**Tokyo (JP)**

(74) Representative: **Weser, Wolfgang**
**Weser & Kollegen,**
**Patentanwälte,**
**Radeckestrasse 43**
**81245 München (DE)**

(56) References cited:
**US-A- 5 004 664**
**US-A- 6 146 665**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 253 475 B1

## Description

BACKGROUND OF THE INVENTION

Field of the Invention

The present invention relates to electrostatic charge image developing toner employed in electrophotography, electrostatic recording or the like, a producing method for such toner and an image forming method utilizing such toner.

Related Background Art

In the field of electrophotography, various methods are known but generally consist of utilizing a photoconductive material, forming an electrical latent image on an image bearing member (photosensitive member) by various means, developing such latent image with toner to obtain a visible image, then transferring if necessary the toner image onto a transfer material such as paper, and fixing the toner image onto the transfer material by heat and/or pressure thereby obtaining a copy. For rendering the electrical latent image visible, there are known a cascade development method, a magnetic brush development method, a pressure development method etc. There is also known a method of employing magnetic toner and a rotary developing sleeve having magnetic poles at the center thereof, thereby causing the magnetic toner to fly from the developing sleeve to the photosensitive member. In the development method for developing the electrostatic latent image, there is known a two-component development method employing two-component developer consisting of toner and carrier, and a one-component development method employing one-component developer consisting solely of toner and not employing carrier.

The colorant generally called toner (hereinafter colorant means a substance which contains a coloring agent as the essential component and may contain other components for providing other functions) contains binder resin and a coloring agent (hereinafter coloring agent means carbon black, pigment, dye or other coloring material itself) as the essential components and further contains magnetic powder or the like if necessary. For a monochromatic copying apparatus there is utilized not only black toner but also monochromatic color toner such as of red, blue, green or brown color, and, in such color toner there is utilized a pigment or a dye for developing respective color instead of the black coloring agent (principally carbon black) in the black toner.

In preparing color toner with such pigment or dye, there is widely employed a method kneading such pigment or dye with binder resin, then executing crushing with a mechanical or air collision crusher and executing classification to achieve desired average particle size and particle size distribution. However, such conventional method has been associated with drawbacks that the coloring agent etc. added in the toner may not be uniformly dispersed depending on the component, or that it may be difficult to produce the toner of a small particle size with a narrow particle size distribution whereby such toner of the conventional method may generate image fog because of the significant loss of charge in certain cases, and that the particle size distribution may become wider to result in selective development thereby leading to deterioration in the image.

Also, since the chargeability and the powder flowability are different for each coloring agent, it is generally conducted to adjust the kind and amount of the internally added charge control agent or the externally added materials, in order to obtain uniform characteristics among the color toners. Also in a full-color copying apparatus, there are employed color toners of magenta, cyan and yellow colors in addition to the black color toner, and, also in this case, there is executed adjustment on the kind and amount of the internally added charge control agent or the externally added material as explained above, in order to obtain uniform characteristics among the color toners. However, such adjustment of the kind and amount of the internally or externally added material as explained in the foregoing for obtaining uniform characteristics in the color toners may practically be quite cumbersome and difficult, so that, both for monochromatic toner and full-color toner, there have been drawbacks of fluctuation in the quality of the copy image or difference in behavior of deterioration among the color toners.

Also in recent years, in the image formation with the full-color electrophotography, though the image quality of the full-color copying apparatus has been improved by the expansion of color presenting area by the improvement in the pigment dispersing technology and by the higher resolution of the image based on the toner of smaller average particle size of 7 to 8 $\mu$m, the electrophotographic image is still deficient in the uniformity of image luster, uniformity or image height and reproducibility of halftone image area in comparison with the offset printed image.

In order to resolve the aforementioned unevenness of the image, there is being investigated reduction of the toner amount loaded on the transfer medium based on a further reduction of the diameter of the toner. However, in order to reproduce a density same as that of the printed image with the toner of smaller diameter, it becomes necessary to increase the concentration of the coloring agent to be added in the toner, but an increase in the concentration of the coloring agent in the toner renders it difficult to control the charge amount of the toner, whereby the fluctuation in the charge amount may increase particularly under a high temperature-high humidity or low temperature-low humidity

condition or in certain coloring agent, resulting in deterioration of the image quality. Also since the coloring agent enhances the influence on the fusing characteristics of the binder resin, there may result difficulty in the fixing ability such as generation of hot offset phenomenon or decrease of image intensity.

In order to alleviate the aforementioned influence of the coloring agent on the characteristics of toner, the Japanese Patent Application Laid-open Nos. 62-17753, 5-88406, 5-289396 and 5-341574 propose, in toner utilizing resin having surface activating effect as an auxiliary dispersant and having a matrix-domain structure formed by fused phase separation of the resins of different compositions, to disperse the coloring agent only in the domain portion, and the Japanese Patent Application Laid-open No. 63-305367 proposes to cover a nucleus particle, prepared by suspension polymerization and containing the coloring agent in a large amount, with resin of a high electrical resistance not containing the coloring agent by two-step polymerization, thereby controlling the electrical resistance of the toner surface, thus balancing the color developing property and the electrical characteristics.

These proposals can control the dispersion area of the coloring agent in the toner to a certain extent and can therefore relax the influence of the coloring agent on the toner characteristics. However, for achieving phase separation of the resins in the former method, the selection range of the resins is limited because the resin to be used in the matrix and that to be used in the domain have to be mutually unmixable, so that there will result drawbacks that the particle size and distribution of the domain is difficult to control and that it is difficult to include the coloring agent only in the domain in case the concentration of the coloring agent is high or in certain types of the coloring agent.

On the other hand, in the latter method, the toner is prepared by preparing the nucleus particle containing the coloring agent in a large amount by suspension polymerization and then by adding a monomer, but, since the nucleus particle and the monomer constituting the covering resin are of similar types and are mutually well soluble, the coloring agent inevitably migrates to the toner surface to result in deterioration of the toner chargeability and the fixing property. Also in the suspension polymerization, since the concentration of the coloring agent that can be introduced into the nucleus particle is limited, it is difficult to attain a high concentration of the coloring agent in the toner and it is not possible to reduce the size of the nucleus particle. Also in the nucleus particle produced by the suspension polymerization, since the polymerization initiator or the surfactant such as the suspension stabilizer used in a large amount remain in the capsule, the type or amount of the surfactant or the polymerization initiator are limited for certain applications and it may become difficult to attain the desired objective.

Also in the aforementioned producing methods, organic solvent is often used for monomer polymerization or for polymer dissolution, so that it is difficult to use the coloring agent soluble in the organic solvent. Also in case the organic solvent is used in a large amount for mass production, there results a large burden on the facility, human body and environment, such methods are not preferred also in such aspect. Further, these methods are associated with a drawback that the reaction conditions are not easily controllable and that the process steps are also complicated.

On the other hand, bioengineering methods for producing polymer compounds are actively investigated in recent years and are partially employed commercially. As examples of microorganism-origin polymer compounds, there are known polyhydroxyalkanoates (which may hereinafter be abbreviated as PHA) such as poly-3-hydroxy-n-butyric acid (which may hereinafter be abbreviated as PHB) or a copolymer of 3-hydroxy-n-butyric acid and 3-hydroxy-n-valeric acid (which may hereinafter be abbreviated as PHB/V), polysaccharides such as bacteria cellulose or purulan, and polyamino acids such as poly-γ-glutamic acid or polylysin. Such PHA produced by the microorganisms can be utilized for producing various products for example by fusion, like the conventional plastics. It also shows satisfactory matching with the living tissues and is expected in the applications as the soft material for medical use.

It has been reported that various microorganisms produce and accumulate PHA. For example, the production of PHB/V by the microorganisms of Alcaligenes eutropus H16 (ATCC No. 17699), Methylobacterium sp., Paracoccus sp., Alcaligenes sp. and Pseudomonas sp. is reported (Japanese Patent Application Laid-open No. 5-74492, Japanese Patent Publications Nos. 6-15604, 7-14352 and 8-19227 etc.).

It is also disclosed that Comamonas acidovorans IFO 13852) produce PHA having monomer units including 3-hydroxy-n-butyric acid and 4-hydroxy-n-butyric acid (Japanese Patent Application Laid-open No. 9-191893). It is also disclosed that Aeromonas caviae produce copolymer of 3-hydroxy-n-butyric acid and 3-hydroxyhexanoic acid (Japanese Patent Application Laid-open Nos. 5-93049 and 7-265065.

The biosynthesis of such PHB or PHB/V is executed by a polymerization reaction by an enzyme utilizing, as the substrate, (R)-3-hydroxybutyryl CoA or (R)-3-hydroxyvaleryl CoA produced from various carbon sources through various metabolism paths in the microorganisms. The enzyme catalyzing such polymerization reaction is PHB synthesizing enzyme (also called PHB polymerase or PHB synthase). CoA means coenzyme A having the following chemical structure:

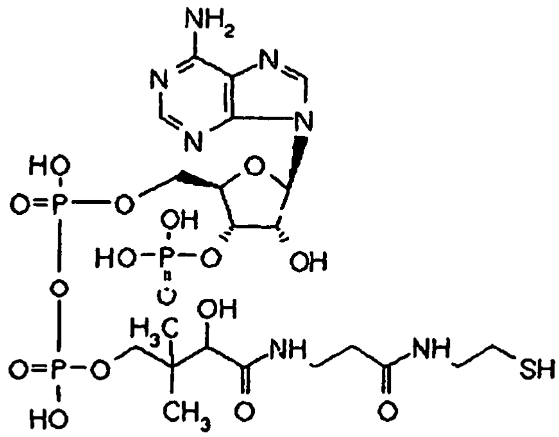

Also in recent years, investigations are actively executed on polyhydroxyalkanoate consisting of 3-hydroxy alkanoic acid unit of a medium-chain-length with 3 to 12 carbon atoms (which may be hereinafter abbreviated as mcl-PHA).

For example, Japanese Patent No. 2642937 discloses that Pseudomonas oleovorans ATCC 29347 supplied with acyclic aliphatic hydrocarbon produces PHA including 3-hydroxy alkanoic acid monomer unit with 6 to 12 carbon atoms. Also, Appl. Environ. Microbiol., 58, 746(1992) reports that Pseudomonas resinovorans produces PHA including 3-hydroxy-n-butyric acid, 3-hydroxy-hexanoic acid, 3-hydroxy-octanoic acid and 3-hydroxy-decanoic acid as monomer units from octanoic acid as the single carbon source, and PHA including 3-hydroxy-n-butyric acid, 3-hydroxy-hexanoic acid, 3-hydroxy-octanoic acid and 3-hydroxy-decanoic acid as monomer units from hexanoic acid as the single carbon source. In these syntheses, the introduction of a 3-hydroxyalkanoic acid unit having a chain length longer than that of the starting fatty acid is assumed to result from a fatty acid synthesis path to be explained later.

Int. J. Biol. Macromol., 16(3), 119(1994) reports that Pseudomonas sp. strain 61-3 produces, from sodium gluconate as a single carbon source, PHA including, as units thereof, 3-hydroxyalkanoic acids such as 3-hydroxy-n-butyric acid, 3-hydroxyhexanoic acid, 3-hydroxyoctanoic acid, 3-hydroxy decanoic acid and 3-hydroxydodecanoic acid, and 3-hydroxyalkenoic acids such as 3-hydroxy-5-cis-decenoic acid and 3-hydroxy-5-cis-dodecenoic acid.

The aforementioned PHA is a PHA consisting of a monomer unit having an alkyl radical in the side chain (which may hereinafter be abbreviated as usual-PHA). However, for wider applications, for example for application as functional polymer, PHA having a substituent other than alkyl radical, such as phenyl radical, unsaturated hydrocarbon radical, ester radical, allyl radical, cyano radical, halogenated hydrocarbon, epoxide etc., in the side chain (such PHA may hereinafter be abbreviated as unusual-PHA) is anticipated to be extremely useful.

As an example of biosynthesis of unusual-PHA containing phenyl radical, Macromolecules, 24, 5256-5260 (1991), Macromol. Chem., 191, 1957-1965(1990) and Chirality, 3, 492-494(1991) report that Pseudomonas oleovorans produces PHA containing 3-hydroxy-5-phenylvaleric acid as a unit, from 5-phenylvaleric acid. Also Macromolecules, 29, 1762-1766(1996) reports that Pseudomonas oleovorans produces PHA containing 3-hydroxy-5-(4-tolyl)valeric acid as a unit, from 5-(4-tolyl)-valeric acid (i.e. 5-(4-methylphenyl)valeric acid). Also Macromolecules, 32, 2889-2895(1999) reports that Pseudomonas oleovorans produces PHA containing 3-hydroxy-5-(2,4-dinitrophenyl)valeric acid and 3-hydroxy-5-(4-nitrophenyl)valeric acid as units, from 5-(2,4-dinitrophenyl)valeric acid.

Also as examples of unusual-PHA containing phenoxy radical, Macromol. Chem. Phys., 195, 1665-1672(1994) reports that Pseudomonas oleovorans produces PHA containing 3-hydroxy-5-hydroxyvaleric acid and 3-hydroxy-9-phenoxynonanoic acid as the units, from 11-phenoxyundecanoic acid. Also Macromolecules, 29, 3432-3435(1996) reports that Pseudomonas oleovolans produces PHA including 3-hydroxy-4-phenoxybutyric acid unit and 3-hydroxy-6-phenoxyhexanoic acid unit from 6-phenoxyhexanoic acid, also produces PHA including 3-hydroxy-4-phenoxybutyric acid unit, 3-hydroxy-6-phenoxyhexanoic acid unit and 3-hydroxy-8-phenoxyoctanoic acid unit from 8-phenoxyoctanoic acid, and produces PHA including 3-hydroxy-5-phenoxyvaleric acid unit and 3-hydroxy-7-phenoxyheptanoic acid unit from 11-phenoxyundecanoic acid.

Also Can. J. Microbiol., 41, 32-43(1995) reports production of PHA containing 3-hydroxy-6-(p-cyanophenoxy) hexanoic acid or 3-hydroxy-6-(p-nitrophenoxy) hexanoic acid as the monomer unit by Pseudomonas oleovorans ATCC 29347 strain and Pseudomonas putida KT2442 strain, from p-cyanophenoxy hexanoic acid or p-nitrophenoxy hexanoic acid. The Japanese Patent No. 2989175 discloses homopolymer consisting of 3-hydroxy-5-(monofluorophenoxy) valeric acid unit or 3-hydroxy-5-(difluorophenoxy) valeric acid unit, copolymer including at least 3-hydroxy-5-(monofluorophenoxy) pentanoate unit or 3-hydroxy-5-(difluorophenoxy) pentanoate unit and a producing method therefor, and describes the advantages thereof such as providing stereo regularity and water repellency while maintaining high melting point

and satisfactory workability.

Also as examples of unusual-PHA having cyclohexyl radical, Macromolecules, 30, 1611-1615(1997) reports that Pseudomonas oleovolans produces such PHA from cyclohexylbutyric acid or cyclohexylvaleric acid.

The biosynthesis of such mcl-PHA or unusual-PHA is executed by a polymerization reaction by an enzyme utilizing, as the substrate, (R)-3-hydroxyacyl CoA produced from various alkanoic acid sources through various metabolism paths (β-oxidation system or fatty acid synthesis path) in the microorganisms. The enzyme catalyzing such polymerization reaction is PHA synthesizing enzyme (also called PHA polymerase or PHA synthase). In the following there are shown reactions from alkanoic acid to PHA through the polymerization reaction by β-oxidation system and PHA synthesizing enzyme.

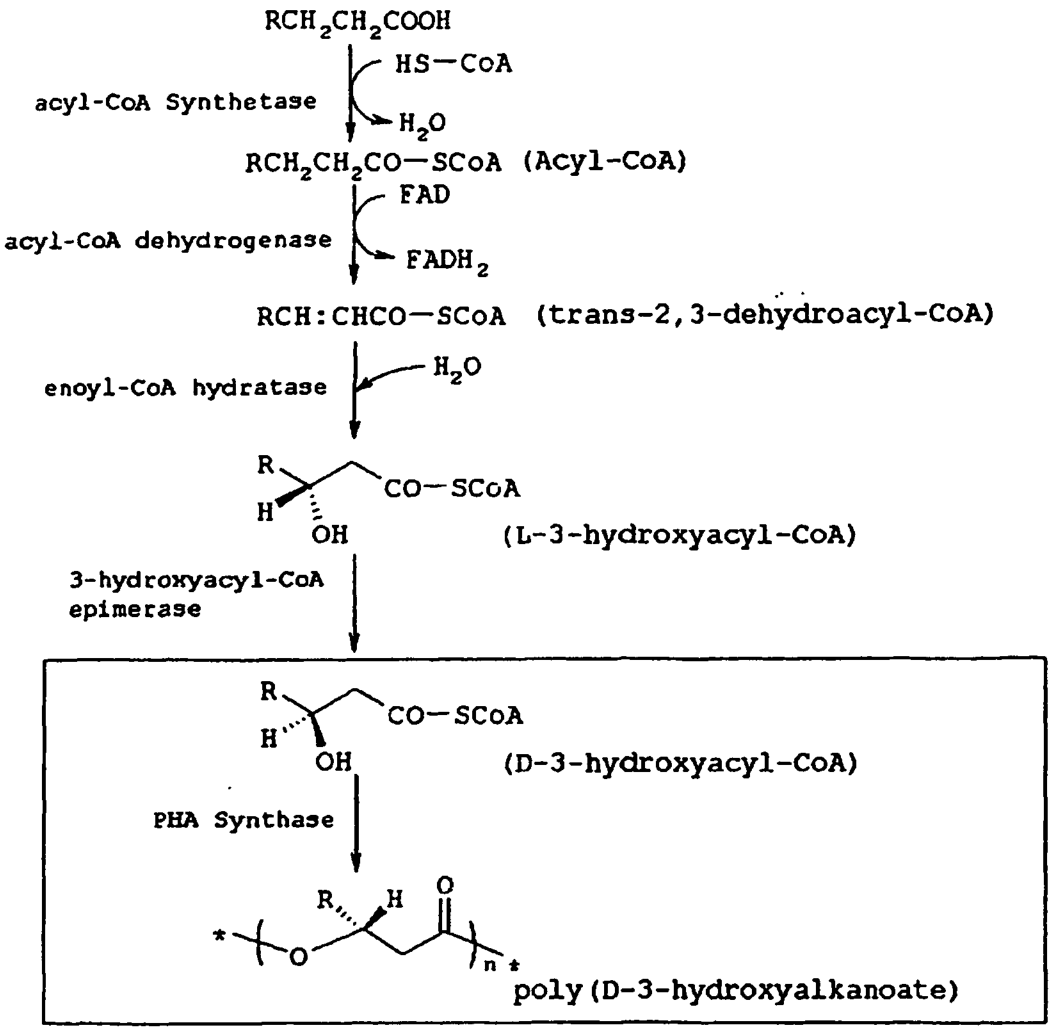


On the other hand, in case through the fatty acid synthesis path, PHA is assumed to be synthesized similarly by the PHA synthesizing enzyme, from (R)-3-hydroxyacyl CoA converted from (R)-3-hydroxyacyl-ACP (ACP means acyl carrier protein) generated in such path as the substrate.

Recently, it is being tried to take out the aforementioned PHB synthesizing enzyme or PHA synthesizing enzyme from the bacteria and to synthesize PHA in cell-free system (in vitro).

For example, Proc. Natl. Acad. Sci. USA, 92, 6279-6283(1995) reports reacting 3-hydroxybutyryl CoA on PHB synthesizing enzyme derived from Alcaligenes eutrophus to produce PHB consisting of 3-hydroxy-n-butyric acid unit. Also Int. J. Biol. Macromol., 25, 55-60(1999) reports reacting 3-hydroxybutyryl CoA or 3-hydroxyvaleryl CoA on PHB synthesizing enzyme derived from Alcaligenes eutrophus to produce PHA consisting of 3-hydroxy-n-butyric acid unit or 3-hydroxy-n-valeric acid unit. Also this report describes that reaction of racemic 3-hydroxy-butyryl CoA results in synthesis of PHB consisting solely of (R)-3-hydroxy-n-butyric acid unit by the stereo selectivity of the enzyme. Also Macromol. Rapid Commun., 21, 77-84(2000) reports in vitro PHB synthesis utilizing PHB synthesizing enzyme derived from Alcaligenes eutrophus.

Also FEMS Microbiol. Lett., 168, 319-324(1998) reports reacting 3-hydroxybutyryl CoA on PHB synthesizing enzyme derived from Chromatium vinosum to produce PHB consisting of 3-hydroxy-n-butyric acid unit.

Appl. Microbiol. Biotechnol., 54, 37-43(2000) reports synthesis of PHA consisting of 3-hydroxy-decanoic acid unit by reacting 3-hydroxydecanoyl CoA on PHA synthesizing enzyme of Pseudomonas aeruginosa.

US 5,004,664 discloses a toner composition comprising semicrystalline polyester resin particles, especially polyhydroxyalkanoates, and pigment particles.

## SUMMARY OF THE INVENTION

In the conventional electrostatic charge image developing toners, as explained in the foregoing, it has often been difficult to provide the electrostatic charge image developing toners having uniform toner characteristics such as chargeability, flowability, stability in time, environmental stability etc. among the toners of different colors including black color. Also in the full-color electrostatic charge image developing toner of small diameter, there may result deterioration of the charging characteristics, powder characteristics, charge amount maintainability and fixing characteristics with an increase in the concentration of the coloring agent.

Also there has been desired electrostatic charge image developing toner of a small particle size, containing the coloring agent dispersed uniformly and finely, excellent in color saturation and transparency, uniformity of charging and durability. Also there has been desired such electrostatic charge image developing toner having low burden on the environment or organisms, low limitation on the material of the coloring agent, and including a capsule structure (colorant) containing the coloring agent at a high concentration and free from contamination of surfactant or polymerization initiator which has been the contamination sources of the conventional capsule structure.

The object of the present invention is to provide electrostatic charge image developing toner capable of resolving the aforementioned drawbacks, a method for producing such electrostatic charge image developing toner, and an image forming method and an image forming apparatus utilizing such electrostatic charge image developing toner.

As a result of intensive investigation for attaining the above-mentioned object, the present inventors have found that the coloring agent can be easily included in minute microcapsules without employing surfactant, by fixing a polyhydroxyalkanoate (hereinafter abbreviated PHA) synthesizing enzyme on the coloring agent and executing reaction by adding 3-hydroxyacyl coenzyme A thereto, also that the coloring agent is included at a high density because PHA directly covers the surface of the coloring agent, and that the mutual solubility of PHA, constituting the covering of the coloring agent, with the binder resin by suitably selecting the type of 3-hydroxyacyl coenzyme A.

It is also found that a structured material improved in various characteristics (particularly control of the aforementioned mutual solubility) by applying chemical modification to such PHA. More specifically, it is found that, by introducing a graft chain to such PHA, there can be obtained colorant in which at least a part of the coloring agent is covered by PHA having various characteristics derived from such graft chain. It is also found that, by crosslinking such PHA, there can be obtained colorant in which at least a part of the coloring agent is covered with PHA provided with the desired physicochemical properties (such as mechanical strength, chemical resistance, heat resistance etc.). In the present invention, chemical modification means a modification of the molecular structure of polymer material by a chemical intramolecular or intermolecular reaction of such polymer material, or by a chemical reaction of the polymer material and another chemical substance. Also crosslinking means intramolecular or intermolecular chemical or physicochemical coupling of the polymer material to form a network structure, and a crosslinking agent means a substance having certain reactivity with the polymer material to be added for forming the aforementioned crosslinking reaction.

It is also found that, by the aforementioned characteristics, the toner characteristics can be made uniform among the toners of different colors, that the high concentration of the coloring agent can be easily attained without deteriorating other functions of the toner, and that the coloring agent can be uniformly and finely dispersed in the toner particle, whereby the present invention is attained.

The electrostatic charge image developing toner of the present invention is featured in that the electrostatic charge image developing toner is at least composed of a colorant at least a part of which is covered with polyhydroxyalkanoate constituting a first resin component, and binder resin constituting a second resin component.

More specifically, the present invention provides, in the electrostatic charge image developer requiring toner of several colors, electrostatic charge image developing toner allowing uniform design of toner characteristics such as chargeability, flowability, stability in time, environmental stability etc. among the toner of different colors including black color. It also provides full-color electrostatic charge image developing toner of small particle size, capable of resolving deterioration in the charging characteristics, powder characteristics, maintenance of charge amount and fixing characteristics resulting from the increase in the concentration of the coloring agent in such toner. It also provides electrostatic charge image developing toner of a small particle size including a coloring agent dispersed uniformly and finely, being excellent in color saturation, transparency, charge uniformity and durability. Also the present invention allows to provide electrostatic charge image developing toner having low burden on the environment or organisms, low limitation on the

material of the coloring agent, and including a capsule structure (colorant) containing the coloring agent at a high concentration and free from contamination of surfactant or polymerization initiator which has been the contamination sources of the conventional capsule structure, by utilizing PHA having a variety of functions.

In the electrostatic charge image developing toner of the present invention, the colorant preferably contains a pigment. Also polyhydroxyalkanoate preferably includes at least one selected from the group consisting of monomer units represented by the following formulas (1) to (10):

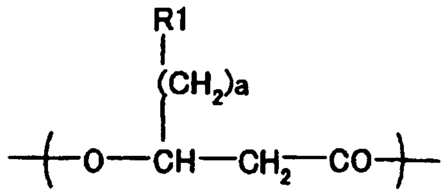

[1]

wherein the monomer unit is at least one selected from the group consisting of monomer units in which the combination of R1 and a is any of the following:

- a monomer unit in which R1 is a hydrogen atom (H) and a is an integer from 0 to 10;
- a monomer unit in which R1 is a halogen atom and a is an integer from 1 to 10;
- a monomer unit in which R1 is a chromophore and a is an integer from 1 to 10;
- a monomer unit in which R1 is a carboxyl group or a salt thereof and a is an integer from 1 to 10; and
- a monomer unit in which R1 is a group represented by the following formula:

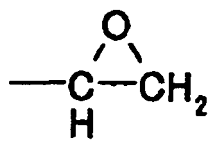

and a is an integer from 1 to 7;

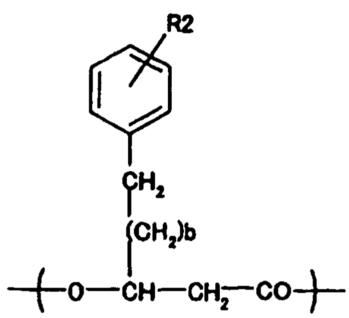

[2]

wherein b is an integer from 0 to 7, and R2 is a substitution selected from the group consisting of a hydrogen atom (H), a halogen atom, -CN, $-NO_2$, $-CF_3$,- $C_2F_5$ and $-C_3F_7$;

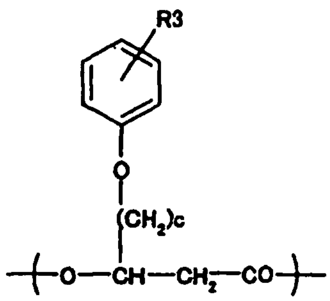

[3]

wherein c is an integer from 1 to 8, and R3 is a substitution selected from the group consisting of a hydrogen atom (H), a halogen atom, -CN, $-NO_2$, $-CF_3$, $-C_2F_5$ and $-C_3F_7$;

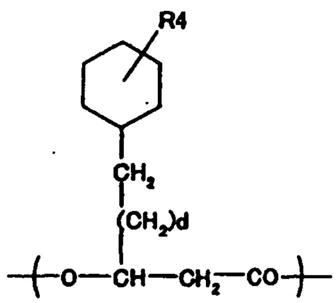

[4]

wherein d is an integer from 0 to 7, and R4 is a substitution selected from the group consisting of a hydrogen atom (H), a halogen atom, -CN, $-NO_2$, $-CF_3$, $-C_2F_5$ and $-C_3F_7$;

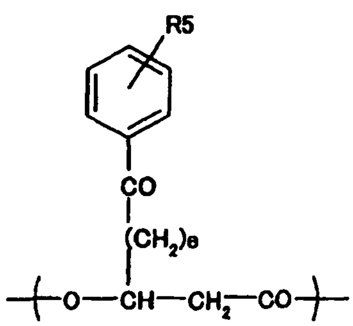

[5]

wherein e is an integer from 1 to 8, and R5 is a substitution selected from the group consisting of a hydrogen atom (H), a halogen atom, -CN, $-NO_2$, $-CF_3$, $-C_2F_5$, $-C_3F_7$, $-CH_3$, $-C_2H_5$ and $-C_3H_7$;

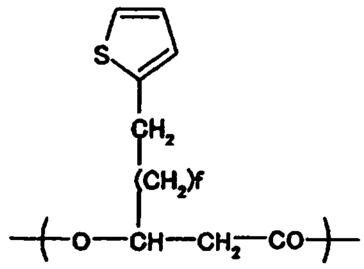

[6]

wherein f is an integer from 0 to 7;

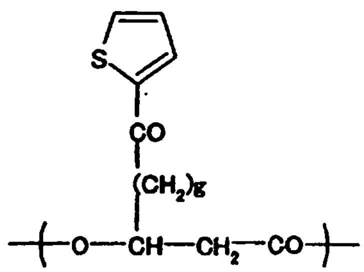

[7]

wherein g is an integer from 1 to 8;

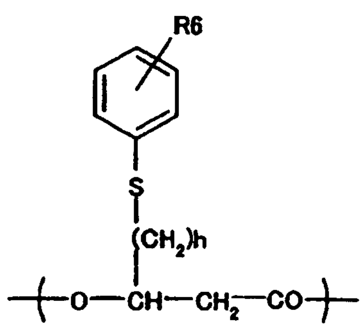

[8]

wherein h is an integer from 1 to 7, R6 is a substitution selected from the group consisting of a hydrogen atom (H), a halogen atom, -CN, $-NO_2$, -COOR', $-SO_2R''$, $-CH_3$, $-C_2H_5$, $-C_3H_7$, $-CH(CH_3)_2$ and $-C(CH_3)_3$, R' is a hydrogen atom, Na, K, $-CH_3$ or $-C_2H_5$, and R'' is -OH, -ONa, -OK, a halogen atom, $-OCH_3$ or $-OC_2H_5$;

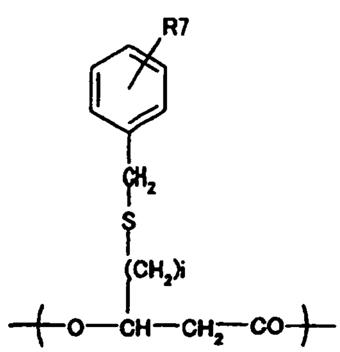

[9]

wherein i is an integer from 1 to 7, R7 is a substitution selected from the group consisting of a hydrogen atom (H), a halogen atom, -CN, $-NO_2$, -COOR' and $-SO_2R''$, R' is a hydrogen atom, Na, K, $-CH_3$ or $-C_2H_5$, and R'' is -OH, -ONa, -OK, a halogen atom, $-OCH_3$ or $-OC_2H_5$; and

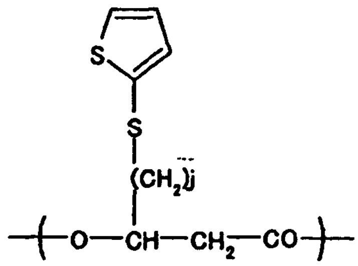

[10]

wherein j is an integer from 1 to 9.

Also polyhydroxyalkanoate preferably has a number-averaged molecular weight within a range from 1,000 to 10,000,000.

Also it is preferred that the monomer unit composition of polyhydroxyalkanoate changes in a direction from the inside to the outside of the colorant.

Also it is preferred that at least a part of polyhydroxyalkanoate is chemically modified polyhydroxyalkanoate, and that the chemically modified polyhydroxyalkanoate includes at least a graft chain. Also the graft chain is preferably a graft chain formed by chemical modification of polyhydroxyalkanoate at least including a monomer unit having an epoxy group, or a graft chain of a compound having an amino group.

The compound having amino group is preferably a compound modified at an end with amino group (referred to as terminal amino-modified compound). The terminal amino-modified compound is preferably at least one selected from the group consisting of polyvinylamine, polyethyleneimine, and polysiloxane modified at the end with amino group. Also at least a part of polyhydroxyalkanoate is preferably crosslinked polyhydroxyalkanoate.

It is also preferred that the crosslinked polyhydroxyalkanoate is obtained by crosslinking polyhydroxyalkanoate at least including a monomer unit having an epoxy group.

The crosslinked polyhydroxyalkanoate is preferably polyhydroxyalkanoate crosslinked by at least one selected from the group consisting of diamine compounds, succinic anhydride, 2-ethyl-4-methylimidazole and electron beam irradiation. In such case, the diamine compound is preferably hexamethylene diamine.

The present invention also provides an image forming method at least including a step of externally applying a voltage to a charging member thereby charging an electrostatic latent image bearing member, a step of forming an electrostatic charge image on the charged electrostatic latent image bearing member, a development step of developing the electrostatic charge image with electrostatic charge image developing toner thereby forming a toner image on the electrostatic latent image bearing member, a transfer step of transferring the toner image on the electrostatic latent image bearing member onto a recording material, and a fixation step of heat fixing the toner image on the recording material, the method being featured by using the aforementioned electrostatic charge image developing toner.

Another embodiment of the image forming method of the present invention at least includes a step of externally applying a voltage to a charging member thereby charging an electrostatic latent image bearing member, a step of forming an electrostatic charge image on the charged electrostatic latent image bearing member, a development step of developing the electrostatic charge image with electrostatic charge image developing toner thereby forming a toner image on the electrostatic latent image bearing member, a first transfer step of transferring the toner image on the electrostatic latent image bearing member onto an intermediate transfer member, a second transfer step of transferring the toner image on the intermediate transfer member onto a recording material, and a fixation step of heat fixing the toner image on the recording material, the method being featured by using the aforementioned electrostatic charge image developing toner.

The present invention further provides an image forming apparatus at least including means for externally applying a voltage to a charging member thereby charging an electrostatic latent image bearing member, means for forming an electrostatic charge image on the charged electrostatic latent image bearing member, development means for developing the electrostatic charge image with electrostatic charge image developing toner thereby forming a toner image on the electrostatic latent image bearing member, transfer means for transferring the toner image on the electrostatic latent image bearing member onto a recording material, and fixation means for heat fixing the toner image on the recording material, the apparatus being featured by containing the aforementioned electrostatic charge image developing toner.

Another embodiment of the image forming apparatus of the present invention at least includes means for externally applying a voltage to a charging member thereby charging an electrostatic latent image bearing member, means for forming an electrostatic charge image on the charged electrostatic latent image bearing member, development means for developing the electrostatic charge image with electrostatic charge image developing toner thereby forming a toner image on the electrostatic latent image bearing member, first transfer means for transferring the toner image on the electrostatic latent image bearing member onto an intermediate transfer member, second transfer means for transferring the toner image on the intermediate transfer member onto a recording material, and fixation means for heat fixing the toner image on the recording material, the apparatus being featured by containing the aforementioned electrostatic charge image developing toner.

The present invention further provides a method for producing electrostatic charge image developing toner including a colorant obtained by covering at least a part of the surface of a coloring agent with polyhydroxyalkanoate constitute a first resin component, the method being featured by executing a polyhydroxyalkanoate synthesizing reaction utilizing 3-hydroxyacyl CoA as the substrate in the presence of polyhydroxyalkanoate synthesizing enzyme fixed on the surface of the coloring agent dispersed in aqueous medium to cover at least a part of the surface of the coloring agent with polyhydroxyalkanoate thereby producing the aforementioned colorant.

In the aforementioned methods, polyhydroxyalkanoate preferably includes at least one selected from the group consisting of monomer units represented by the following formulas (1) to (10), and the respectively corresponding 3-hydroxyacyl coenzyme A is any of those represented by the chemical formulas (11) to (20):

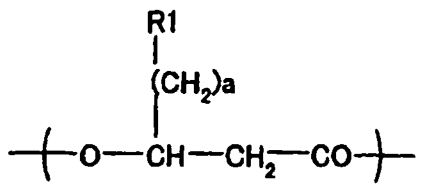

[1]

wherein the monomer unit is at least one selected from the group consisting of monomer units in which the combination of R1 and a is any of the following:

a monomer unit in which R1 is a hydrogen atom (H) and a is an integer from 0 to 10;
a monomer unit in which R1 is a halogen atom and a is an integer from 1 to 10;
a monomer unit in which R1 is a chromophore and a is an integer from 1 to 10;
a monomer unit in which R1 is a carboxyl group or a salt thereof and a is an integer from 1 to 10; and
a monomer unit in which R1 is a group represented by the following formula:

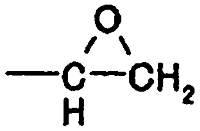

and a is an integer from 1 to 7;

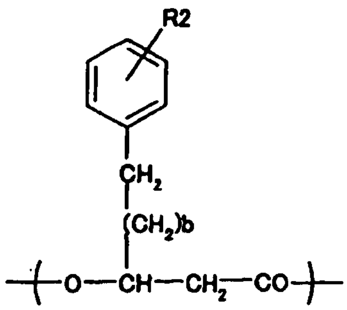

[2]

wherein b is an integer from 0 to 7, and R2 is a substitution selected from the group consisting of a hydrogen atom (H), a halogen atom, -CN, $-NO_2$, $-CF_3$,- $C_2F_5$ and $-C_3F_7$;

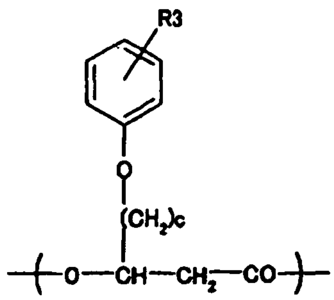

[3]

wherein c is an integer from 1 to 8, and R3 is a substitution selected from the group consisting of a hydrogen atom (H), a halogen atom, -CN, $-NO_2$, $-CF_3$, $-C_2F_5$ and $-C_3F_7$;

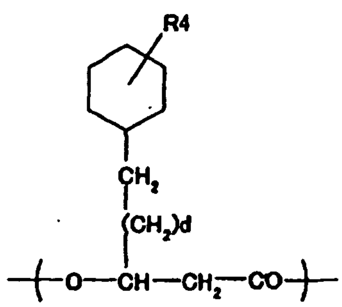

[4]

wherein d is an integer from 0 to 7, and R4 is a substitution selected from the group consisting of a hydrogen atom (H), a halogen atom, -CN, $-NO_2$, $-CF_3$, $-C_2F_5$ and $-C_3F_7$;

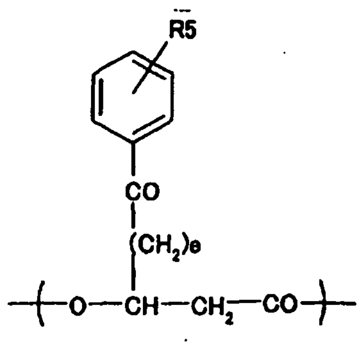

[5]

wherein e is an integer from 1 to 8, and R5 is a substitution selected from the group consisting of a hydrogen atom (H), a halogen atom, -CN, $-NO_2$, $-CF_3$, $-C_2F_5$, $-C_3F_7$, $-CH_3$, $-C_2H_5$ and $-C_3H_7$;

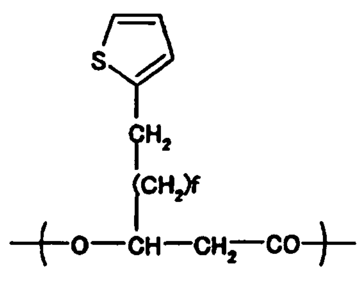

[6]

wherein f is an integer from 0 to 7;

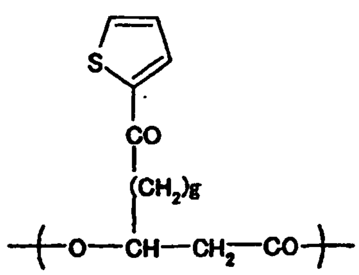

[7]

wherein g is an integer from 1 to 8;

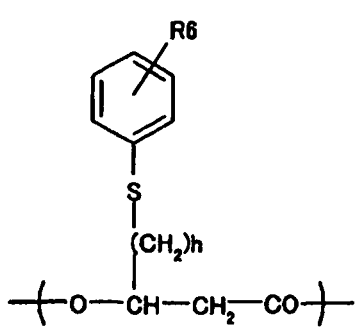

[8]

wherein h is an integer from 1 to 7, R6 is a substitution selected from the group consisting of a hydrogen atom (H),

a halogen atom, -CN, -$NO_2$, -COOR', -$SO_2$R", -$CH_3$, -$C_2H_5$, -$C_3H_7$, -CH$(CH_3)_2$ and -C$(CH_3)_3$, R' is a hydrogen atom, Na, K, -$CH_3$ or -$C_2H_5$, and R" is -OH, -ONa, -OK, a halogen atom, -$OCH_3$ or -$OC_2H_5$;

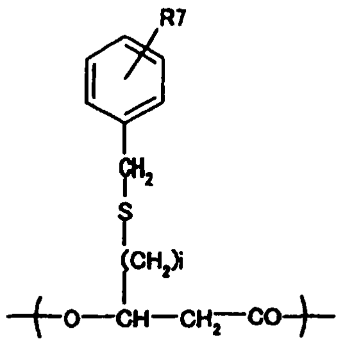

[9]

wherein i is an integer from 1 to 7, R7 is a substitution selected from the group consisting of a hydrogen atom (H), a halogen atom, -CN, -$NO_2$, -COOR' and -$SO_2$R", R' is a hydrogen atom, Na, K, -$CH_3$ or -$C_2H_5$, and R" is -OH, -ONa, -OK, a halogen atom, -$OCH_3$ or -$OC_2H_5$;

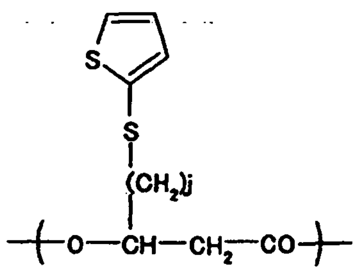

[10]

wherein j is an integer from 1 to 9;

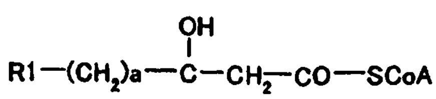

[11]

wherein -SCoA indicates coenzyme A bonded to alkanoic acid, and the combination of R1 and a is any of the following and corresponds to the combination of R1 and a in the foregoing chemical formula (1):

a monomer unit in which R1 is a hydrogen atom (H) and a is an integer from 0 to 10;
a monomer unit in which R1 is a halogen atom and a is an integer from 1 to 10;
a monomer unit in which R1 is a chromophore and a is an integer from 1 to 10;
a monomer unit in which R1 is a carboxyl group or a salt thereof and a is an integer from 1 to 10; and
a monomer unit in which R1 is a group represented by the following formula:

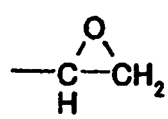

and a is an integer from 1 to 7;

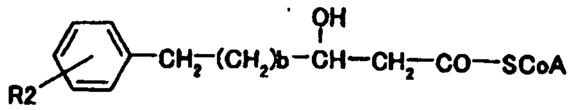

[12]

wherein -SCoA indicates coenzyme A bonded to alkanoic acid, b is an integer from 0 to 7 corresponding to b in the foregoing chemical formula (2), and R2 is a substitution selected from the group consisting of a hydrogen atom (H), a halogen atom, -CN, $-NO_2$, $-CF_3$, $-C_2F_5$ and $-C_3F_7$, corresponding to R2 in the foregoing chemical formula (2);

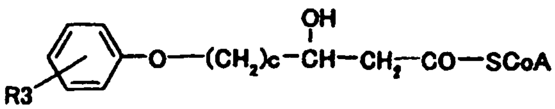

[13]

wherein -SCoA indicates coenzyme A bonded to alkanoic acid, c is an integer from 1 to 8 corresponding to c in the foregoing chemical formula (3), and R3 is a substitution selected from the group consisting of a hydrogen atom (H), a halogen atom, -CN, $-NO_2$, $-CF_3$, $-C_2F_5$ and $-C_3F_7$, corresponding to R3 in the foregoing chemical formula (3);

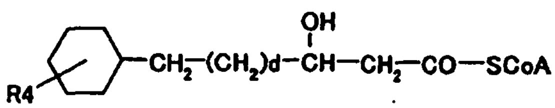

[14]

wherein -SCoA indicates coenzyme A bonded to alkanoic acid, d is an integer from 0 to 7 corresponding to d in the foregoing chemical formula (4), and R4 is a substitution selected from the group consisting of a hydrogen atom (H), a halogen atom, -CN, $-NO_2$, $-CF_3$, $-C_2F_5$ and $-C_3F_7$, corresponding to R4 in the foregoing chemical formula (4);

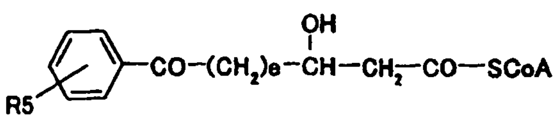

[15]

wherein -SCoA indicates coenzyme A bonded to alkanoic acid, e is an integer from 1 to 8 corresponding to e in the foregoing chemical formula (5), and R5 is a substitution selected from the group consisting of a hydrogen atom (H), a halogen atom, -CN, $-NO_2$, $-CF_3$, $-C_2F_5$, $-C_3F_7$, $-CH_3$, $-C_2H_5$ and $-C_3H_7$, corresponding to R5 in the foregoing chemical formula (5);

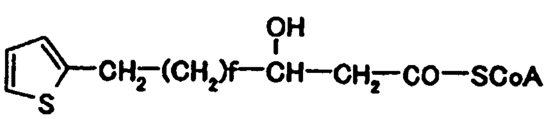

[16]

wherein -SCoA indicates coenzyme A bonded to alkanoic acid, and f is an integer from 0 to 7 corresponding to f in the foregoing chemical formula (6);

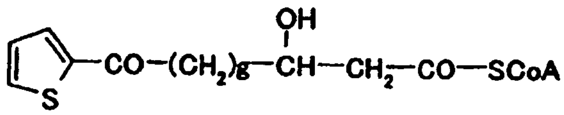

[17]

wherein -SCoA indicates coenzyme A bonded to alkanoic acid, and g is an integer from 1 to 8 corresponding to g in the foregoing chemical formula (7);

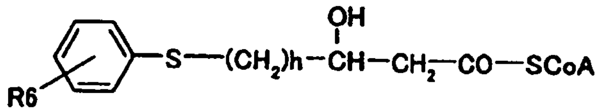

[18]

wherein -SCoA indicates coenzyme A bonded to alkanoic acid, h is an integer from 1 to 7 corresponding to h in the foregoing chemical formula (8), R6 is a substitution selected from the group consisting of a hydrogen atom (H), a halogen atom, -CN, $-NO_2$, -COOR',- $SO_2R''$, $-CH_3$, $-C_2H_5$, $-C_3H_7$, $-CH(CH_3)_2$ and $-C(CH_3)_3$ corresponding to R6 in the foregoing chemical formula (8), R' is a hydrogen atom, Na, K, $-CH_3$ or $-C_2H_5$, and R" is -OH, -ONa, -OK, a halogen atom, $-OCH_3$ or $-OC_2H_5$;

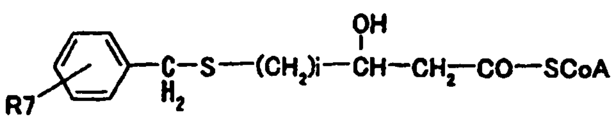

[19]

wherein -SCoA indicates coenzyme A bonded to alkanoic acid, i is an integer from 1 to 7 corresponding to i in the foregoing chemical formula (9), R7 is a substitution selected from the group consisting of a hydrogen atom (H), a halogen atom, -CN, $-NO_2$, -COOR' and $-SO_2R''$ corresponding to R7 in the foregoing chemical formula (9), R' is a hydrogen atom, Na, K, $-CH_3$ or $-C_2H_5$, and R" is -OH, -ONa, -OK, a halogen atom, $-OCH_3$ or $-OC_2H_5$; and

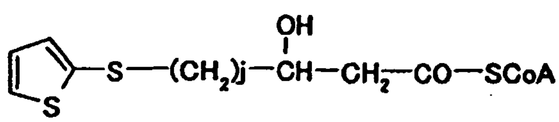

[20]

wherein -SCoA indicates coenzyme A bonded to alkanoic acid, and j is an integer of 1 to 9 corresponding to the foregoing chemical formula (10).

Also it is preferable to change the monomer unit composition of polyhydroxyalkanoate in a direction from the inside to the outside of the colorant, by changing in time the composition of 3-hydroxyacyl coenzyme A.

Also it is preferable to further have a step of applying chemical modification in at least a part of polyhydroxyalkanoate covering the colorant. The aforementioned step of applying chemical modification is preferably a step of adding a graft chain in at least a part of polyhydroxyalkanoate.

Also it is preferable that the aforementioned step of adding the graft chain is a step of reacting at least a part of polyhydroxyalkanoate with a compound having a reactive functional group at the end.

Also polyhydroxyalkanoate preferably includes at least a monomer unit having an epoxy group.

The aforementioned compound having a reactive functional group at the end is preferably a compound having an amino group, and the compound having amino group is preferably a terminal amino-modified compound. The terminal amino-modified compound is preferably at least one selected from the group consisting of polyvinylamine, polyethyleneimine, and polysiloxane modified at the end with amino group.

The aforementioned step of applying chemical modification is preferably a step of crosslinking at least a part

of polyhydroxyalkanoate, and the aforementioned crosslinking step is preferably a step of reacting at least a part of polyhydroxyalkanoate with a crosslinking agent.

The aforementioned polyhydroxyalkanoate is preferably polyhydroxyalkanoate at least including a monomer unit having an epoxy group.

The aforementioned crosslinking agent is preferably at least one selected from the group consisting of diamine compounds, succinic anhydride, and 2-ethyl-4-methylimidazole. In such case, the diamine compound is preferably hexamethylene diamine.

The aforementioned crosslinking step can be a step of irradiating polyhydroxyalkanoate with an electron beam.

The aforementioned polyhydroxyalkanoate synthesizing enzyme is preferably polyhydroxyalkanoate synthesizing enzyme produced by microorganism having ability of producing such enzyme, or a transformant obtained by introducing a gene relating to such producing ability into host microorganisms.

The microorganisms having the ability of producing the aforementioned polyhydroxyalkanoate synthesizing enzyme are preferably those of Pseudomonas sp. of at least one species selected from the group consisting of Pseudomonas putida P91 (FERM BP-7373), Pseudomonas cichorii H45 (FERM BP-7374), Pseudomonas cichorii YN2 (FERM BP-7375), and Pseudomonas jessenii P161 (FERM BP-7376).

The microorganisms having the ability of producing polyhydroxyalkanoate synthesizing enzyme can also be those of Burkholderia sp. of at least one species selected from the group consisting of Burkholderia cepacia KK01 (FERM BP-4235), Burkholderia sp. OK3 (FERM P-17370), and Burkholderia sp. OK4 (FERM P-17371).

The microorganisms having the ability of producing polyhydroxyalkanoate synthesizing enzyme can also be those of Alcaligenes sp. and can be Alcaligenes sp. TL2 (FERM BP-6913).

The microorganisms having the ability of producing polyhydroxyalkanoate synthesizing enzyme can also be those of Ralstonia sp. and can be Ralstonia eutropha TB64 (FERM BP-6933).

Also the host microorganisms for the transformant having the ability for producing the aforementioned polyhydroxyalkanoate synthesizing enzyme can be Escherichia coli.

The present invention enables to provide, in the electrostatic charge image developer requiring toner of several colors, electrostatic charge image developing toner composed of a colorant at least a part thereof is covered with polyhydroxyalkanoate constituting a first resin component, and binder resin constituting a second resin component, thereby allowing uniform design of toner characteristics such as chargeability, flowability, stability in time, environmental stability etc. among the toner of different colors including black color. It also allows to provide full-color electrostatic charge image developing toner of small particle size, capable of resolving deterioration in the charging characteristics, powder characteristics, maintenance of charge amount and fixing characteristics resulting from the increase in the concentration of the coloring agent in such toner.

It also allows to provide electrostatic charge image developing toner of a small particle size including a coloring agent dispersed uniformly and finely, being excellent in color saturation, transparency, charge uniformity and durability. Also the present invention allows to provide electrostatic charge image developing toner having low burden on the environment or organisms, low limitation on the material of the coloring agent, and including a capsule structure (colorant) containing the coloring agent at a high concentration and free from contamination of surfactant or polymerization initiator which has been the contamination sources of the conventional capsule structure.

In addition, the present invention allows to provide a method for producing the aforementioned electrostatic charge image developing toner, and an image forming method and an image forming apparatus utilizing the aforementioned electrostatic charge image developing toner.

The above and other objects, effects, features and advantages of the present invention will become more apparent from the following description of embodiments thereof taken in conjunction with the accompanying drawings.

## BRIEF DESCRIPTION OF THE DRAWINGS

Fig. 1 is a schematic view of an image forming apparatus;

Fig. 2 is a partial cross-sectional view of a developing device for two-component developer;

Fig. 3 is a schematic view of an image forming apparatus having a toner reuse mechanism;

Fig. 4 is a partial cross-sectional view of a developing device for one-component developer;

Fig. 5 is a partial exploded perspective view of a fixing device;

Fig. 6 is a magnified cross-sectional view showing the film state of the fixing device in a nondriven state;

Fig. 7 is a schematic view showing a blow-off charge amount measuring apparatus for measuring the charge amount of toner;

Fig. 8 is a schematic cross-sectional view of a toner particle of the present invention; and

Fig. 9 is a schematic cross-sectional view of a conventional toner particle.

DESCRIPTION OF THE PREFERRED EMBODIMENTS

Now the present invention will be clarified in more detail in the following. The electrostatic charge image developing toner of the present invention is composed of a colorant covered in at least a part thereof by polyhydroxyalkanoate constituting a first resin component, and binder resin constituting a second resin component. As the polyhydroxyalkanoate constituting the first resin component, there can be advantageously employed at least one selected from the group consisting of monomer units represented by the following formulas (1) to (10):

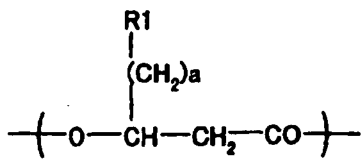

[1]

wherein the monomer unit is at least one selected from the group consisting of monomer units in which the combination of R1 and a is any of the following:

- a monomer unit in which R1 is a hydrogen atom (H) and a is an integer from 0 to 10;
- a monomer unit in which R1 is a halogen atom and a is an integer from 1 to 10;
- a monomer unit in which R1 is a chromophore and a is an integer from 1 to 10;
- a monomer unit in which R1 is a carboxyl group or a salt thereof and a is an integer from 1 to 10; and
- a monomer unit in which R is a group represented by the following formula:

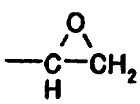

and a is an integer from 1 to 7;

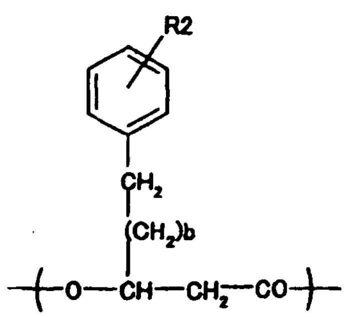

[2]

wherein b is an integer from 0 to 7, and R2 is a substitution selected from the group consisting of a hydrogen atom (H), a halogen atom, -CN, $-NO_2$, $-CF_3$, $-C_2F_5$ and $-C_3F_7$;

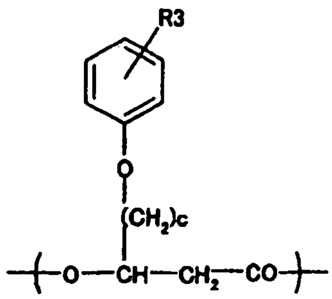

[3]

wherein c is an integer from 1 to 8, and R3 is a substitution selected from the group consisting of a hydrogen atom (H), a halogen atom, -CN, $-NO_2$, $-CF_3$, $-C_2F_5$ and $-C_3F_7$;

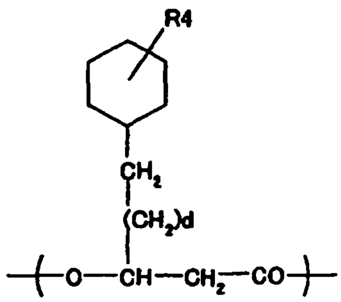

[4]

wherein d is an integer from 0 to 7, and R4 is a substitution selected from the group consisting of a hydrogen atom (H), a halogen atom, -CN, $-NO_2$, $-CF_3$, $-C_2F_5$ and $-C_3F_7$;

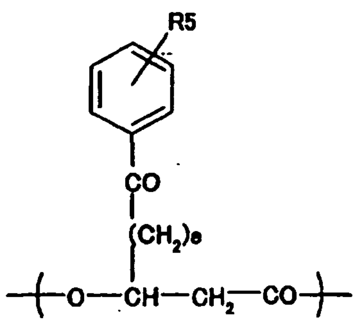

[5]

wherein e is an integer from 1 to 8, and R5 is a substitution selected from the group consisting of a hydrogen atom (H), a halogen atom, -CN, $-NO_2$, $-CF_3$, $-C_2F_5$, $-C_3F_7$, $-CH_3$, $-C_2H_5$ and $-C_3H_7$;

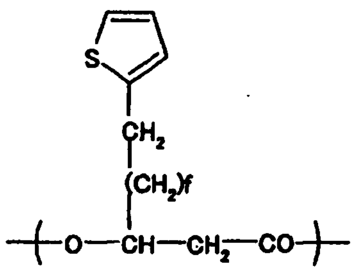

[6]

wherein f is an integer from 0 to 7;

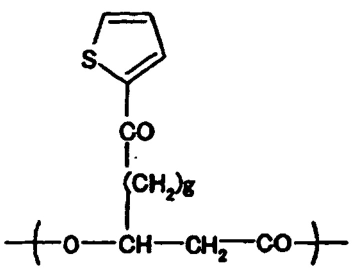

[7]

wherein g is an integer from 1 to 8;

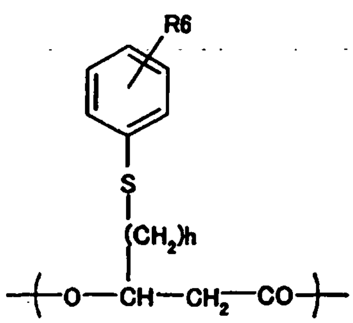

[8]

wherein h is an integer from 1 to 7, R6 is a substitution selected from the group consisting of a hydrogen atom (H), a halogen atom, -CN, $-NO_2$, -COOR', $-SO_2R''$, $-CH_3$, $-C_2H_5$, $-C_3H_7$, $-CH(CH_3)_2$ and $-C(CH_3)_3$, R' is a hydrogen atom, Na, K, $-CH_3$ or $-C_2H_5$, and R" is -OH, -ONa, -OK, a halogen atom, $-OCH_3$ or $-OC_2H_5$;

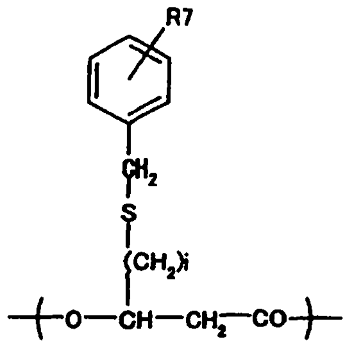

[9]

wherein i is an integer from 1 to 7, R7 is a substitution selected from the group consisting of a hydrogen atom (H), a halogen atom, -CN, $-NO_2$, -COOR' and $-SO_2R''$, R' is a hydrogen atom, Na, K, $-CH_3$ or $-C_2H_5$, and R" is -OH, -ONa, -OK, a halogen atom, $-OCH_3$ or $-OC_2H_5$; and

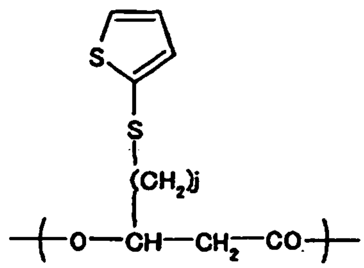

[10]

wherein j is an integer from 1 to 9.

Such PHA can be synthesized with the respectively corresponding 3-hydroxyacyl coenzyme A, namely 3-hydroxyacyl coenzymes A of a necessary number selected from those represented by the following chemical formulas (11) to (20):

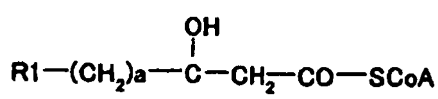

[11]

wherein -SCoA indicates coenzyme A bonded to alkanoic acid, and the combination of R1 and a is any of the following and corresponds to the combination of R1 and a in the foregoing chemical formula (1):

a monomer unit in which R1 is a hydrogen atom (H) and a is an integer from 0 to 10;
a monomer unit in which R1 is a halogen atom and a is an integer from 1 to 10;
a monomer unit in which R1 is a chromophore and a is an integer from 1 to 10;
a monomer unit in which R1 is a carboxyl group or a salt thereof and a is an integer from 1 to 10; and
a monomer unit in which R1 is a group represented by the following formula:

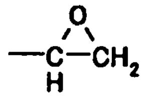

and a is an integer from 1 to 7;

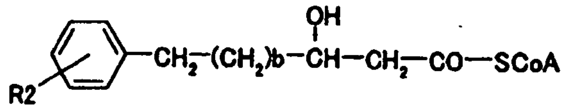

[12]

wherein -SCoA indicates coenzyme A bonded to alkanoic acid, b is an integer from 0 to 7 corresponding to b in the foregoing chemical formula (2), and R2 is a substitution selected from the group consisting of a hydrogen atom (H), a halogen atom, -CN, $-NO_2$, $-CF_3$, $-C_2F_5$ and $-C_3F_7$, corresponding to R2 in the foregoing chemical formula (2);

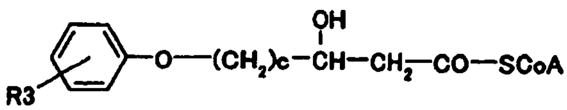

[13]

wherein -SCoA indicates coenzyme A bonded to alkanoic acid, c is an integer from 1 to 8 corresponding to c in the foregoing chemical formula (3), and R3 is a substitution selected from the group consisting of a hydrogen atom (H), a halogen atom, -CN, $-NO_2$, $-CF_3$, $-C_2F_5$ and $-C_3F_7$, corresponding to R3 in the foregoing chemical formula (3);

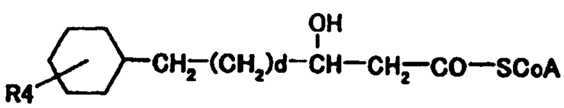

[14]

wherein -SCoA indicates coenzyme A bonded to alkanoic acid, d is an integer from 0 to 7 corresponding to d in the foregoing chemical formula (4), and R4 is a substitution selected from the group consisting of a hydrogen atom (H), a halogen atom, -CN, $-NO_2$, $-CF_3$, $-C_2F_5$ and $-C_3F_7$, corresponding to R4 in the foregoing chemical formula (4);

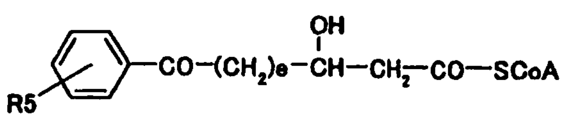

[15]

wherein -SCoA indicates coenzyme A bonded to alkanoic acid, e is an integer from 1 to 8 corresponding to e in the foregoing chemical formula (5), and R5 is a substitution selected from the group consisting of a hydrogen atom (H), a halogen atom, -CN, $-NO_2$, $-CF_3$, $-C_2F_5$, $-C_3F_7$, $-CH_3$, $-C_2H_5$ and $-C_3H_7$, corresponding to R5 in the foregoing chemical formula (5);

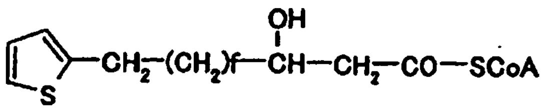

[16]

wherein -SCoA indicates coenzyme A bonded to alkanoic acid, and f is an integer from 0 to 7 corresponding to f in the foregoing chemical formula (6);

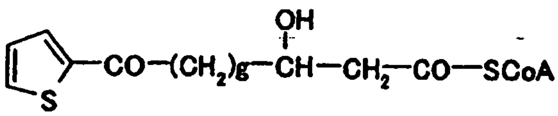

[17]

wherein -SCoA indicates coenzyme A bonded to alkanoic acid, and g is an integer from 1 to 8 corresponding to g in the foregoing chemical formula (7);

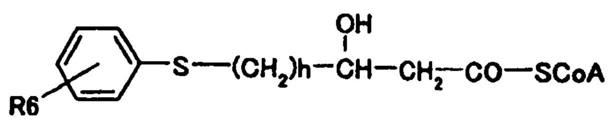

[18]

wherein -SCoA indicates coenzyme A bonded to alkanoic acid, h is an integer from 1 to 7 corresponding to h in the foregoing chemical formula (8), R6 is a substitution selected from the group consisting of a hydrogen atom (H), a halogen atom, -CN, $-NO_2$, -COOR', $-SO_2R''$, $-CH_3$, $-C_2H_5$, $-C_3H_7$, $-CH(CH_3)_2$ and $-C(CH_3)_3$ corresponding to R6 in the foregoing chemical formula (8), R' is a hydrogen atom, Na, K, $-CH_3$ or $-C_2H_5$, and R'' is -OH, -ONa, -OK, a halogen atom, $-OCH_3$ or $-OC_2H_5$;

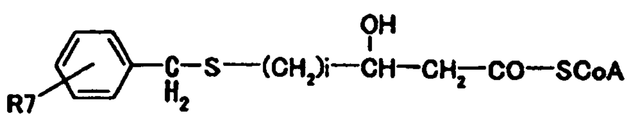

[19]

wherein -SCoA indicates coenzyme A bonded to alkanoic acid, i is an integer from 1 to 7 corresponding to i in the foregoing chemical formula (9), R7 is a substitution selected from the group consisting of a hydrogen atom (H), a halogen atom, -CN, $-NO_2$, -COOR' and $-SO_2R''$ corresponding to R7 in the foregoing chemical formula (9), R' is a hydrogen atom, Na, K, $-CH_3$ or $-C_2H_5$, and R'' is -OH, -ONa, -OK, a halogen atom, $-OCH_3$ or $-OC_2H_5$; and

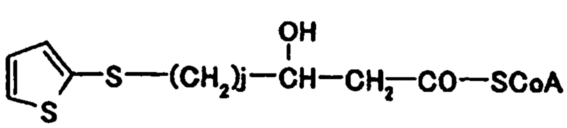

[20]

wherein -SCoA indicates coenzyme A bonded to alkanoic acid, and j is an integer of 1 to 9 corresponding to the foregoing chemical formula (10).

As explained in the foregoing, the electrostatic charge image developing toner of the present invention is featured by, instead of directly dispersing the coloring agent such as pigment into the toner, dispersing colorant, covered with outer shell resin consisting of PHA constituting the first resin, in the binder resin containing thermoplastic resin. Fig. 8 is a schematic cross-sectional view of a toner particle in which the colorant is dispersed in the toner binder resin, while

Fig. 9 is a cross-sectional view of a conventional toner particle. In case of the conventional toner, pigment 1 is dispersed in binder resin 4, but, in case of the present invention, pigment 1 is covered with first resin component 2 and is further dispersed in second resin component 3.

In the toner of the present invention, since the colorant covered with the outer shell resin is bound by the thermoplastic resin, the combination of the coloring agent contained in the colorant and the resin for binding the colorant is not limited and there can be obtained large freedom in the material selection. Also the coloring agent, for example pigment particles cause less migration to the exterior of the colorant (exposure to the surface of the colorant). Besides, the colorant, covered with the outer shell, can be produced with a sharper particle size distribution even in case of containing the coloring agent at a higher concentration. The interior of the colorant covered with polyhydroxyalkanoate generally consists of the coloring agent itself, and the coloring agent is preferably composed of pigment in consideration of the light fastness and antibleeding resistance of the coloring agent.

In the following there will be given a detail explanation on the aforementioned colorant.

<PHA>

The PHA to be employed in the present invention can be any PHA that can be synthesized by a PHA synthesizing enzyme relating to the PHA biosynthesizing reaction.

The biosynthesis of PHA is executed from various alkanoic acids as starting material by a polymerization reaction by an enzyme, utilizing, as the substrate, (R)-3-hydroxyacyl CoA generated through various metabolic paths (for example β-oxidation system or fatty acid synthesis path) in the organisms. The enzyme catalyzing such polymerization reaction is PHA synthesizing enzyme (also called PHA polymerase or PHA synthase).

CoA is an abbreviation for coenzyme A, having the aforementioned chemical structure. Also the reaction path from alkanoic acid to PHA through the β-oxidation system and polymerization reaction by the PHA synthesizing enzyme is as explained in the foregoing. On the other hand, in case of synthesis through the fatty acid synthesis path, PHA is assumed to be synthesized similarly by the PHA synthesizing enzyme, utilizing, as the substrate, (R)-3-hydroxyacyl CoA converted from (R)-3-hydroxyacyl ACP (ACP meaning acyl carrier protein) generated in such path. It is already known and reported, as explained in the foregoing, that PHA can be synthesized in a cell-free system (in vitro) by taking out the aforementioned PHB synthesizing enzyme or PHA synthesizing enzyme from the bacteria. As explained in the foregoing, the PHA synthesizing enzyme catalyzes the final stage in the PHA synthesizing reaction system in the organisms, so that any PHA known to be synthesizable in the organisms is synthesized by the catalyzing effect of such enzyme. Therefore, it is possible to prepare microcapsules formed by covering the coloring agent with any PHA known to be synthesizable in the organisms, by reacting 3-hydroxyacyl CoA corresponding to the desired PHA on the aforementioned enzyme fixed on the coloring agent of the present invention.

Specific examples of the PHA employable in the present invention include the aforementioned PHA. Specific examples of the aforementioned halogen atom include fluorine, bromine and chlorine. Also the aforementioned chromophore is not particularly limited as long as its 3-hydroxyacyl CoA bonding form can be catalyzed by the PHA synthesizing enzyme, but, in consideration of steric hindrance in the polymer systhesis, it is desirable, in the 3-hydroxyacyl CoA molecule, that a methylene chain with 1 to 5 carbon atoms is present between the carboxyl group bonded to CoA and the chromophore. Also the colored composition consisting of microcapsule pigment based on the PHA having such chromophore is expected, for example, to exhibit more effective color development by a composite action with the color developing component of the pigment.

Also as the PHA to be employed in the present invention, there can be utilized random copolymer or block copolymer including a plurality of the aforementioned monomer units, and there can be achieved control of the physical properties, realization of plural functions and realization of novel functions utilizing the properties of the monomer units or the functional groups contained therein or the interaction of such functional groups. It is also possible to synthesize, on the surface of the coloring agent, a block copolymer of arbitrary sequence and composition by suitably controlling the amount and order of addition of 3-hydroxyacyl CoA constituting the substrate. It is also possible, if necessary, to execute chemical modification after or during the PHA synthesis.

For example, it is possible to vary the monomer unit composition of PHA in a direction from the inner side to the outer side of the colorant, by changing in time the type and concentration of 3-hydroxyacyl CoA constituting the substrate. In this manner it is possible to form PHA showing higher mutual solubility with the binder resin in the outer surfacial layer of the colorant and to form PHA showing higher affinity with the coloring agent in the inner surfacial layer of the colorant, thereby enhancing the effect of the present invention. More specifically, in case PHA having mutual solubility with the binder resin shows low affinity with the coloring agent, there can be employed a process of at first covering the coloring agent with PHA showing higher affinity therewith and changing the monomer unit of the PHA toward higher mutual solubility with the binder resin from the inner side of the colorant to the outer side thereof for example in a multi-layered or gradient structure, thereby producing colorant showing stronger bonding with the coloring agent and having a PHA covering mutually soluble with the binder resin.

Also by introducing a graft chain in the PHA on the surface of the colorant, there can be obtained the colorant showing mutual solubility with the binder resin, based on such graft chain. Also there can be obtained colorant of superior mechanical strength by crosslinking the PHA on the surface of the colorant.

The PHA synthesized by the PHA synthesizing enzyme and employed in the structured material of the present invention is generally an isotactic polymer consisting solely of R-isomer.

The method for synthesizing 3-hydroxyacyl CoA, serving as the PHA synthesizing substrate, can be suitably selected for example from in vitro synthesis utilizing enzyme, in vivo synthesis utilizing organisms such as microorganisms or plants, and chemical synthesis. In particular, the enzyme synthesis method is generally employed for synthesizing such substrate, and there is for example known a method based on the following reaction:

3-hydroxyalkanoic acid + CoA → (acyl CoA synthetase) → 3-hydroxyacyl CoA

utilizing the commercially available acyl CoA synthetase (acyl CoA ligase, E.C.6.2.1.3) (Eur. J. Biochem., 250, 432-439 (1997); Appl. Microbiol. Biotechnol., 54, 37-43(2000), etc). The synthesis utilizing enzyme or organisms can be executed in batch or in continuous manner utilizing fixed enzyme or fixed cells.

<PHA synthesizing enzyme and producing bacteria therefor>

The PHA synthesizing enzyme to be employed in the present invention can be produced by microorganisms suitably selected from those capable of producing such enzyme, or produced by a transformant obtained by introducing a PHA synthesizing enzyme gene of such microorganisms into host microorganisms.

The microorganisms producing the PHA synthesizing enzyme can be PHB or PHB/V producing bacteria, and include those of Aeromonas sp., Alcaligenes sp., Chromatium sp., Comamonas sp., Methylobacterium sp., Paracoccus sp. and Pseudomonas sp., and there can also be utilized Burkholderia cepacia KK01, Ralstonia eutropha TB64 or Alcaligenes sp. TL2 separated by the present inventors. The KK01 strain, TB64 strain and TL2 strain are deposited, respectively under deposition numbers FERM BP-4235, BP-6933 and BP-6913, at the International Patent Organism Depositary (IPOD), National Institute of Advanced Industrial Science and Technology (AIST), Japan.

The microorganisms producing the PHA synthesizing enzyme can also be mcl-PHA or unusual-PHA producing bacteria, and include Pseudomonas oleovolans, Pseudomonas resinovolans, Pseudomonas sp.61-3, Pseudomonas putida KT2442 and Pseudomonas aeruginosa, and there can also be utilized the microorganisms of Pseudomonas species such as Pseudomonas putida P91, Pseudomonas cichorii H45, Pseudomonas cichorii YN2 and Pseudomonas jessenii P161 separated by the present inventors, those of Burkholderia species such as Burkholderia sp. OK3 (FERM P-17370) described in the Japanese Patent Application Laid-open 2000-78753, or Burkholderia sp. OK4 (FERM P-17371) described in the Japanese Patent Application Laid-open 2001-69968. In addition, there can also be employed microorganisms of Aeromonas sp. or Comamonas sp. capable of producing mcl-PHA or unusual-PHA.

The P91 strain, H45 strain, YN2 strain and P161 strain are deposited, under the Budapest Treaty on the International Recognition of the Deposit of Microorganisms for the Purposes of Patent Procedure and respectively assigned deposition numbers FERM BP-7373, BP-7374, BP-7375 and BP-7376, at the IPOD, AIST.

For the ordinary culture of the microorganisms to be utilized in the production of PHA synthesizing enzyme of the present invention, for example for preparation of reserve strain or for proliferation for securing the number and active state of bacteria required for production of the PHA synthesizing enzyme, there is suitably selected a culture medium containing components necessary for the proliferation of the microorganisms. For example, unless detrimental to the growth or living of the microorganisms, there can be employed any culture medium, such as usual natural culture medium (broth culture medium, yeast extract etc.) or chemically defined culture medium in which nutrition sources are added.

The culture can be executed in any culture method such as liquid culture or solid culture. Also there may be employed any of batch culture, fed batch culture, semi-continuous culture or continuous culture. For example for liquid batch culture, there can be employed oxygen supply method by shaking in a shaking flask or by agitated aeration in a jar fermenter.

In case of producing the PHA synthesizing enzyme with the aforementioned PHA producing microorganisms, there can be employed, for example, a method of proliferating such microorganisms in an inorganic culture medium containing alkanoic acid such as octanoic acid or nonanoic acid and recovering the microorganisms in the logarithmic proliferation stage to the initial stage of the stationary stage for example by centrifuging thereby extracting the desired enzyme. In case of the culture under the aforementioned condition, the mcl-PHA resulting from the added alkanoic acid is synthesized in the bacteria, and, in such case, the PHA synthesizing enzyme is generally considered to be bonded to the fine PHA particles formed in the bacteria. However, the investigation by the present inventors indicates that a considerable enzyme activity is present also in the supernatant after the centrifuging separation of the bacteria cultured in the aforementioned methods. Such phenomenon is presumably ascribable to a fact that the PHA synthesizing enzyme in free state is also present in a considerable amount, since such enzyme is actively produced in the bacteria in the relatively early stage of culture, from the logarithmic proliferation stage to the early stage of stationary stage as mentioned above.

Also in the aforementioned culture methods, there may be employed any inorganic culture medium containing components necessary for the proliferation of the microorganisms, such as phosphor source (for example phosphate salt) and nitrogen source (for example ammonium salt or nitrate salt), such as MSB culture medium, E culture medium (J. Biol. Chem., 218 97-106(1956)) or M9 culture medium. In the following there will be shown the composition of the inorganic salt M9 culture medium employed in the examples of the present invention to be explained later:

| | |
|---|---|
| $Na_2HPO_4$ | 6.2 g |
| $KH_2PO_4$ | 3.0 g |
| NaCl | 0.5 g |
| $NH_4Cl$ | 1.0 g |

(in 1 liter of culture medium; pH 7.0)

For satisfactory proliferation and resulting PHA production, the above-mentioned inorganic culture medium has to be replenished with the essential minor elements by adding the following minor component solution by about 0.3 %(w/v).

(Minor component solution) nitrilotriacetic acid 1.5 g;

| | |
|---|---|
| $MgSO_4$ | 3.0 g; |
| $MnSO_4$ | 0.5 g; |
| NaCl | 1.0 g; |
| $FeSO_4$ | 0.1 g; |
| $CaCl_2$ | 0.1 g; |
| $CoCl_2$ | 0.1 g; |
| $ZnSO_4$ | 0.1 g; |
| $C_USO_4$ | 0.1 g; |
| $AlK(SO_4)_2$ | 0.1 g; |
| $H_3BO_3$ | 0.1 g; |
| $Na_2MoO_4$ | 0.1 g; |
| $NiCl_2$ | 0.1 g; |

(in 1 liter).

The culture temperature can be any temperature at which the aforementioned strains can satisfactorily proliferate, for example 14 to 40˚C, preferably 20 to 35˚C.

It is also possible to produce the desired PHA synthesizing enzyme utilizing a transformant in which introduced is the PHA synthesizing enzyme gene of the aforementioned PHA producing bacteria. The cloning of the PHA synthesizing enzyme gene, preparation of expression vector and preparation of transformant can be executed according to the known methods. In the transformant obtained by utilizing Escherichia coli or the like as the host bacteria, there can be employed a natural or chemically defined culture medium such as LB culture medium or M9 culture medium for the culture. The proliferation of the microorganisms is executed by the aerobic culture for 8 to 27 hours at 25 to 37˚C. Then the bacteria are collected and the PHA synthesizing enzyme accumulated therein can be recovered. In the culture medium, there may be added, if necessary, antibiotics such as kanamycin, ampicillin, tetracycline, chloramphenicol, streptomycin etc. Also in case an inductive promoter is employed in the expression vector, an inductive substance corresponding to such promoter may be added to the culture medium in the culture of the transformant in order to accelerate expression. Examples of such inductive substance includes isopropyl-β-thiogalactopyranoside (IPTG), tetracycline and indole acrylic acid (IAA).

As the PHA synthesizing enzyme, there can be utilized crude enzyme such as bacteria crushed extract of the microorganisms or salted-out substance in which the protein component is precipitated and recovered with ammonium sulfate or the like, or pure enzyme purified with various methods. Such enzyme may be suitably added with a stabilizer or an activator such as a metal salt, glycerin, dithiothreitol, EDTA or bovine serum albumin (BSA).

The separation and purification of the PHA synthesizing enzyme can be executed in any method as long as the enzyme activity thereof can be conserved. For example the pure enzyme can be obtained by crushing the bacteria with French press, ultrasonic crusher, lysozyme or various surfactants, and subjecting crude enzyme obtained by centrifuging or salted-out substance prepared therefrom to affinity chromatography, cation or anion exchange chromatography, or gel permeation chromatography singly or in a suitable combination. Particularly a gene recombinant protein can be more simply purified by expressing it in the form of a fusion protein in which a "tag" such as a histidine residue

is bonded to the N- or C-end and bonding it to resin having affinity through such tag. The desired protein can be separated from the fused protein by breakage with protease such as thrombin or blood coagulating factor Xa, by pH decrease or by addition of imidazole at a high concentration as a competitive binding agent. Otherwise, in case the tag contains intein, as in the case of utilizing pTYB1 (New England Biolab. Inc.) as the expression vector, breakage is executed under a reducing condition for example with dithiothreitol. As the fused protein enabling purification by affinity chromatography, there are known, in addition to the histidine tag, glutathione s-transferase (GST), chitin-binding domain (CBD), maltase-binding protein (MBP) and thioredoxin (TRX). The GST fused protein can be purified with GST affinity resin.

The activity of the PHA synthesizing enzyme can be measured by various known methods, for example by the following method, based on a principle of measuring the color, developed by 5,5'-dithiobis-(2-nitrobenzoic acid), of CoA released in the course of polymerization of 3-hydroxyacyl CoA to form PHA by the catalytic action of the PHA synthesizing enzyme. Reagent 1: bovine serum albumin (Sigma Co.) being dissolved in 0.1 M tris hydrochloric acid buffer (pH 8.0) in 3.0 mg/mL; Reagent 2: 3-hydroxyoctanoyl CoA being dissolved in 0.1 M tris hydrochloric acid buffer (pH 8.0) in 3.0 mM; Reagent 3: trichloroacetic acid being dissolved in 0.1 M tris hydrochloric acid buffer (pH 8.0) in 10 mg/mL; Reagent 4: 5,5'-dithiobis-(2-nitrobenzoic acid) being dissolved in 0.1 M tris hydrochloric acid buffer (pH 8.0) in 2.0 mM. First reaction (PHA synthesizing reaction): 100 μL of the reagent 1 is added and mixed to 100 μL of specimen (enzyme) solution, and the mixture is pre-incubated for 1 minute at 30˚C. Then 100 μL of the reagent 2 is added and mixed, and the mixture is pre-incubated for 1 to 30 minutes at 30˚C, and then the reaction is terminated by adding the reagent 3. Second reaction (color developing reaction of free CoA): The first reaction mixture after termination of reaction is centrifuged (147,000 $m/s^2$ (15,000 G), 10 minutes), and 500 μL of the supernatant is added with 500 μL of the reagent 4 and, after incubation for 10 minutes at 30˚C, the optical absorbance at 412 nm is measured. Calculation of enzyme activity: An enzyme amount causing release of CoA of 1 μmol in 1 minute is defined as 1 unit (U).

<Colorant producing method>

As an example of the method for producing the electrostatic charge image developing toner utilizing the colorant of the present invention, there can be employed a method for producing the colorant, at least including the following preparation steps:

- Preparation steps of colorant -

a step of dispersing a coloring agent in aqueous medium;
a step of fixing polyhydroxyalkanoate sinthesizing agent to the coloring agent dispersed in the aqueous medium;
a step of adding 3-hydroxyacyl CoA serving as the substrate; and
a step of executing polyhydroxyalkanoate synthesizing reaction to cover at least a part of the surface of the coloring agent with polyhydroxyalkanoate.

The step of dispersing the coloring agent in the aqueous medium is executed by adding one or plural selected coloring agents, for example pigment, in the aqueous medium, and executing dispersion, if necessary followed by classification into a desired particle size range.

In the following there will be given a more detailed explanation in case the coloring agent consists of pigment.

The pigment to be employed in the present invention can be organic or inorganic, but is desirably of pigment excellent in heat resistance and light fastness. Examples of organic pigment include those of azo, phthalocyanine, benzimidazolone, quinacridone, isoindolinone, pyranthrone, dibromanthanthrone, indanthrone, anthrapyrimidine, flavanthrone, perylene, perinone, quinophthalone, phthalone, thioindigo, indigo, dioxazine, anthraquinone, xanthene, methine and azomethine types and other condensed polycyclic pigments including metal complex system. Examples of the inorganic pigment includes Miroli blue, iron oxide, cobalt violet, manganese violet, Prussian blue, ultramarine, cobalt blue, celurian blue, billidian, emerald green and cobalt green, and one or more can be suitably selected. Such pigment may be used after known surface treatments. Examples of such surface treatment include treatment with surfactant, coupling treatment or pigment derivative treatment.

The dispersion can be executed for example by a homomixer, a horizontal mini mill, a ball mill, a roll mill, a sand grinder, a crusher or ultrasonic treatment. There can also be utilized a method of passing the mixture through multiple nozzles under a liquid pressure at least equal to 1000 psi (about 70.3 $kg/cm^2$) in a liquid jet interaction chamber.

The dispersed pigment preferably has, from the standpoint of light transmittance and uniformity of the printed surface, a particle size at least not exceeding 0.7 μm, more preferably 0.01 to 0.4 μm, and is preferably in a monodisperse state. If the dispersed pigment is not in the desired particle size range, the particle size can be regulated by classification for example by filtration or precipitation.

The particle size of the dispersed pigment can be measured by known methods such as optical absorption, static light scattering, dynamic light scattering or centrifugal precipitation, and there can be utilized a particle size measuring apparatus such as Coulter Counter Multisizer for this purpose.

The aqueous medium for the synthesizing reaction in the present step can have any composition capable of dispersing the pigment in a desired dispersed state and not hindering the succeeding steps of fixing the enzyme to the pigment and executing PHA synthesis, but, in order to simplify the succeeding steps, the aqueous medium of the present step may have a composition allowing to exhibit the activity of the PHA synthesizing enzyme. As such composition, the aqueous medium can be composed for example of buffer. For such buffer, there can be advantageously employed ordinary buffer utilized in the biochemical reactions, such as acetic acid buffer, phosphoric acid buffer, potassium phosphate buffer, 3-(N-morpholino)propanesulfonic acid (MOPS) buffer, N-tris(hydroxymethyl)methyl-3-aminopropane sulfonic acid (TAPS) buffer, tris hydrochloric acid buffer, glycin buffer, 2-(cyclohexylamino)ethanesulfonic acid (CHES) buffer etc. The aqueous medium allowing to exhibit the PHA synthesizing enzyme can be employed in an ordinary concentration, namely 5 mM to 1.0 M, but preferably in a range of 10 to 200 mM. The buffer is so prepared that pH comes into a range of 5.5 to 9.0, preferably 7.0 to 8.5, but the condition may be set outside the aforementioned range, depending on the optimum pH or pH stability of the PHA synthesizing enzyme to be employed.

Also in order to maintain the dispersed state of the pigment in the aqueous medium, there may be added surfactant in type and concentration not hindering the succeeding steps and not hindering the objects of the colored composition of the present invention. Examples of such surfactant include anionic surfactants such as sodium oleate, sodium dodecylsulfonate, sodium dodecylsulfate, sodium dedecyl-N-sarcosinate, sodium cholate, sodium deoxycholate or sodium taurodeoxycholate; cationic surfactants such as cetyl trimethyl ammonium bromide or dodecyl pyridinium chloride; amphoteric surfactants such as 3-[(cholamidopropyl)dimethylammonio]-1-propane sulfonic acid (CHAPS) 3-[(3-cholamidopropyl)dimethylammonio]-2-hydroxy-1-propane sulfonic acid (CHAPSO), palmitoyl lysolecithin or dodecyl-β-alanine; and nonionic surfactants such as octyl glucoside, octyl thioglucoside, heptyl thioglucoside, decanoyl-N-methylglucamide (MEGA-10), polyoxyethylene dodecyl ether (Brij, Lubrol), polyoxyethylene i-octylphenyl ether (Triton X), polyoxyethylene nonylphenyl ether (Nonidet P-40, Triton N), polyoxyethylene fatty acid ester (Span) or polyoxyethylene sorbitol ester (Tween).

Also in order to maintain the dispersed state of the pigment in the aqueous medium, there may be added suitable auxiliary solvent in type and concentration not hindering the succeeding steps and not hindering the objects of the colored composition of the present invention. Such auxiliary solvent can be one or more selected from straight-chain aliphatic hydrocarbons such as hexane, monovalent alcohols such as methanol or ethanol, polyvalent alcohols such as glycerin, fatty acid esters and carboxylic acid esters.

The step of fixing the PHA synthesizing enzyme to the pigment can be executed by adding PHA synthesizing enzyme to the aforementioned pigment dispersion and executing fixation treatment. The fixation can be arbitrarily selected from the ordinary enzyme fixation methods as long as the enzyme activity can be retained and it is applicable to the desired pigment. Examples of such fixation method include covalent bonding, ionic adsorption, hydrophobic adsorption, physical adsorption, affinity adsorption, crosslinking and lattice inclusion, but particularly simple is the fixation utilizing ionic adsorption or hydrophobic adsorption.

The enzyme protein such as the PHA synthesizing enzyme is a polypeptide formed by a plurality of amino acids, and shows the property as ionically adsorbable substance by the amino acids having free ionic group such as lysine, histidine, arginine, aspartic acid or glutamic acid, and also shows the property as hydrophobically adsorbable substance by the amino acids having free hyrdrophobic group such as alanine, valine, isoleucine, methionine, tryptophan, phenylalanine or proline and also by being an organic polymer. It can therefore be adsorbed by pigment showing ionic character and/or hydrophobicity in variable levels.

In the method of fixing the PHA synthesizing enzyme principally by ionic adsorption, there can be employed pigment having ionic functional group on the surface, such as inorganic pigment principally composed of clay minerals or metal oxides.

Also in the method of fixing the PHA synthesizing enzyme principally by hydrophobic adsorption, there can be employed pigment having nonpolar surface, for example organic pigments such as azo pigment including plural aromatic rings, phthalocyanine pigment including condensed polycyclic structure, or anthraquinone pigment, or inorganic pigments composed of carbon crystals such as carbon black.

The fixation of the PHA synthesizing enzyme onto the pigment by ionic adsorption or hydrophobic adsorption can be achieved by mixing the pigment and the PHA synthesizing enzyme at predetermined concentrations in predetermined aqueous medium. In this operation, it is desirable to shake or agitate the reaction vessel at suitable intensity, in order that the enzyme can be uniformly adsorbed on the pigment surface.

In such fixation treatment, the aqueous medium containing the pigment and the enzyme in mixture desirably has a composition determined in consideration of a fact that the polarity of surface charge, charge amount and hydrophobicity of the pigment and the PHA synthesizing enzyme change depending on the pH and salt concentration of the aqueous medium. For example, if the pigment is principally ionically adsorbable, a decrease in the salt concentration

allows to increase the charge amount contributing to the adsorption between the pigment and the PHA synthesizing enzyme. Also a change in pH allows to increase the opposite charges of the two. In case the pigment is principally hydrophobically adsorbable, an increase in the salt concentration allows to increase the hydrophobicity of the two. It is also possible to select a composition suitable for adsorption by executing in advance electrophoresis or measuring the wetting angle to determine the charge state or hydrophobicity of the pigment and the PHA synthesizing enzyme. Also the composition can be determined by directly measuring the adsorption amount of the pigment and the PHA synthesizing enzyme. The adsorption amount can be measured, for example, by adding solution of PHA synthesizing enzyme of a known concentration to pigment dispersion, then executing adsorption process, measuring the concentration of the PHA synthesizing enzyme in the mixture and determining the adsorbed enzyme amount by subtraction.

In case of pigment for which it is difficult to fix the enzyme by ionic adsorption or hydrophobic adsorption, there may be employed covalent bonding method in consideration of the cumbersomeness of the operations or the possibility of deactivation of the enzyme. For example there can be utilized a method of converting pigment having an aromatic amino group into a diazo compound and executing diazo coupling of the enzyme thereto, a method of forming a peptide bond between pigment having a carboxyl group or an amino group and the enzyme, a method of executing alkylation between pigment having a halogen atom and the enzyme, a method of crosslinking an amino group of a solid particle and an amino group of the enzyme, a method of reacting pigment having a carboxyl group and an amino group and the enzyme in the presence of a compound having an aldehyde group or a ketone group and an isocyanide compound, or a method of executing exchange reaction between pigment having a disulfide group and a thiol group of the enzyme.

Also the enzyme may be fixed by affinity adsorption to the pigment in which a ligand is introduced.

In such case, there may be employed any ligand capable of executing affinity adsorption while maintaining the activity of the PHA synthesizing enzyme. The enzyme fixation may also be executed by bonding another organism-origin polymer such as protein to the PHA synthesizing enzyme and affinity adsorbing the thus bonded organism-origin polymer. Also the bonding of the PHA synthesizing enzyme and the organism-origin polymer can be executed either by gene recombination or by a chemical method. For example, as described later in the examples, fixation can be achieved by fusing glutathione S-transferase with PHA synthesizing enzyme by transformation and executing affinity adsorption of the fused protein by sepharose in which introduced is glutathione which is a ligand of glutathione-S-transferase.

Also fixation of polyhydroxyalkanoate onto the pigment surface can be achieved by having a peptide, including an amino acid sequence having bonding ability to the pigment, expressed with polyhydroxyalkanoate synthesizing enzyme, and fixing the PHA synthesizing enzyme to the pigment surface based on the bonding property of such peptide portion to the pigment.

The amino acid sequence having bonding ability to the pigment can be determined for example by screening of random peptide library. For example there can be advantageously employed a phage display peptide library prepared by linking a randomly synthesized gene to the N-end side gene of the surface protein (for example gene III protein) of M13 phage, and, in such case, the amino acid sequence having bonding ability to the pigment is determined by the following procedure. The phage display peptide library is added to and contacted with the pigment, and then the coupled phage and uncoupled phage are separated by rinsing. The phage coupled with the pigment is dissolved out with an acid, then neutralized with buffer and infected on Escherichia coli for phage amplification. Such selection is repeated plural times whereby plural clones having the bonding ability to the desired pigment are concentrated. In order to obtain a single clone, a colony is formed on the culture medium plate in a state infected again on Escherichia coli. Each single colony is cultured in liquid culture medium, and the phage present in the supernatant of the culture medium is purified by precipitation with polyethylene glycol etc. and the base sequence thereof is analyzed to know the structure of the peptide.

Thus obtained amino acid sequence of the peptide having the bonding ability to the pigment is utilized by fusing with the PHA synthesizing enzyme by an ordinary genetic engineering method. The peptide having the bonding ability to the pigment can be expressed by linking the N-end or C-end of the PHA synthesizing enzyme. It can also be expressed by inserting a suitable spacer sequence. The spacer sequence is preferably composed of 3 to 400 amino acids, and may contain any amino acid. There is most preferred is a spacer sequence that does not hinder the function of the PHA synthesizing enzyme nor coupling of the PHA synthesizing enzyme to the pigment.

The enzyme-fixed pigment prepared by the aforementioned method can be utilized in such state or after lyophilization etc.

By defining 1 unit (U) of the amount of the PHA synthesizing enzyme releasing 1 μmol/minute of CoA in the PHA synthesis reaction by polymerization of 3-hydroxyacyl CoA, the amount of enzyme fixed on the pigment is within a range of 10 to 1,000 U per 1g of pigment, preferably 50 to 500 U.

The enzyme fixation process is executed desirably within a time of 1 minute to 24 hours, more desirably 10 minutes to 1 hour. Excessively long standing is undesirable because it results in coagulation of pigment and deterioration of enzyme activity.

It is also possible to omit the initial step of pigment dispersion but to directly add the pigment, prior to dispersion in the aqueous medium, to the enzyme solution and to fix the enzyme to the pigment under dispersion in the enzyme

solution. In such case it is rendered possible, by the electrical repulsion or steric hindrance by the ionic functional groups contained in the enzyme fixed to the pigment, to facilitate dispersion of the pigment into the aqueous medium, thereby dispensing with the addition of surfactant to the aqueous medium or reducing the amount of such surfactant.

The step of adding 3-hydroxyacyl CoA as the substrate can be achieved by adding separately prepared reserve liquid of 3-hydroxyacyl CoA so as to attain a desired concentration. The 3-hydroxyacyl CoA serving as the substrate is added so as to attain an end concentration of 0.1 mM to 1.0 M, preferably 0.2 mM to 0.2 M, more preferably 0.2 mM to 1.0 mM.

Also in the above-mentioned step, by varying in time the composition such as type and concentration of the 3-hydroxyacyl CoA in the aqueous reaction mixture, the monomer unit composition of the PHA covering the coloring agent can be varied in a direction from the inner side to the outer side if the coloring agent has a spherical shape.

In such colorant showing change in the monomer unit composition, there can be assumed a configuration in which the PHA covering layer shows continuous change in the composition, and PHA of a single layer involving a gradient composition in the radial direction covers the coloring agent. Such configuration can be realized, in the course of synthesis of PHA, by adding 3-hydroxyacyl CoA of different composition.

There can also be another configuration, in which the PHA layer has stepwise changes in the composition and the coloring agent is covered by plural layers of PHA with different compositions. Such configuration can be realized for example by synthesizing PHA with a certain composition of 3-hydroxyacyl CoA, then recovering the colorant under preparation from the reaction mixture for example by centrifuging, and adding again reaction mixture having a different composition of 3-hydroxyacyl CoA.

The step of PHA synthesizing reaction is executed by regulating the reaction time and the reaction temperature and also regulating so as to obtain a composition capable of exhibiting the activity of the PHA synthesizing enzyme, in case the composition of the reaction mixture is not regulated in the preceding step, in order to obtain the microcapsule (colorant) of a desired shape by the synthesized PHA.

The concentration of the reaction solution capable of exhibiting the activity of the PHA synthesizing enzyme can be an ordinary concentration, namely within a range of 5 mM to 1.0 M, more preferably 10 to 200 mM. The pH is adjusted within a range of 5.5 to 9.0, preferably 7.0 to 8.5, but the condition may be outside the aforementioned range depending on-the optimum pH or pH stability of the PHA synthesizing enzyme to be used.

The reaction temperature is suitably set according to the characteristics of the PHA synthesizing enzyme to be used, usually within a range of 4 to 50˚C, preferably 20 to 40˚C, but the condition may be outside the aforementioned range depending on the optimum temperature or heat resistance of the PHA synthesizing enzyme to be used.

The reaction time varies depending on the stability etc. of the PHA synthesizing enzyme to be used, but is usually selected within a range of 1 minute to 24 hours, preferably 30 minutes to 3 hours.

The present step provides microcapsules, and the monomer unit structure of PHA constituting such microcapsule (colorant) can be determined, after extracting PHA from the microcapsule (colorant) with chloroform, by composition analysis with gas chromatography or by time of flight type secondary ion mass spectrometer (TOF-SIMS) and ion sputtering.

The molecular weight of PHA is not particularly limited, but the number average molecular weight is preferably selected within a range of 1,000 to 10,000,000, more preferably 10,000 to 1,000,000 in order to maintain the strength of the microcapsule (colorant) and to realize the glass transition point to be explained later. The molecular weight of PHA can be measured by GPC (gel permeation chromatography) after PHA extraction from the microcapsule (colorant) with chloroform.

For sufficiently attaining the objects of the present invention, it is desirable that the number average molecular weight satisfies the following relationship:

(number average molecular weight of first resin component) > (number average molecular weight of second resin component).

In case the first and second resin components satisfy the aforementioned relationship, in obtaining the toner by crushing the kneaded substance, the probability of crack generation in the second resin component or in the interface between the first and second resin components becomes high, so that the almost entire toner surface is covered with the first and second resin components. Stated differently, there is lowered the probability that the coloring agent such as pigment is directly exposed to the surface of the toner particle. It is therefore rendered possible to resolve the drawbacks of low charge amount, a large difference in the charge amount under a high temperature/high humidity condition or a low temperature/low humidity condition (environmental dependence), and fluctuation in the charge amount

among the toners of different colors utilizing pigments of cyan, magenta, yellow and black colors depending on the pigment types, for example in case of full-color image recording. Also it becomes possible to use the external addition material of a same type or a same amount for the toners of different colors.

Also the colorant of the present invention reduces the distribution of toner composition and improves the flowability of powder even in case the coloring agent such as pigment is used in a large amount. Particularly in case of adding the coloring agent such as pigment in a large amount to the small-sized toner not exceeding 5 μm, it is rendered possible to maintain the charging characteristics, fixing characteristics and powder characteristics that cannot be attained in the conventional methods. In the method of the present invention for producing the microcapsule (colorant), since the coloring agent such as pigment is directly covered with PHA, the density of the coloring agent can be elevated in the microcapsule. On the other hand, as the covering amount of PHA is requested to be increased in order to improve the dispersibility and mechanical strength of the microcapsule (colorant), it is within a range of 1 to 30 mass % in mass ratio with respect to the coloring agent, preferably 1 to 20 mass% and more preferably 1 to 15 mass%.

The conventional matrix-domain structure formed by fused phase separation of different resins is associated with the drawbacks of small selection range of the usable resins and difficulty in controlling the particle size and distribution of the domain, but, in the toner of the present invention, since the coloring agent covered with outer shell resin is bound by the thermoplastic resin, there is no limitation in the combination of the coloring agent and the binder resin and the concentration of the coloring agent in the colorant and the particle size thereof can be easily controlled.

The particle size of the microcapsule (colorant) obtained by the aforementioned steps is normally not exceeding 1 μm, preferably not exceeding 0.7 μm and more preferably 0.01 to 0.4 μm. The particle size of the microcapsule pigment can be measured by known methods such as optical absorption, static light scattering, dynamic light scattering or centrifugal precipitation, and there can be utilized a particle size measuring apparatus such as Coulter Counter Multisizer for this purpose.

Also the microcapsule (colorant) obtained in the aforementioned steps can be used after various secondary processes or chemical modification.

For example, by applying chemical modification to the PHA on the surface of the colorant, there can be obtained the colorant having more useful functions and characteristics. For example by introducing a graft chain, there can be obtained colorant improved in various characteristics, for example mutual solubility with the binder resin, derived from such graft chain. Also by crosslinking the PHA on the surface of the colorant, there can be improved the mechanical strength, chemical resistance, heat resistance etc. of the colorant.

The method of chemical modification is not particularly limited as long as it can attain the desired functions and structure, but there can be advantageously employed a method of synthesizing PHA having reactive functional group in the side chain and executing chemical modification utilizing the chemical reaction of such functional group.

The type of the aforementioned reactive functional group is not particularly limited as long as it can attain the desired functions and structure, but the aforementioned epoxy group can be cited as an example. The PHA having an epoxy group in the side chain can be subjected to chemical conversion as in the ordinary polymer having an epoxy group. More specifically, there can be executed conversion into a hydroxyl group or introduction of a sulfon group. It is also possible to add a compound having thiol or amine, and, more specifically, the graft chain of the polymer can be formed by reaction under addition of a compound having an end amino group highly reactive with the epoxy group.

Examples of the compound having an amino group at the end include amino modified polymers such as polyvinylamine, polyethylenimine or amino-modified polysiloxane (amino-modified silicone oil). Among these, amino-modified polysiloxane can be commercially available modified silicone oil or can be synthesized by the method described for example in J. Amer. Chem. Soc., 78, 2278(1956), and is expected to provide effects by the addition of graft chain in the polymer, such as improvement in the mutual solubility with the binder resin.

Other examples of chemical conversion of the polymer having an epoxy group include crosslinking reaction with a diamine compound such as hexamethylene diamine, succinic anhydride or 2-ethyl-4-methylimidazole, and examples of physicochemical conversion include crosslinking reaction by electron beam irradiation. Among these, the reaction between PHA having an epoxy group in the side chain and hexamethylene diamine proceeds in the following manner to produce crosslinked polymer:

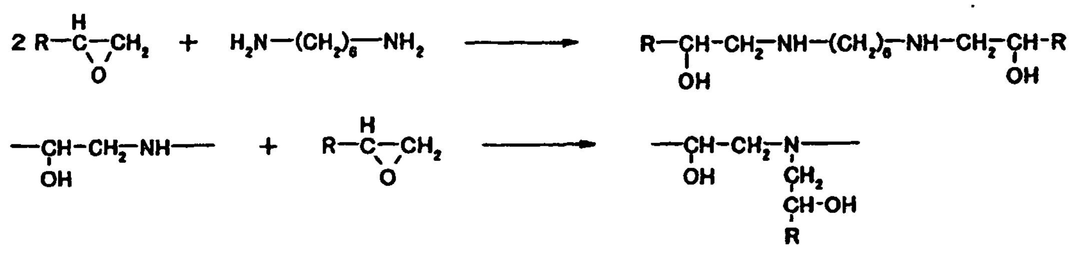

Since the microcapsule (colorant) of the present invention is featured by a high concentration of the coloring agent as explained in the foregoing and also by the small size thereof, the electrostatic charge image developing toner containing such microcapsule (colorant) allows to form an image excellent in transparency, color development and contrast.

<Examples of coloring agent>

The coloring agent constituting the electrostatic charge image developing toner of the present invention is not particularly limited and can be any coloring agent ordinarily employed in the toner manufacture. A coloring agent effectively employable in the present invention is pigment.

As the pigment, there can be utilized known organic or inorganic pigments. Examples of black pigment include inorganic ones such as carbon black and triiron tetraoxide, and organic ones such as cyanine black. Examples of white pigment include zinc white, titanium oxide, antimony white and zinc sulfide.

Examples of yellow pigment include inorganic ones such as lead yellow, cadmium yellow, yellow iron oxide, titanium yellow and ochre.

Also examples of monoazo pigment of acetoacetic acid anilide type which is a low-soluble metal salt (azo rake) include Hanza yellow G (C.I. Pigment Yellow 1), Hanza yellow 10G (C.I. Pigment Yellow 3), Hanza yellow RN (C.I. Pigment Yellow 65), Hanza brilliant yellow 5GX (C.I. Pigment Yellow 74), Hanza brilliant yellow 10GX (C.I. Pigment Yellow 98), Permanent yellow FGL (C.I. Pigment Yellow 97), Shimura rake fast yellow 6G (C.I. Pigment Yellow 133), and Rionol yellow K-2R (C.I. Pigment Yellow 169). Also examples of acetoacetic acid anilide type disazo pigment include disazo yellow G (C.I. Pigment Yellow 12), disazo yellow GR (C.I. Pigment Yellow 13), disazo yellow 5G (C.I. Pigment Yellow 14), disazo yellow 8G (C.I. Pigment Yellow 17), disazo yellow R (C.I. Pigment Yellow 55), and permanent yellow HR

(C.I. Pigment Yellow 83).

Also examples of condensed azo pigment include chromophthal yellow 3G (C.I. Pigment Yellow 93), chromophthal yellow 6G (C.I. Pigment Yellow 94), and chromophthal yellow GR (C.I. Pigment Yellow 95). Also examples of benzimidazolone type monoazo pigment include hostapalm yellow H3G (C.I. Pigment Yellow 154), hostapalm yellow H4G (C.I. Pigment Yellow 151), hostapalm yellow H2G (C.I. Pigment Yellow 120), hostapalm yellow H6G (C.I. Pigment Yellow 175) and hostapalm yellow HLR (C.I. Pigment Yellow 156). Also examples of isoindolinone type pigment include irgazin yellow 3RLTN (C.I. Pigment Yellow 110), irgazin yellow 2RLT, irgazin yellow 2GLT (C.I. Pigment Yellow 109), fastgen super yellow GROH (C.I. Pigment Yellow 137), fastgen super yellow GRO (C.I. Pigment Yellow 110), and sandrin yellow 6GL (C.I. Pigment Yellow 173). There can also be utilized threne type pigments such as flavanthrone yellow (C.I. Pigment Yellow 24), anthrapyrimidine (C.I. Pigment Yellow 108), phthaloylamide type anthraquinone (C.I. Pigment Yellow 123) and heliofast yellow E3R (C.I. Pigment Yellow 99); metal complex pigments such as azo nickel complex pigment (C.I. Pigment Green 10), nitroso nickel complex pigment (C.I. Pigment Yellow 153) and azomethine copper complex pigment (C.I. Pigment Yellow 117), or quinophtharone yellow (C.I. Pigment Yellow 138) which is a phthalimide type pigment.

Examples of magenta pigment include inorganic ones such as cadmium red, Indian red, silver vermilion, red lead and antimony vermilion. Also examples of azo rake of azo pigment include brilliant carmine 6B (C.I. Pigment Red 57:1), rake red (C.I. Pigment Red 53:1), permanent red F5R (C.I. Pigment Red 48), lysol red (C.I. Pigment Red 49), persian orange (C.I. Pigment Orange 17), chrosay orange (C.I. Pigment Orange 18), helio orange TD (C.I. Pigment Orange 19), pigment scarlet (C.I. Pigment Red 60:1), brilliant scarlet G (C.I. Pigment Red 64:1), helio red RMT (C.I. Pigment Red 51), bordeaux 10B (C.I. Pigment Red 63) and helio bordeaux (C.I. Pigment Red 54). Also examples of insoluble azo (monoazo, disazo, condensed azo) pigment include para red (C.I. Pigment Red 1), rake red 4R (C.I. Pigment Red 3), permanent orange (C.I. Pigment Orange 5), permanent red FR2 (C.I. Pigment Red 2), permanent red FRLL (C.I. Pigment Red 9), permanent red FGR (C.I. Pigment Red 112), brilliant carmine BS (C.I. Pigment Red 114), permanent carmine FB (C.I. Pigment Red 5), P.V. carmine HR (C.I. Pigment Red 150), permanent carmine FBB (C.I. Pigment Red 146), nova palm red F3RK-F5RK (C.I. Pigment Red 170), nova palm red HFG (C.I. Pigment Orange 38), nova palm red HF4B (C.I. Pigment Red 187), nova palm orange HL.HL-70 (C.I. Pigment Orange 86), P.V. carmine HF4C (C.I. Pigment Red 185), hosta balm brown HFR (C.I. Pigment Brown 25), Vulcan orange (C.I. Pigment Orange 16), pyrazolone orange (C.I. Pigment Orange 13) and pyrazolone red (C.I. Pigment Red 36). Examples of condensed azo pigment include chromophthal orange 4R (C.I. Pigment Orange 31), chromophthal scarlet R (C.I. Pigment Red 166) and chromophthal red BR (C.I. Pigment Red 144).

Also among anthraquinone pigments which are condensed polycyclic pigments, there can be used pyranthrone orange (C.I. Pigment Orange 40), anthanthrone orange (C.I. Pigment Orange 168), and dianthraquinonyl orange (C.I. Pigment Red 177). Among thioindigo pigment there can be used thioindigo magenta (C.I. Pigment Violet 38), thioindigo violet (C.I. Pigment Violet 36) and thioindigo Red (C.I. Pigment Red 88). Among perinone pigments there can be used

perinone orange (C.I. Pigment Orange 43). Among perylene pigments there can be used perylene red (C.I. Pigment Red 190), perylene vermilion (C.I. Pigment Red 123), perylene maroon (C.I. Pigment Red 179), perylene scarlet (C.I. Pigment Red 149), and perylene red (C.I. Pigment Red 178). Among quinacridone pigment there can be used quinacridone red (C.I. Pigment Violet 19), quinacridone magenta (C.I. Pigment Red 122), quinacridone maroon (C.I. Pigment Red 206) and quinacridone scarlet (C.I. Pigment Red 207). Also there can be used pyrrocoline pigment, red fluorobin pigment and vat rake pigments (soluble dye + precipitator = fixed rake) as condensed polycyclic pigment.

Among cyan pigments there can be used inorganic ones such as Prussian blue, ultramarine, cobalt blue, and celurian blue. Also among phthalocyanine pigments there can be used fastgen blue BB (C.I. Pigment Blue 15), sumiton cyanine blue HB (C.I. Pigment Blue 15), cyanine blue 5020 (C.I. Pigment Blue 15:1), Simikaprint cyanine blue GN-O (C.I. Pigment Blue 15), fast sky blue A-612 (C.I. Pigment Blue 17), cyanine Green GB (C.I. Pigment Green 7), cyanine green S537-2Y (C.I. Pigment Green 36) and Sumiton fast violet RL (C.I. Pigment Violet 23). There can also be used indanthrone blue (PB-60P, PB-22, PB-21, PB-64) which is threne pigment, and methyl violet phosphor molybdenate rake (PV-3) which is a basic dye rake pigment. Also as base pigment, there can be used baryte powder, barium carbonate, clay, silica, white carbon, talk and alumina white. In addition there can be used pigments obtained by resin coating on the surface of the aforementioned pigments.

The aforementioned pigments can be used singly or in an arbitrary mixture for obtaining desired color of the toner. Also in consideration of the environmental security or safety to human body, there can be advantageously utilized various edible rakes, such as edible red 40 aluminum rake, edible red 2 aluminum rake, edible red 3 aluminum rake, edible red 106 aluminum rake, edible yellow 5 aluminum rake, edible yellow 4 aluminum rake, edible blue 1 aluminum rake, or edible blue 2 aluminum rake.

The content of the aforementioned coloring agent in the toner can be widely variable according to the desired coloring effect. Ordinarily, in order to obtain optimum toner characteristics, namely in consideration of the print coloring power, shape stability of toner, and toner scattering, such coloring agent is contained in a proportion of 0.1 to 60 mass parts, preferably 0.5 to 20 mass parts with respect to 100 mass parts of the binder resin.

<Post treatment of colorant>

The capsule structure (colorant) obtained by the producing method of the present invention can be utilized in the state of aqueous dispersion in the reaction mixture, or after recovery of the colorant by mild centrifuging or suction filtration followed by dispersion in another aqueous medium. It is also possible to use as solvent dispersion by dispersing the recovered capsule structure (colorant) in organic solvent in which PHA is insoluble, or as dispersion in organic solvent in which PHA is insoluble obtained by solvent replacement. It is furthermore possible to rinse the capsule structure (colorant) by the above-mentioned method.

The capsule structure (colorant) in powder state can be obtained, in case the particle size is large, by obtaining a wet cake through mild centrifuging or suction filtration, followed by drying by vacuum drying or jet mill. Also in case the particle size is small, drying can be executed by spray drying.

The particle size of the produced capsule structure (colorant) can be made uniform to a certain extent by employing the coloring agent of uniform particle size, but classification may be executed after the preparation of the capsule structure (colorant) in order to obtain more uniform particle size.

<Toner constituting materials>

In the following there will be explained other constituents of the electrostatic charge image developing toner of the present invention. The electrostatic charge image developing toner of the present invention includes at least the aforementioned colorant and the binder resin, and further contains a charge control agent and other additives if necessary.

(Binder resin)

The binder resin is not particularly limited, and there can be used any binder resin usually employed in the toner preparation. Examples of the binder resin include styrenic polymers such as polystyrene, polyacrylate ester and styrene-acrylate ester copolymer; polyvinyl chloride, polyvinyl acetate, polyvinylidene chloride, phenolic resin, epoxy resin, polyester resin etc.

(Biodegradable plastics)

In the present invention, the commercially available biodegradable plastics can also be used advantageously. Examples of the biodegradable plastics include "Ecostar", "Ecostar plus" (Hagiwara Kogyo), "Biopol" (ICI Japan), "Azicoat" (Ajinomoto), "Placcel", "Polycaprolactone" "Daicel Chemical", "Sholex", "Bionolle" (Showa Denko), "Lacty" (Shimadzu Mfg.), and "Lacea" (Mitsui Chemical).

(Other resins)

Examples of the styrene polymer include copolymer of styrene and (meth)acrylate ester, copolymer thereof with another monomer capable of copolymerizing therewith, copolymer of styrenic and dienic monomer (butadiene, isoprene etc.) and copolymer thereof with another monomer capable of copolymerizing therewith. Examples of polyester polymer include condensation product of an aromatic dicarboxylic acid and addition product of aromatic diol with alkylene oxide. Examples of epoxy polymer include reaction product of aromatic diol and epichlorohydrin and modified products thereof. Examples of polyolefin polymer include polyethylene, polypropylene and a copolymerized chain with another monomer capable of copolymerizing therewith. Examples of polyurethane polymer include poly addition product of aromatic diisocyanate and alkylene oxide addition product of aromatic diol.

Specific examples of the binder resin employed in the present invention include polymers of following polymerizable monomers, mixtures thereof, and copolymerization products obtained by using at least two of the following polymerizable monomers. Specific examples of such compounds include styrenic copolymers such as styrene-acrylic acid copolymer or styrene-methacrylic acid copolymer, polyester polymers, epoxy polymers, polyolefin polymers and polyurethane polymers.

(Polymerizable monomer)

Specific examples of the polymerizable monomer include styrene and derivatives thereof such as styrene, o-methylstyrene, m-methylstyrene, p-methylstyrene, p-methoxystyrene, p-phenylstyrene, p-chlorostyrene, 3,4-dichlorostyrene, p-ethylstyrene, 2,4-dimethylstyrene, p-n-butylstyrene, p-tert-butylstyrene, p-n-hexylstyrene, p-n-octylstyrene, p-n-nonylstyrene, p-n-decylstyrene or p-n-dodecylstyrene; ethylenic unsaturated monoolefins such as ethylene, propylene, butylene or isobutylene; unsaturated polyenes such as butadiene; halogenated vinyls such as vinyl chloride, vinylidene chloride, vinyl bromide or vinyl fluodide; vinyl esters such as vinyl acetate, vinyl propionate or vinyl benzoate; aliphatic $\alpha$-methylene monocarboxylic acid esters such as methyl methacrylate, ethyl methacrylate, propyl methacrylate, n-butyl methacrylate, isobuty methacrylate, n-octyl methacrylate, dodecyl methacrylate, 2-ethylhexyl methacrylate, stearyl methacrylate, phenyl methacrylate, dimethylaminoethyl methacrylate or diethylaminoethyl methacrylate; acrylate esters such as methyl acrylate, ethyl acrylate, n-butyl acrylate, isobutyl acrylate, propyl acrylate, n-octyl acrylate, dodecyl acrylate, 2-ethylhexyl acrylate, stearyl acrylate, 2-chloroethyl acrylate or phenyl acrylate; vinyl ethers such as vinylmethyl ether, vinylethyl ether or vinylisobutyl ether; vinyl ketones such as vinylmethyl ketone, vinylhexyl ketone or methylisopropenyl ketone; N-vinyl compounds such as N-vinylpyrrol, N-vinylcarbazole, N-vinylindole or N-vinylpyrrolidone; vinylnaphthalenes; acrylic or methacrylic acid derivatives such as acrylonitrile, methacrylonitrile acrylamide; esters of the aforementioned $\alpha,\beta$-unsaturated acids or diesters of dibasic acids; dicarboxylic acids such as maleic acid, methyl maleate, butyl maleate, dimethyl maleate, phthalic acid, succinic acid or terephthalic acid; polyols such as ethylene glycol, diethylene glycol, triethylene glycol, 1,2-propylene glycol, 1,3-propylene glycol, 1,4-butanediol, 1.6-hexanediol, bisphenol-A, hydrogenated bisphenol-A or polyoxyethylenated bisphenol-A; isocyanates such as p-phenylene diisocyanate, p-xylylene diisocyanate or 1,4-tetramethylene diisocyanate; amines such as ethylamine, butylamine, ethylenediamine, 1,4-diaminobenzene, 1,4-diaminobutane or monoethanolamine; and epoxy compounds such as diglycidyl ether, ethyleneglycol diglycidyl ether, bisphenol-A glycidyl ether or hydroquinone glycidyl ether.

(Crosslinking agent)

In the formation of the binder resin to be used in the present invention, there may be employed the following crosslinking agent if necessary. Examples of the crosslinking agent with two functional groups include divinylbenzene, bis(4-acryloxypolyethoxyphenyl) propane, ethyleneglycol diacrylate, 1,3-butyleneglycol diacrylate, 1,4-butanediol diacrylate, 1,5-pentanediol diacrylate, 1,6-hexanediol diacrylate, neopentylglycol diacrylate, diethyleneglycol diacrylate, triethyleneglycol diacrylate, tetraethyleneglycol diacrylate, diacrylates of polyethyleneglycol #200, #400 and #600, dipropyleneglycol diacrylate, polypropyleneglycol diacrylate, polyester type diacrylate, and methacrylates corresponding to the foregoing acrylates.

Examples of the crosslinking agent with more than two functional groups include pentaerythritol triacrylate, trimethyrolethane triacrylate, trimethyrolpropane triacrylate, tetramethyrolmethane tetraacrylate, oligoester acrylate and methacrylate, 2,2-bis(4-methacryloxy-polyethoxyphenyl) propane, diallyl phthalate, triallyl cyanurate, triallyl isocyanurate, triallyl trimellitate, and diaryl chlorendate.

(Polymerization starter)

In the formation of the binder resin to be used in the present invention, there may be employed the following polymerization starter if necessary. Examples of the polymerization starter include t-butylperoxy-2-ethyl hexanoate, cumin perpivarate, t-butyl peroxylaurate, benzoyl peroxide, lauroyl peroxide, octanoyl peroxide, di-t-butyl peroxide, t-butylcumyl peroxide, dicumyl peroxide, 2,2'-azobisisobutylonitrile, 2,2'-azobis(2-methylbutylonitrile), 2,2'-azobis(2,4-dimethylvaleronitrile), 2,2'-azobis(4-methoxy-2,4-dimethylvaleronitrile), 1,1-bis(t-butylperoxy)-3,3,5-trimethylcyclohexane, 1,1-bis(t-butylperoxy) cyclohexane, 1,4-bis(t-butylperoxycarbonyl) cyclohexane, 2,2-bis(t-butylperoxy) octane, n-butyl-4,4-bis(t-butylperoxy) valylate, 2,2-bis(t-butylperoxy) butane, 1,3-bis(t-butylperoxy-isopropyl) benzene, 2,5-dimethyl-2,5-di(t-butylperoxy) hexane, 2,5-dimethyl-2,5-di(benzoylperoxy) hexane, di-t-butyldiperoxy isophthalate, 2,2-bis (4,4-di-t-butylperoxycyclohexyl) propane, di-t-butylperoxy-α-methyl succinate, di-t-butylperoxydimethyl glutarate, di-t-butylperoxy hexahydroterephthalate, di-t-butylperoxy azelate, 2,5-dimethyl-2,5-di(t-butylperoxy) hexane, diethyleneglycol-bis(t-butylperoxycarbonate), di-t-butylperoxy trimethyladipate, tris(t-butylperoxy) triazine, and vinyltris(t-butylperoxy) silane.

These materials can be used singly or in combination. These materials are used at a concentration of at least 0.05 mass parts (preferably 0.1 to 15 mass parts) with respect to 100 mass parts of the monomer.

(Charge control agent)

There can be employed a charge control agent employed conventionally. Specific examples thereof include nigrosin dyes, quaternary ammonium salts and monoazo metal complex salt dyes. The amount of the charge control agent is determined in consideration of the charging property of the binder resin, the producing method including the amount and dispersing method of the colorant and the charging ability of other additives, but is generally employed in an amount of 0.1 to 20 mass parts, preferably 0.5 to 10 mass parts with respect to 100 mass parts of the binder resin. There may also be employed inorganic particles such as metal oxide or an inorganic substance surfacially treated with the aforementioned organic materials. Such charge control agent may be mixed in the binder resin or attached to the surface of the toner particles.

(Other components of toner)

The electrostatic charge image developing toner of the present invention may include, in addition to the aforementioned binder resin, colorant and charge control agent, the following compounds. Examples of such compound include silicone resin, polyester, polyurethane, polyamide, epoxy resin, polyvinylbutyral, rosin, denatured rosin, terpene resin, phenolic resin, aliphatic or alicyclic hydrocarbon resin such as low molecular weight polyethylene or low molecular weight polypropylene, aromatic petroleum resin, chlorinated paraffin and paraffin wax. Among these, there is preferably employed wax, and the examples thereof include low molecular weight polypropylene and byproducts thereof, low molecular weight polyester, ester wax and aliphatic derivatives. Wax classified by the molecular weight by various methods is also preferably employed in the present invention. After the classification, there may be also executed oxidation, block copolymerization or graft modification.

The electrostatic charge image developing toner of the present invention provides particularly excellent characteristics in case the toner includes the aforementioned wax component and such wax component is dispersed in substantially spherical- and/or spindle-shaped islands in the binder resin in the observation of the toner section under the transmission electron microscope (TEM).

(Method of toner preparation)

The electrostatic charge image developing toner of the present invention, having the above-described configuration, can be prepared by any known method. The dispersion of the colorant of the present invention into the binder resin can be achieved by fused kneading of the dried colorant into the binder resin under such a shearing force as not to break the colorant, or by melt flushing method in which a water-containing cake in the colorant preparing process, in which the colorant can be relatively easily dispersed into primary particles, is replaced by the fused binder resin.

(Crushing method)

The electrostatic charge image developing toner of the present invention can be prepared by so-called crushing method for obtaining the toner by the following steps. More specifically, the electrostatic charge image developing toner of the present invention can be prepared by sufficiently mixing the aforementioned colorant of the present invention, resins such as the binder resin, charge control agent and wax to be added if necessary by a mixer such as a Henshell

mixer or a ball mill, then mutually dissolving the resins by fusion kneading with a heat kneader such as heated rollers, a kneader or an extruder, then dispersing or dissolving therein additives such as a magnetic material and a metallic compound if necessary, then solidifying the mixture by cooling, crushing the solidified mixture with a crusher such as a jet mill or a ball mill and executing classification to a desired particle size.

In the classifying step, there is preferably employed a multi-division classifier in consideration of the production efficiency. In the present invention, in the fused kneading, there can be obtained electrostatic charge image developing toner in which the coloring agent such as pigment in a state covered by the first resin component is dispersed in the second resin component, without dissolving of the coloring agent such as pigment, covered by the first resin component, into the second resin component or without recoagulation of the coloring agent.

The electrostatic charge image developing toner of the present invention can also be obtained by mixing the binder resin, the charge control agent etc. in solution, utilizing solvent (for example aromatic hydrocarbon such as toluene or xylene, halogenated solvent such as chloroform or ethylene dichloride, ketone such as acetone or methylethyl-ketone, or amide such as dimethylformamide), putting the solution into water after agitation to achieve reprecipitation, then filtering and drying the precipitate, crushing the solid with a crusher such as a jet mill or a ball mill and executing classification to obtain the desired particle size. In the classifying step, there is preferably employed a multi-division classifier in consideration of the production efficiency.

(Polymerization method)

The electrostatic charge image developing toner of the present invention can also be obtained by so-called polymerization method explained in the following. In this case, a polymerizable monomer, the colorant of the present invention, a magnetic material, a crosslinking agent, a polymerization starter, wax and other additives if necessary are mixed and dispersed and are subjected to suspension polymerization in aqueous dispersion medium in the presence of a surfactant to obtain polymerized colored resin particles, which are then separated from the liquid phase, dried and subjected to classification if necessary to obtain the electrostatic charge image developing toner of the present invention.

(Externally added silica)

In the present invention, it is preferable to externally add, to the toner prepared in the above-described methods, fine silica powder in order to improve the charge stability, developability, flowability and durability. The fine silica powder to be employed for this purpose provides satisfactory result in case the specific surface area measured by the nitrogen adsorption by the BET method is at least equal to 20 $m^2/g$ (particularly 30 to 400 $m^2/g$). In such case, the fine silica powder is used in an amount of 0.01 to 8 mass parts, preferably 0.1 to 5 mass parts, with respect to 100 mass parts of toner particles. The fine silica powder to be used is preferably processed, in necessary in order to control the hydrophobicity and charging ability, with silicone varnish, modified silicon varnish, silicone oil, modified silicone oil, silane coupling agent, silane coupling agent containing functional groups, or other organic silicon compounds. These processing agents may be used as a mixture.

(Inorganic powder)

It is also preferable to add the following inorganic powder in order to improve the developability and durability of the toner. Examples of such inorganic powder include oxides of metals such as magnesium, zinc, aluminum, cerium, cobalt, iron, zirconium, chromium, manganese, strontium, tin or antimony; complex metal oxides such as calcium titanate, magnesium titanate or strontium titanate; metal salts such as calcium carbonate, magnesium carbonate or aluminum carbonate; clay minerals such as caolin; phosphate compounds such as apatite; silicon compounds such as silicon carbide or silicon nitride; and carbon powder such as carbon black or graphite. Among these, particularly preferred is fine powder of zinc oxide, aluminum oxide, cobalt oxide, manganese dioxide, strontium titanate or magnesium titanate.

(Lubricant)

It is also possible to add following lubricant powder to the toner. Examples of such lubricant include fluorinated resins such as teflon polyfluorovinylidene; fluorinated compounds such as carbon fluoride; metal salts of fatty acids such as zinc stearate; fatty acid derivatives such as fatty acid or fatty acid ester; and molybdenum sulfide.

In the toner of the present invention, the surface thereof is almost covered by the first resin component and the second resin component, so that the charging property of the toner is not influenced by the type of the pigment. Consequently, the externally added material of a same type or amount can be used in various toner.

Such binder resin, charge control agent and other additive employed if necessary are preferably composed of biodegradable ones if possible, in consideration of the situation after discarding.

<Carrier>

The electrostatic charge image developing toner of the present invention can be used singly as the non-magnetic one-component developer, or applied to the conventionally known various toners such as non-magnetic toner constituting the magnetic two-component developer together with magnetic carrier or magnetic toner to be singly used as the magnetic one-component toner. In case of use in the two-component developing method, there can be utilized any known carrier. More specifically, the carrier particles can be constituted by particles of an average particle size of 20 to 300 μm composed of a surfacially oxidized or unoxidized metal such as iron, nickel, cobalt, manganese, chromium or a rare earth metal, or alloys or oxides thereof. The carrier to be employed in the present invention is preferably covered, on the surface of the carrier particles, with styrene resin, acrylic resin, silicone resin, fluorinated resin, polyester resin or the like.

<Magnetic toner>

The electrostatic charge image developing toner of the present invention can also be formed as magnetic toner by including a magnetic material in the toner particles. In such case, the magnetic material may also serve as a coloring agent. Examples of such usable magnetic material include iron oxides such as magnetite, hematite or ferrite; metals such as iron, cobalt or nickel; and alloys of such metals with other metals such as aluminum, cobalt, copper, lead, magnesium, tin, zinc, antimony, beryllium, bismuth, cadmium, calcium, manganese, selenium, titanium, tungsten or vanadium; and mixtures thereof. Such magnetic material to be used in the present invention has an average particle size not exceeding 2 μm, preferably 0.1 to 0.5 μm. The content in the toner is preferably 20 to 200 mass parts, more preferably 40 to 150 mass parts with respect to 100 mass parts of the binder resin.

Also for achieving higher image quality, it is necessary to enable faithful development of smaller latent image dots, and, for this purpose, it is preferable that the electrostatic charge image developing toner of the present invention has a weight-averaged particle size within a range of 4 to 9 μm. The toner particles with a weight-averaged particle size less than 4 μm is undesirable because the transfer efficiency becomes lower to increase the toner amount remaining on the photosensitive member thereby resulting in image fog and uneven image caused by defective transfer. Also the toner particles with a weight-averaged particle size exceeding 9 μm tends to cause scattering of characters and line images.

In the present invention, the average particle size and the particle size distribution of the toner were measured with the Coulter Counter TA-II or Coulter Multisizer (supplied by Coulter Inc.) connected to an interface (supplied by Nippon Kagaku Kikai Co.) and a personal computer PC9801 (supplied by NEC) for outputting the number distribution and the volume distribution. As the electrolyte used in the measurement, there was prepared 1% NaCl aqueous solution with 1st grade sodium chloride. The electrolyte can also be for example composed of commercially available ISOTON R-II (supplied by Coulter Scientific Japan Inc.). In the measurement, a surfactant (preferably alkylbenzene sulfonate salt) as the dispersant was added in an amount of 0.1 to 5 ml in 100 to 150 ml of the aforementioned aqueous electrolyte solution, and a specimen for measurement was added by 2 to 20 mg to obtain the measurement sample. At the measurement, the electrolyte solution in which the measurement specimen was suspended was subjected to dispersion for 1 to 3 minutes by an ultrasonic disperser, and was subjected to the measurement of volume and number of toner of 2 μm or larger in the Coulter Counter TA-II with an aperture of 100 μm, thereby calculating the volume distribution and the number distribution. Then there were determined the weight average particle size (D4) based on the volume calculated from the volume distribution of the present invention, and the number average particle size (D1) based on the number calculated from the number distribution.

<Charge amount>

The electrostatic charge image developing toner of the present invention preferably has a charge amount per unit mass (two component method) of -10 to -80 μC/g, preferably -15 to -70 μC/g in order to improve the transfer efficiency in the transfer method utilizing a voltage-applied transfer member.

In the following there will be explained the method for measuring the charge amount by the two-component method (two-component tribo) employed in the present invention. For the measurement, there was employed a charge amount measuring apparatus shown in Fig. 7. At first, a mixture consisting of 9.5 g of carrier, composed of EFV 200/300 (supplied by Powdertech Inc.) and 0.5 g of the toner to be measured, was put in a polyethylene bottle of 50 to 100 ml, then placed on a shaker of a constant amplitude and was shaked for a predetermined time under an amplitude of 100 mm and a shaking speed of 100 cycle/minute. Then 1.0 to 1.2 g of the aforementioned mixture was put in a metallic measurement container 42 of the charge amount measuring apparatus shown in Fig. 7, having a screen 43 of 500 mesh at the bottom, and a metal cover 44 was placed. The mass of the entire measurement container 42 was measured as W1 (g)Then suction was executed with an unrepresented suction device (made insulating at least in a portion in contact with the measurement container 22) from a suction aperture 47, and an air amount adjusting valve 46 was so adjusted

that a vacuum meter 45 indicated a pressure of 2450 Pa (250 mm Aq). The suction was executed for 1 minute in this state to eliminate the toner by suction. The potential indicated by a potential meter 49 was selected as V (volt). A capacitor 48 had a capacity C (μF). The tribocharge amount of the toner (μC/g) was calculated from these measured values according to the following formula:

$$\text{Tribocharge amount } (\mu C/g) = C \times V/(W1 - W2).$$

<Molecular weight of binder resin>

In the present invention, the molecular weight of the binder resin was measured by GPC (gel permeation chromatography). More specifically, the molecular weight measurement by the GPC was executed by employing a sample obtained by extracting the toner with THF (tetrahydrofurane) for 20 hours in a Soxlet extractor, also employing a column configuration formed by connecting A-801, 802, 803, 804, 805, 806 and 807 supplied by Showa Denko Co. and utilizing a calibration line of standard polystyrene resin. Also in the present invention, it is preferable to use binder resin having a ratio (Mw/Mn) of the weight-average molecular weight (Mw) and the number-averaged molecular weight (Mn), measured as explained in the foregoing, within a range of 2 to 100.

<Glass transition point of toner>

Furthermore, the toner of the present invention is so prepared, with suitable materials, as to have a glass transition point Tg preferably within a range of 30 to 80°C, more preferably 50 to 70°C in consideration of the fixability and storability. The glass transition point Tg can be measured, for example, by a highly precise scanning differential thermal analyzer of internal input compensation type such as Perkin Elmer DSC-7. The measurement is executed according to ASTM D3418-82. In the present invention, the measurement of the glass transition point is preferably executed by once heating the specimen to eliminate the prior hysteresis, then rapidly cooling the specimen and utilizing a DSC curve obtained heating the specimen again within a range of 0 to 200°C with a heating rate of 10°C/min.

<Image forming method and apparatus>

The electrostatic charge image developing toner of the present invention having the aforementioned configuration is particularly preferably applied to an image forming method at least including a charging step of externally applying a voltage to a charging member thereby charging an electrostatic latent image bearing member, a step of forming an electrostatic charge image on the charged electrostatic latent image bearing member, a development step of developing the electrostatic charge image with electrostatic charge image developing toner thereby forming a toner image on the electrostatic latent image bearing member, a transfer step of transferring the toner image on the electrostatic latent image bearing member onto a recording material, and a heat fixation step of heat fixing the toner image on the recording material, or an image forming method in which the above-mentioned transfer step consists of a first transfer step of transferring the toner image on the electrostatic latent image bearing member onto an intermediate transfer member and a second transfer step of transferring the toner image on the intermediate transfer member onto the recording material.

Also the apparatus to be employed in this method is preferably provided means respectively corresponding to the aforementioned steps, namely charging means, electrostatic charge image forming means, developing means, transfer means and heat fixing means.

In the following, the present invention will be clarified further by examples thereof, in which (%) is based on mass unless otherwise specified.

(Reference example 1) Preparation of transformant capable of producing PHB synthesizing enzyme

The method for preparing a transformant capable of producing TB64 strain-originated PHB synthesizing enzyme was already applied for patent by the present inventors, and a specific example of such method will be explained in the following. After the TB64 strain was cultured overnight at 30°C in 100 mL of LB culture medium (1% polypeptone, 0.5% yeast extract, 0.5% sodium chloride, pH 7.4), the chromosomal DNA was separated and recovered by the method of Marmer et. al. The obtained chromosomal DNA was partially decomposed by restriction enzyme Sau3AI. Vector pUC18 was digested with restriction enzyme BamHI, and, after dephosphorylation process (Molecular Cloning, Vol. 1, p572 (1989): Cold Spring Harbor Laboratory), it was ligated with the fragment of the chromosomal DNA obtained by partial decomposition with Sau3AI, utilizing a DNA ligation kit Ver. II (Takara Shuzo). Then Escherichia Coli HB101 strain was

transformed with the ligated DNA fragments to obtain a chromosomal DNA library of the TB 64 strain.

Then there was executed pheno-type screening for obtaining the DNA fragment of the TB64 strain including the genes for the PHB synthesizing enzyme group. An LB culture medium containing 2% glucose was employed as the selection culture medium, and, when the colonies on the flat agar culture medium grew to an appropriate size, Sudan black B solution was sprayed and colonies emitting fluorescence under the UV light irradiation were acquired. Plasmid was recovered from the acquired colonies by the alkali method to obtain the DNA fragment containing the genes of the PHB synthesizing enzyme group.

The acquired gene fragment was recombined to a vector pBBR 122(Mo Bi Tec) including a broad host range replication origin not belonging to the incompatibility group IncP, IncQ or IncW, and the recombinant plasmid was used to transforme Ralstogna eutropha TB64m1 strain (lacking PHB synthesizing ability) by electroporation in whereby the PHB synthesizing ability of the TB64m1 strain was restored and complementary character was shown.

Then there was designed and synthesized an oligonucleotide having a base sequence in the region around the starting codon of the PHB synthesizing enzyme gene (Amasham Pharmacia Biotech), and PCR was executed utilizing such oligonucleotide as the primer to amplify the fragment including the PHB synthesizing enzyme genes (LA-PCR kit: Takara Shuzo).

Then thus obtained PCR amplified fragment was completely decomposed by restriction enzyme BamHI and ligated with an expression vector pTrc99A, which was completely decomposed by restriction enzyme BamHI and dephosphorylated (Molecular Cloning, Vol. 1, 5.7.2 (1989): Cold Spring Harbor Laboratory), with DNA ligation kit Ver. II (Takara Shuzo). Then Escherichia coli HB101 strain was transformed with the obtained recombinant plasmid by calcium chloride method (Takara Shuzo), and a recombinant plasmid recovered from the obtained transformant was named pTB64-PHB.

Escherichia coli HB101 was transformed with pTB64-PHB by calcium chloride method to obtain a pTB64-PHB recombinant strain.

(Reference example 2) Preparation of transformant capable of producing GST-fused PHA synthesizing enzyme

The pTB64-PHB recombinant strain was inoculated in 200 mL of LB culture medium, and was subjected to shaking culture for 12 hours at 37˚C and 125 stroke/minute. Thus obtained bacteria was recovered by centrifuging, and the plasmid DNA was recovered by an ordinary method.

Oligonucleotide (sequence number 1) constituting an upstream primer for the pTB64-PHB and oligonucleotide (sequence number 2) constituting a downstream primer were designed and synthesized (Amasham Pharmacia Biotech). PCR was executed utilizing the oligonucleotides as the primers and pTB64-PHB as the template to amplify the entire length of the PHB synthesizing enzyme gene having the BamHI restriction site at the upstream side and the Xhol restriction site at the downstream side (LA-PCR kit: Takara Shuzo).

The purified PCR amplification product was digested with BamHI and Xhol and was inserted into a corresponding restriction site in a plasmid pGEX-6P-1 (Amasham Pharmacia Biotech Inc.). Escherichia coli JM109 strain was transformed with the recombinant plasmid to obtain a strain for the enzyme expression. The confirmation of the recombinant strain was executed by a DNA fragment obtained by digesting the plasmid DNA, prepared in large scale with Miniprep (Wizard Minipreps DNA Purification System, PROMEGA Inc.) with BamHI and Xhol.

(Reference example 3) Preparation of PHB synthesizing enzyme

The obtained recombinant strain for the enzyme expression was pre-cultured overnight at 30˚C in 100 mL of 2xYT culture medium (polypeptone 16g/L, yeast extract 10 g/L, NaCl 5 g/L, pH 7.0) added with ampicillin (100 μg/L).

Then it was added to 10 liters of 2xYT culture medium (polypeptone 16g/L, yeast extract 10 g/L, NaCl 5 g/L, pH 7.0) added with ampicillin (100 μg/L) and culture was executed for 3 hours at 30˚C. Then isopropyl-β-D-thiogalactopyranocide (IPTG) was added to obtain a final concentration of 1mM, and the culture was executed for 3 hours at 30˚C.

The recovered culture medium was centrifuged for 10 minutes at 4˚C, 78000 $m/s^2$ (= 8000G), and, after the elimination of supernatant, the bacterial pellet was re-suspended in 500 mL of PBS solution at 4˚C. The bacterial suspension was poured, 40 mL each time, in a vessel cooled to 4˚C in advance, and, under pressurization of 216 MPA (= 2200 $kg/cm^2$) by a French press, the bacterial liquid was released little by little from the nozzle, thereby executing crushing process. The crushed bacterial suspension was centrifuged for 10 minutes at 4˚C, 78000 $m/s^2$ (= 8000G), and the supernatant was recovered. The supernatant was filtered with a filter of 0.45 μm to eliminate the debris. The expression of the desired PHB synthesizing enzyme fused with glutathione S transferase (GST) in the supernatant was confirmed by SDS-PAGE.

Then the GST-fused PHB synthesizing enzyme was purified with glutathione sepharose 4B (Amasham Pharmacia Biotech Inc.). 6.65 mL of 75% slurry of glutathione sepharose 4B was centrifuged for 5 minutes at 4˚C, 4900 $m/s^2$ (= 500G), and, after the elimination of supernatant, it was re-suspended in 200 mL of PBS solution at 4˚C. Centrifuging

was executed again for 5 minutes at 4°C, 4900 m/s$^2$ (= 500G), and the supernatant was eliminated. Then it was re-suspended in 5 mL of PBS solution at 4°C to obtain 50% slurry of glutathione sepharose 4B.

The entire amount of the supernatant prepared before was added to 10 mL of thus obtained 50% slurry of glutathione sepharose 4B, and the mixture was mildly shaken to cause the desired fused protein in the supernatant to be adsorbed by affinity onto glutathione sepharose 4B. The mixture was centrifuged for 5 minutes at 4°C, 4900 m/s$^2$ (= 500G), and, after the elimination of supernatant, it was re-suspended in 5 mL of PBS solution at 4°C, and subjected to similar centrifuging again and the supernatant was eliminated. The glutathione sepharose 4B on which GST-fused PHB synthesizing enzyme was adsorbed was rinsed by repeating resuspension in PBS solution and centrifuging twice, and was finally suspended in 5 mL of Cleavage buffer (Tris-HCl 50 mM, NaCl 150 mM, EDTA 1 mM, dithiothreitol 1 mM, pH 7). Then 0.5 mL of 4% solution of prescission protease (Amasham Pharmacia Biotech) in cleavage buffer was added, and the mixture was mildly shaken for 4 hours at 5°C. The mixture was centrifuged for 5 minutes at 4°C, 4900 m/s$^2$ (= 500G), and the supernatant was recovered. Then 1 mL of 50% slurry of glutathione sepharose 4B prepared as explained in the foregoing was centrifuged for 5 minutes at 4°C, 4900 m/s$^2$ (= 500G), and the above recovered supernatant was added to glutathione sepharose 4B after the elimination of supernatant, and the mixture was mildly agitated to cause glutation sepharose 4B to adsorb prescission protease remaining in the supernatant. Then centrifuging was executed for 5 minutes at 4°C, 4900 m/s$^2$ (= 500G), and, the supernatant was recovered. The supernatant had a single band by SDS-PAGE, indicating the purification.

The activity of the PHB synthesizing enzyme was measured in the following manner. At first bovine serum albumin (Sigma Co.) was dissolved in 0.1 M tris hydrochloric acid buffer (pH 8.0) in 3.0 mg/mL, and 100 μL of thus obtained solution was added to 100 μL of enzyme solution and the mixture was pre-incubated for 1 minute at 30°C. Then 100 μL of solution of 3-hydroxybutyryl CoA dissolved in 0.1 M tris hydrochloric acid buffer (pH 8.0) in 3.0 mM was added, then the mixture was incubated for 1 to 30 minutes at 30°C, and then the reaction is terminated by adding solution of trichloroacetic acid dissolved in 0.1 M tris hydrochloric acid buffer (pH 8.0) at 10 mg/mL. The solution after termination of reaction was centrifuged (147,000 m/s$^2$ (15,000 G), 10 minutes), and 500 μL of 2mM solution of 5,5'-dithiobis-(2-nitrobenzoic acid) dissolved in 0.1 M tris hydrochloric acid buffer (pH 8.0) was added to 500 μL of the supernatant. After incubation for 10 minutes at 30°C, the optical absorbance at 412 nm was measured. The enzyme activity was calculated by taking an enzyme amount causing release of CoA of 1 μmol in 1 minute as 1 unit (U). As a result there was obtained a relative activity of 7.5 U/mL. The obtained solution was concentrated by ultrafiltration under the addition of Reiho gel to 10 U/mL, thereby obtaining purified enzyme solution (1).

(Reference example 4) Preparation of crude enzyme solution containing PHB synthesizing enzyme

The KK01 and TL2 strains were cultured for 24 hours at 30°C in 10 liters of M9 culture medium (following composition) containing 0.5% of yeast extract and 0.3% of mineral solution (see following), and the recovered culture medium was centrifuged for 10 minutes at 4°C, 78000 m/s$^2$ (= 8000G), and, after the elimination of supernatant, the bacterial pellet was re-suspended in 500 mL of PBS solution at 4°C. The bacterial suspension was poured, 40 mL each time, in a vessel cooled to 4°C in advance, and, under pressurization of 2200 kg/cm$^2$ by a French press, the bacterial suspension was released little by little from the nozzle, thereby executing crushing process. The crushed bacterial suspension was centrifuged for 10 minutes at 4°C, 78000 m/s$^2$ (= 8000G), and the supernatant was recovered. The supernatant was filtered with a filter of 0.45 μm to eliminate the debris, and the activity of the PHB synthesizing enzyme was measured by the aforementioned method. As a result there were obtained relative activities of 1.6 U/mL for the KK01 strain and 1.2 U/mL for the TL2 strain. The solution was concentrated by ultrafiltration under the addition of a biological sample condenser (trade name: Mizubutorikun; Ato Co.) to 10 U/mL, thereby obtaining crude enzyme solution (1) derived from the KK01 strain and (2) derived from the TL2 strain.

| | |
|---|---|
| (M9 culture medium) | |
| $Na_2HPO_4$ | 6.2 g |
| $KH_2PO_4$ | 3.0 g |
| NaCl | 0.5 g |
| $NH_4Cl$ | 1.0 g |
| (in 1 liter of culture medium; pH 7.0) - | (Mineral solution) |
| nytrilotriacetic acid | 1.5 g; |
| $MgSO_4$ | 3.0 g; |
| $MnSO_4$. | 0.5 g; |
| NaCl | 1.0 g; |
| $FeSO_4$ | 0.1 g; |

(continued)

| | |
|---|---|
| $CaCl_2$ | 0.1 g; |
| $CoCl_2$ | 0.1 g; |
| $ZnSO_4$ | 0.1 g; |
| $CuSO_4$ | 0.1 g; |
| $AlK(SO_4)_2$ | 0.1 g; |
| $H_3BO_3$ | 0.1 g; |
| $Na_2MoO_4$ | 0.1 g; |
| $NiCl_2$ | 0.1 g; |
| (in 1 liter). | |

(Reference example 5) Preparation of transformant capable of producing PHA synthesizing enzyme

Transformant capable of producing PHA synthesizing enzyme was prepared in the following manner.

The YN2 strain was cultured overnight at 30˚C in 100 mL of LB culture medium (1% polypeptone (Nippon Pharmaceuticals), 0.5% yeast extract (Difco), 0.5% sodium chloride, pH 7.4), and the chromosomal DNA was separated and recovered by the method of Marmer et. al. The obtained chromosomal DNA was completely decomposed by restriction enzyme Hind III. Vector pUC18 was digested with restriction enzyme Hind III, and, after dephosphorylation process (Molecular Cloning, Vol. 1, p572 (1989): Cold Spring Harbor Laboratory), the digested site (cloning site) of the vector was ligated with the Hind III-decomposed fragments of the chromosomal DNA, utilizing a DNA ligation kit Ver. II (Takara Shuzo). Then Escherichia coli HB101 strain was transformed with the plasmid vector incorporating the ligated DNA fragment to obtain the DNA library of the YN2 strain.

Then there was prepared a probe for colony hybridization, for selecting the DNA fragment of the YN2 strain including the PHA synthesizing enzyme genes. Oligonucleotides of sequence number 3 and sequence number 4 were synthesized (Amasham Pharmacia Biotech), and PCR was executed utilizing such oligonucleotides as the primers and chromosomal DNA of the YN2 strain as the template. The DNA fragment obtained by PCR amplification was used as a probe. The probe was labeled with a commercial labeling kit AlkPhosDirect (Amasham Pharmacia Biotech Inc.).

The obtained labeled probe was used for colony hybridization method to select the Escherichia coli strain having a recombinant plasmid including the PHA synthesizing enzyme genes from the chromosomal DNA library of the YN2 strain. Plasmid was recovered by the alkali method from the selected strain to obtain a DNA fragment containing the PHA synthesizing enzyme genes.

The acquired DNA fragment was recombined to a vector pBBR 122(Mo Bi Tec) including a broad host range replication origin not belonging to the incompatibility group IncP, IncQ or IncW, and such recombinant plasmid was used to transform Pseudomonas chicorii YN2mL strain (lacking PHA synthesizing ability) by electroporation whereby the PHA synthesizing ability of the YN2mL strain was restored and complementary character was shown. Consequently it was confirmed that the selected DNA fragment includes a PHA synthesizing enzyme gene region that can be translated into the PHA synthesizing enzyme.

The base sequence of the DNA fragment was determined by Sanger method. As a result, it was confirmed that the determined base sequence contained sequences of a sequence number 5 and a sequence number 6 respectively coding peptide chains. PCR was executed on such PHA synthesizing enzyme genes utilizing the chromosomal DNA as the template to prepare the entire fragment containing the PHA synthesizing enzyme genes.

More specifically, an upstream primer (sequence number 7) and a downstream primer (sequence number 8) for the PHA synthesizing enzyme gene represented by the sequence number 5, and an upstream primer (sequence number 9) and a downstream primer (sequence number 10) for the PHA synthesizing enzyme gene represented by the sequence number 6 were respectively synthesized (Amasham Pharmacia Biotech). PCR was executed respectively for the base sequence of the number 5 and that of the number 6, utilizing such primers to amplify the entire fragment containing the PHA synthesizing enzyme genes (LA-PCR kit: Takara Shuzo).

Then the obtained PCR amplified fragments and the expression vector pTrc99A were digested with restriction enzyme Hind III, and, after dephosphorylation process (Molecular Cloning, Vol. 1, 5.7.2 (1989): Cold Spring Harbor Laboratory), the DNA fragments, including the entire region of the PHA synthesizing enzyme genes excluding the unnecessary base sequences on both ends, was ligated to the cloning site of the expression vector pTrc 99A by the DNA ligation kit Ver. II (Takara Shuzo).

Then Escherichia coli HB101 strain (Takara Shuzo) were transformed with the obtained recombinant plasmids by calcium chloride method (Takara Shuzo). The obtained recombinant plasmids were amplified by culture and were recovered. The recombinant plasmids having the gene of sequence number 5 and 6 were respectively named as pYN2-C1 and pYN2-C2. Escherichia coli HB101 fB, lacking fadB, was transformed with pYN2-C1, pNY2-C2 by the calcium

chloride method to obtain recombinant strains respectively having each recombinant plasmid.

(Reference example 6) Production 1 of PHA synthesizing enzyme

Oligonucleotide (sequence number 11) constituting an upstream primer for the pYN2-C1 and oligonucleotide (sequence number 12) constituting a downstream primer were designed and synthesized (Amasham Pharmacia Biotech). PCR was executed utilizing such oligonucleotides as the primers and pYN2-C1 as the template to amplify the entire fragment containing the PHA synthesizing enzyme gene having the BamHI restriction site at the upstream side and the XhoI restriction site at the downstream side (LA-PCR kit: Takara Shuzo).

Similarly, Oligonucleotide (sequence number 13) constituting an upstream primer for the pYN2-C2 and oligonucleotide (sequence number 14) constituting a downstream primer were designed and synthesized (Amasham Pharmacia Biotech). PCR was executed utilizing such oligonucleotides as the primers and pYN2-C2 as the template to amplify the entire fragment containing the PHA synthesizing enzyme gene having the BamHI restriction site at the upstream side and the XhoI restriction site at the downstream side (LA-PCR kit: Takara Shuzo).

The purified PCR amplification products were digested with BamHI and XhoI and were inserted in a corresponding site of a plasmid pGEX-6P-1 (Amasham Pharmacia Biotech Inc.). Escherichia coli JM109 strain was transformed with the recombinant plasmids to obtain a strain for the enzyme expression. The confirmation of the recombinant strain was executed by a DNA fragment obtained by digesting the plasmid DNA, prepared in large scale Miniprep . (wizard Minipreps DNA Purification System, PROMEGA Inc.) with BamHI and XhoI. The obtained recombinant strain was pre-cultured overnight at 30˚C in 10 mL of LB-Amp culture medium, and 0.1 mL of the culture medium was added to 10 mL of LB-Amp culture medium and shaking culture was executed for 3 hours at 37˚C, 170 rpm. Then IPTG was added (final concentration 1mM), and the culture was executed for 4 to 12 hours at 37˚C.

Escherichia coli induced by IPTG was collected (78000 $m/s^2$ (= 8000 G), 2 minutes, 4˚C), and was re-suspended in phosphate buffer physiological saline solution (PBS; 8g NaCl, 1.44 g $Na_2HPO_4$, 0.24 g $KH_2PO_4$, 0.2 g KCl, 1,000 mL purified water) of 1/10 amount, 4˚C. The bacterial cells were crushed by freeze/thawing and sonication, and the debris was eliminated by centrifuging (78000 $m/s^2$ (= 8000 G), 2 minutes, 4˚C). After the expression of the desired protein in the supernatant was confirmed by SDS-PAGE, the induced and expressed GST fused protein was purified with glutathione sepharose 4B (Amasham Pharmacia Biotech).

The glutathione sepharose used was subjected in advance to a treatment for suppressing non-specific adsorption. More specifically glutathione sepharose was rinsed three times with PBS of a same amount (78000 $m/s^2$ (= 8000 G), 2 minutes, 4˚C) and then processed for 1 hour at 4˚C by adding PBS containing 4% bovine serum albumin of a same amount. After the processing, it was rinsed twice with PBS of a same amount, and was re-suspended in PBS of a 1/2 amount.

40 μL of pre-processed glutathione sepharose was added to 1 mL of cell-free extract and was gently agitated at 4˚C. In this manner the fused proteins GST-YN2-C1 and GST-YN2-C2 were adsorbed by glutathione sepharose. After the adsorption, glutathione sepharose was recovered by centrifuging (78000 $m/s^2$ (= 8000 G), 2 minutes, 4˚C) and was rinsed with PBS of 400 μL. Then 40 μL of 10 mM glutathione was added and the mixture was agitated for 1 hour at 4˚C to dissolve out the adsorbed fused protein. After the recovery of the supernatant by centrifuging (78000 $m/s^2$ (= 8000 G), 2 minutes, 4˚C), dialysis was executed for PBS to purify the GST fused protein. A single band was confirmed by SDS-PAGE.

500 μg of each GST-fused protein was digested with Prescission protease (5 U, Amasham Pharmacia Biotech), and it was passed by glutathione sepharose to eliminate protease and GST. The flow-through fraction was further passed through a sephadex G200 column equilibrized with PBS to obtain the final products of the expression proteins YN2-C1 and YN2-C2. Single band was confirmed by SDS-PAGE respectively at 60.8 kDa and 61.5 kDa.

**[0229]** - Such enzyme was concentrated with a biological sample condenser (trade name: Mizubutorikun; Ato Co.) to obtain the purified enzyme solution of 10 U/mL.

The activity of each purified enzyme was measured by the aforementioned method. Also the protein concentration in the specimen was measured by a micro BCA protein analyzing reagent kit (Pierce Chemical Inc.). The measured activities of the purified enzymes are shown in Table 1.

Table 1

| Origin | Activity | Relative activity |
|---|---|---|
| Purified enzyme solution (2) pYN2-C1 | 2.1 U/mL | 4.1 U/mg protein |
| Purified enzyme solution (3) pYN2-C2 | 1.5 U/mL | 3.6 U/mg protein |

(Reference example 7) Production 2 of PHA synthesizing enzyme

The P91, H45, YN2 and P161 strains were inoculated in 200 mL of M9 culture medium containing 0.5% of yeast extract (Difco Inc.) and 0.1% of octanoic acid, and were subjected to shaking culture at 30˚C, 125 stroke/minute. After 24 hours, the bacterial cells were recovered by centrifuging (98000 m/s$^2$ (= 10000 G), 10 minutes, 4˚C), and rinsed by resuspending in 200 mL of 0.1M tris hydrochloric acid buffer (pH 8.0) and centrifuging again. The bacterial cells were re-suspended in 2.0 mL of 0.1M tris hydrochloric acid buffer (pH 8.0) and crushed with an ultrasonic crusher, then centrifuging (118000 m/s$^2$ (= 12000 G), 10 minutes, 4˚C) was executed to recover the supernatant, thereby obtaining crude enzyme solution.

The activity of each crude enzyme was measured by the aforementioned method, and the results are shown in Table 2.

Table 2

| | Origin | Activity |
|---|---|---|
| Crude enzyme solution(3) | P91 strain | 0.1 U/mL |
| Crude enzyme solution(4) | H45 strain | 0.2 U/mL |
| Crude enzyme solution(5) | YN2 strain | 0.4 U/mL |
| Crude enzyme solution(6) | P161 strain | 0.2 U/mL |

Such enzyme was concentrated with a biological sample condenser (trade name: Mizubutorikun; Ato Co.) to obtain the crude enzyme solution of 10 U/mL.

<Example 1>

The purified PHB synthesizing enzyme solution was employed in preparing the colorant of the present invention, utilizing phthalocyanine blue (C.I. Pigment Blue 15:3), in the following manner.

Phthalocyanine blue was dispersed by a sand mill to obtain a particle size not exceeding 0.1 μm, and 1 mass part thereof was added with 10 mass parts of the purified enzyme solution (1) and 39 mass parts of PBS, and mild shaking was executed for 30 minutes at 30˚C to cause the PHB synthesizing enzyme to be adsorbed on the pigment surface. Then centrifuging (98000 m/s$^2$ (= 10000 G), 10 minutes, 4˚C) was executed, then the precipitate was suspended in PBS solution and again centrifuged (98000 m/s$^2$ (= 10000 G), 10 minutes, 4˚C) to adsorb PHB synthesizing enzyme on phthalocyanine blue.

The aforementioned adsorbed enzyme was suspended in 48 mass parts of 0.1M phosphate buffer (pH 7.0), then added with 1 mass part of (R)-3-hydroxybutyryl CoA (Sigma Aldrich Japan Co.) and 0.1 mass parts of bovine serum albumin (Sigma Co.) and the mixture was mildly shaken for 2 hours at 30˚C. The generated blue microencapsulated pigment (hereinafter represented as colorant) was filtered, rinsed and dried to obtain colorant 1.

0.01 parts of the aforementioned reaction mixture was placed on a slide glass, and added and mixed with 0.01 parts of 1% Nile blue A aqueous solution on the slide glass, and the mixture was covered with a cover glass and was observed under a fluorescent microscope (330 to 380 nm excitation filter, 420 nm long-path absorption filter; Nikon Co.). As a result, fluorescence from the surface of the colorant 1 was confirmed. It was therefore identified that the surface of the colorant was covered with PHB. As a reference, 10 mass parts of phthalocyanine blue, added to 100 mass parts of 0.1M sodium phosphate buffer (pH 7.0), then mildly shaken for 2.5 hours at 30˚C and was observed under the fluorescent microscope with similar dyeing with Nile blue A. As a result, the surface of the reference phthalocyanine blue did not emit any fluorescence.

Also the colorant 1, after vacuum drying, was suspended in 20 mL of chloroform and agitated for 20 hours at 60˚C to extract PHB constituting the outer shell. The extract was filtered with a membrane filter of a pore size of 0.45 μm, then vacuum concentrated in a rotary evaporator, subjected to methanolysis in the ordinary manner and analyzed in a gas chromatography mass-spectrometer (GC-MS, Shimadzu QP-5050, EI method) to identify the methyl esterified substance of the PHB monomer unit. As a result, since the main peak in the obtained chromatogram showed a retention time same as that of a standard methylated compound of hydroxybutyric acid, the principal component of the outer shell of the obtained colorant 1 was confirmed as PHB.

Further, the molecular weight of PHB was evaluated by gel permeation chromatography (GPC: Toso HLC-8020, column: Polymer Laboratory PLgel MIXED-C (5 μm), solvent: chloroform, column temp.: 40˚C, converted as polystyrene) to obtain a result Mn = 75,000.

Also the volume-averaged particle size of the pigment before and after covering was measured by a laser

Doppler particle size distribution measuring apparatus (UPA-150: Nikkiso Co.). The particles sizes before and after were respectively 0.064 and 0.082 μm and it was presumed that the pigment was covered by PHB.

<Example 2>

The example 1 was reproduced except that the phthalocyanine pigment in the example 1 was replaced by carmine 6B (C.I. Pigment Red 57:1) and that the purified enzyme solution (1) was replaced by the crude enzyme solution (1), to obtain colorant 2.

Evaluation as in the example 1 confirmed the fluorescent emission from the surface of the colorant 2. It was therefore confirmed that the surface of the colorant 2 was covered with PHB. Also the analysis with gas chromatography mass-spectrometer confirmed that the principal component of the outer shell of the colorant 2 was PHB. Also the analysis by gel permeation chromatography indicated that the number-averaged molecular weight of the obtained colorant 2 was 73,000. Also the particle size before and after covering was respectively 0.071 and 0.086 μm.

<Example 3>

The example 1 was reproduced except that the phthalocyanine pigment in the example 1 was replaced by disazo yellow (C.I. Pigment Yellow 12) and that the purified enzyme solution (1) was replaced by the crude enzyme solution (2), to obtain colorant 3.

Evaluation as in the example 1 confirmed the fluorescent emission from the surface of the colorant 3. It was therefore confirmed that the surface of the colorant 3 was covered with PHB. Also the analysis with gas chromatography mass-spectrometer confirmed that the principal component of the outer shell of the colorant 3 was PHB. Also the analysis by gel permeation chromatography indicated that the number-averaged molecular weight of the obtained colorant 3 was 69,000. Also the particle size before and after covering was respectively 0.073 and 0.085 μm.

<Example 4>

The example 1 was reproduced except that the purified enzyme solution (1) was replaced by the purified enzyme solution (2) and that (R)-3-hydroxybutyryl CoA was replaced by (R)-3-hydroxyoctanoyl CoA (prepared by the method described in Eur. J. Biochem., 250, 432-439(1997)) to obtain colorant 4.

Evaluation as in the example 1 confirmed the fluorescent emission from the surface of the colorant 4. It was therefore confirmed that the surface of the colorant 4 was covered with PHA. Also the analysis with gas chromatography mass-spectrometer confirmed that the principal component of the outer shell of the colorant 4 was PHA consisting of 3-hydroxyoctanoic acid unit. Also the analysis by gel permeation chromatography indicated that the number-averaged molecular weight of the obtained colorant 4 was 27,000. Also the particle size before and after covering was respectively 0.064 and 0.080 μm.

<Example 5>

The example 4 was reproduced except that the phthalocyanine pigment in the example 4 was replaced by carmine 6B (C.I. Pigment Red 57:1) and that the purified enzyme solution (3) was replaced by the purified enzyme solution (3), to obtain colorant 5.

Evaluation as in the example 1 confirmed the fluorescent emission from the surface of the colorant 5. It was therefore confirmed that the surface of the colorant 5 was covered with PHA. Also the analysis with gas chromatography mass-spectrometer confirmed that the principal component of the outer shell of the colorant 5 was PHA consisting of 3-hydroxyoctanoic acid unit. Also the analysis by gel permeation chromatography indicated that the number-averaged molecular weight of the obtained colorant 5 was 24,000. Also the particle size before and after covering was respectively 0.071 and 0.085 μm.

<Example 6>

The example 4 was reproduced except that the phthalocyanine pigment in the example 4 was replaced by disazo yellow (C.I. Pigment Yellow 12) and that the purified enzyme solution (2) was replaced by the crude enzyme solution (3), to obtain colorant 6.

Evaluation as in the example 1 confirmed the fluorescent emission from the surface of the colorant 6. It was therefore confirmed that the surface of the colorant 6 was covered with PHA. Also the analysis with gas chromatography mass-spectrometer confirmed that the principal component of the outer shell of the colorant 6 was PHA consisting of 3-hydreoxyoctanoic acid unit. Also the analysis by gel permeation chromatography indicated that the number-averaged

molecular weight of the obtained colorant 6 was 25,000. Also the particle size before and after covering was respectively 0.073 and 0.088 μm.

<Example 7>

The example 4 was reproduced except that the purified enzyme solution (2) was replaced by the crude enzyme solution (4), to obtain colorant 7.

Evaluation as in the example 1 confirmed the fluorescent emission from the surface of the colorant 7. It was therefore confirmed that the surface of the colorant 7 was covered with PHA. Also the analysis with gas chromatography mass-spectrometer confirmed that the principal component of the outer shell of the colorant 7 was PHA consisting of 3-hydroxyoctanoic acid unit. Also the analysis by gel permeation chromatography indicated that the number-averaged molecular weight of the obtained colorant 7 was 24,000. Also the particle size before and after covering was respectively 0.064 and 0.079 μm.

<Example 8>

The example 4 was reproduced except that the phthalocyanine pigment in the example 4 was replaced by carmine 6B (C.I. Pigment Red 57:1) and that the purified enzyme solution (2) was replaced by the crude enzyme solution (5), to obtain colorant 8.

Evaluation as in the example 1 confirmed the fluorescent emission from the surface of the colorant 8. It was therefore confirmed that the surface of the colorant 8 was covered with PHA. Also the analysis with gas chromatography mass-spectrometer confirmed that the principal component of the outer shell of the colorant 8 was PHA consisting of 3-hydreoxyoctanoic acid unit. Also the analysis by gel permeation chromatography indicated that the number-averaged molecular weight of the obtained colorant 8 was 27,000. Also the particle size before and after covering was respectively 0.071 and 0.088 μm.

<Example 9>

The example 4 was reproduced except that the phthalocyanine pigment in the example 4 was replaced by disazo yellow (C.I. Pigment Yellow 12) and that the purified enzyme solution (2) was replaced by the crude enzyme solution (6), to obtain colorant 9.

Evaluation as in the example 1 confirmed the fluorescent emission from the surface of the colorant 9. It was therefore confirmed that the surface of the colorant 9 was covered with PHA. Also the analysis with gas chromatography mass-spectrometer confirmed that the principal component of the outer shell of the colorant 9 was PHA consisting of 3-hydroxyoctanoic acid unit. Also the analysis by gel permeation chromatography indicated that the number-averaged molecular weight of the obtained colorant 9 was 25,000. Also the particle size before and after covering was respectively 0.073 and 0.086 μm

<Example 10>

The example 4 was reproduced except that (R)-3-hydroxyoctanoyl CoA was replaced by (R)-3-hydroxy-5-phenylvaleryl CoA (prepared by hydrolyzing 3-hydroxy-5-phenylvaleric acid ester obtained by a Reformatsky reaction to obtain 3-hydroxy-5-phenylvaleric acid and then by the method described in Eur. J. Biochem., 250, 432-439(1997)) to obtain colorant 10.

Evaluation as in the example 1 confirmed the fluorescent emission from the surface of the colorant 10. It was therefore confirmed that the surface of the colorant 10 was covered with PHA. Also the analysis with gas chromatography mass-spectrometer confirmed that the principal component of the outer shell of the colorant 10 was PHA consisting of 3-hydroxy-5-phenoxylvaleric acid unit. Also the analysis by gel permeation chromatography indicated that the number-averaged molecular weight of the obtained colorant 10 was 27,000. Also the particle size before and after covering was respectively 0.064 and 0.081 μm.

<Example 11>

The example 4 was reproduced except that (R)-3-hydroxyoctanoyl CoA was replaced by (R,S)-3-hydroxy-5-phenoxylvaleryl CoA (prepared by starting from ethyl bromoacetate and 3-phenoxypropanal synthesized by the method described in J. Org. Chem., 55, 1490-1492(1990), then hydrolyzing 3-hydroxy-5-phenoxyvaleric acid ester obtained by a Reformatsky reaction to obtain 3-hydroxy-5-phenoxyvaleric acid and then by the method described in Eur. J. Biochem., 250, 432-439(1997)) to obtain colorant 11.

Evaluation as in the example 1 confirmed the fluorescent emission from the surface of the colorant 11. It was therefore confirmed that the surface of the colorant 11 was covered with PHA. Also the analysis with gas chromatography mass-spectrometer confirmed that the principal component of the outer shell of the colorant 11 was PHA consisting of 3-hydroxy-5-phenoxyvaleric acid unit. Also the analysis by gel permeation chromatography indicated that the number-averaged molecular weight of the obtained colorant 11 was 29,000. Also the particle size before and after covering was respectively 0.064 and 0.080 μm.

<Example 12>

The example 10 was reproduced to obtain colorant 10 covered with PHA consisting of 3-hydroxy-5-phenylvaleric acid unit.

Then the example 11 was reproduced except that the colorant 10 was used as a core, to obtain colorant 12.

The mass of the polymer formed on the surface of such capsule structure was measured with a flight time secondary ion mass spectrometer (TOF-SIMS IV, Cameca Inc.). Based on the obtained mass spectrum, it was confirmed that the PHA on the capsule surface consisted of 3-hydroxy-5-phenoxyvaleric acid unit. Also a similar mass spectrum measurement with TOF-SIMS under successive scraping of the capsule surface by ion sputtering, it was confirmed that the monomer unit of PHA constituting the capsule structure was replaced, at a certain point, by 3-hydroxy-5-phenylvaleric acid unit. This result confirmed that the capsule structure of the present example was a desired capsule structure in which poly(3-hydroxy-5-phenylvaleric acid) covering phthalocyanine blue (C.I. Pigment Blue 15:3) was covered by poly (3-hydroxy-5-phenoxyvaleric acid). Also the analysis by gel permeation chromatography indicated that the number-averaged molecular weight of the obtained colorant 12 was 23,000. Also the particle size before and after covering was respectively 0.064 and 0.096 μm.

<Example 13>

The example 4 was reproduced except that (R)-3-hydroxyoctanoyl CoA was replaced by 0.8 mass parts of (R, S)-3-hydroxy-5-phenylvaleryl CoA and 0.2 mass parts of (R,S)-3-hydroxy-7,8-epoxyoctanoyl CoA (prepared by epoxylating unsaturated portion of 3-hydroxy-7-octenoic acid, synthesized by the method described in Int. J. Biol. Macromol., 12, 85-91(1990), with 3-chlorobenzoic acid, and then by the method described in Eur. J. Biochem., 250, 432-439(1997) ) to obtain colorant 13.

Evaluation as in the example 1 confirmed the fluorescent emission from the surface of the colorant 13. It was therefore confirmed that the surface of the colorant 13 was covered with PHA. Also an analysis by $^1$H-NMR (FT-NMR: Bruker DPX400, measured nucleus: $^1$H, solvent: heavy chloroform (containing TMS)) indicated that the outer shell of the obtained colorant 13 consisted of PHA consisting of 3-hydroxy-5-phenylvaleric acid unit by 75% and 3-hydroxy-7,8-epoxyoctanoic acid unit by 25%. Also the analysis by gel permeation chromatography indicated that the number-averaged molecular weight of the obtained colorant 13 was 22,000. Also the particle size before and after covering was respectively 0.064 and 0.079 μm.

<Example 14>

On 50 mass parts of the aforementioned colorant 13, there were repeated three times a process of recovering by centrifuging (10,000 x g, 4˚C, 10 minutes) and suspension in 50 mass parts of purified water, and 0.5 mass parts of hexamethylene diamine were dissolved as a crosslinking agent in such suspension. After confirmation of dissolution, water was eliminated by lyophilizing, and reaction was executed for 12 hours at 70˚c to obtain colorant 14.

Infrared absorption measurement (FT-IR: Perkin-Elmer Inc., 1720X) on the colorant 14 indicated that the peaks of amine (about 3340 $cm^{-1}$) and epoxy (about 822 $cm^{-1}$) observed prior to heating vanished in the colorant 14. It was therefore identified that the reaction between PHA having an epoxy unit in the side chain with hexamethylene diamine provided the colorant 14 covered with the crosslinked polymer.

<Example 15>

On 50 mass parts of the aforementioned colorant 13, there were repeated three times a process of recovering by centrifuging (10,000 x g, 4˚C, 10 minutes) and suspension in 50 mass parts of purified water, and water was eliminated by lyophilizing. Then 10 mass parts of terminal amino-modified polysiloxane (modified silicone oil TSF4700, GE-Toshiba Silicone Co.) and reaction was executed for 2 hours at 70˚c. The product was rinsed by repeating suspension in methanol and centrifuging (10,000 x g, 4˚C, 20 minutes) and dried to obtain colorant 15 having polysiloxane graft chain.

Infrared absorption measurement (FT-IR: Perkin-Elmer Inc., 1720X) on the colorant 15 indicated that the peaks of amine (about 3340 $cm^{-1}$) and epoxy (about 822 $cm^{-1}$) observed prior to heating vanished in the colorant 15. It was

therefore identified that the reaction between PHA having an epoxy unit in the side chain with terminal amino-modified polysiloxane provided the colorant 15 having polysiloxane graft chain.

<Example 16>

Following composition:

styrene-butyl acrylate copolymer (glass transition temperature: 70˚C): 100 mass parts
colorant 1 (example 1): 5 mass parts
charge control agent (Hoechst NXVP 434): 2 mass parts was mixed and fusion kneaded in a two-axis extruder (L/D = 30). After cooling, there were executed crude crushing with a hammer mill, fine crushing with a jet mill and classification to obtain cyan colored particles (1), which shows a weight-averaged particle size of 7.1 μm and a fine powder amount of 6.0 number %.

100 mass parts of thus prepared cyan colored particles (1) were dry mixing by a Henshell mixer, with 1.5 mass parts of hydrophobic silica powder (BET: 250 $m^2/g$) treated with hexamethyl disilazane as the flowability improving agent, to obtain cyan toner (1) of the present example. Further, 7 mass parts of the cyan toner (1) and 93 mass parts of resin-coated magnetic ferrite carrier (average particle size 45 μm) to obtain two-component cyan developer (1) for magnetic brush development.

<Examples 17 to 24>

Cyan toners (2) to (9) of the examples 17 to 24 were obtained by a method similar to that in the example 16 except that the colorant 1 was respectively replaced by 5 mass parts of colorant 4, 7, 10 to 15. The characteristics of these toners were measured as in the example 16, and the results are shown in Table 3. Also these toners were used as in the example 16 to respectively obtain two-component cyan developers (2) to (9).

<Comparative example 1>

Cyan toner (10) of the comparative example 1 was obtained by a method similar to that of the example 16, except that the colorant 1 was replaced by 15 mass parts of phthalocyanine blue (C.I. Pigment Blue 15:3). The characteristics of such toner were measured as in the example 16, and the results are shown in Table 3. Also such toner was used as in the example 16 to obtain two-component cyan developer 10 of the comparative example 1.

<Examples 25 to 27>

Magenta toners (1) to (3) of the examples 25 to 27 were obtained by a method similar to that in the example 16 except that the colorant 1 was respectively replaced by 5 mass parts of the colorant 2, 5 and 8. The characteristics of these toners were measured as in the example 16, and the results are shown in Table 3. Also these toners were used as in the example 16 to respectively obtain two-component magenta developers (1) to (3).

<Comparative example 2>

Magenta toner (4) of the comparative example 2 was obtained by a method similar to that of the example 16, except that the colorant 1 was replaced by 5 mass parts of carmine 6B (C.I. Pigment Red 57:1). The characteristics of such toner were measured as in the example 16, and the results are shown in Table 3. Also such toner was used as in the example 16 to obtain two-component magenta developer (4) of the comparative example 2.

<Examples 28 to 30>

Yellow toners (1) to (3) of the examples 28 to 30 were obtained by a method similar to that in the example 16 except that the colorant 1 was respectively replaced by 5.0 mass parts of the colorant 3, 6 and 9. The characteristics of these toners were measured as in the example 16, and the results are shown in Table 3. Also these toners were used as in the example 16 to respectively obtain two-component yellows developers (1) to (3).

<Comparative example 3>

Yellow toner (4) of the comparative example 3 was obtained by a method similar to that of the example 16,

except that the colorant 1 was replaced by disazo yellow (C.I. Pigment Yellow 12). The characteristics of such toner were measured as in the example 16, and the results are shown in Table 3. Also such toner was used as in the example 16 to obtain two-component yellow developer (4) of the comparative example 3.

<Evaluation>

The two-component cyan developers (1) to (9), magenta and yellow developers (1) to (3) obtained in the examples 16 to 30, and the two-component cyan developer (10), magenta and yellow developers (4) obtained in the comparative examples 1 to 3 were subjected to the measurement of toner charge amount after agitation for 10 or 300 seconds, by the aforementioned charge amount measuring method, respectively under an environment of normal temperature and normal humidity (25˚C, 60 %RH) and an environment of high temperature and high humidity (30˚C, 80 %RH). The results are summarized in Table 3:

Table 3

| Example | Coloring agent No. | Toner No | Particle size distribution | | Charge amount (μC/g) | | | |
|---|---|---|---|---|---|---|---|---|
| | | | wt.ave. part.size (μm) | Fine powder amount (No.%) | Normal temp, Normal humidity (Q/M) | | High temp.high humidity (Q/M) | |
| | | | | | 10sec. agit. | 300sec agit. | 10sec agit. | 300sec agit. |
| 16 | 1 | blue 1 | 7.1 | 6.0 | -23.5 | -27.6 | -22.5 | -26.5 |
| 17 | 4 | blue 2 | 7.3 | 4.5 | -23.3 | -27.7 | -22.2 | -26.1 |
| 18 | 7 | blue 3 | 7.7 | 4.6 | -23.9 | -27.3 | -22.4 | -26.3 |
| 19 | 10 | blue 4 | 7.9 | 4.6 | -23.0 | -27.0 | -22.0 | -26.1 |
| 20 | 11 | blue 5 | 7.2 | 5.0 | -23.5 | -27.8 | -22.3 | -26.9 |
| 21 | 12 | blue 6 | 7.3 | 4.9 | -23.3 | -27.9 | -22.3 | -26.8 |
| 22 | 13 | blue 7 | 7.7 | 4.5 | -22.7 | -27.0 | -22.0 | -26.0 |
| 23 | 14 | blue 8 | 7.2 | 4.6 | -23.2 | -27.2 | -22.2 | -26.1 |
| 24 | 15 | blue 9 | 7.4 | 5.1 | -23.4 | -27.9 | -22.9 | -26.9 |
| 25 | 2 | red 1 | 7.1 | 5.6 | -23.3 | -27. | -22.1 | -26.3 |
| 26 | 5 | red 2 | 7.9 | 3.7 | -23.0 | -27.9 | -22.2 | -26.9 |
| 27 | 8 | red 3 | 7.6 | 4.9 | -23.2 | -27.3 | -22.1 | -26.6 |
| 28 | 3 | yellow 1 | 7.2 | 5.0 | -23.0 | -27.3 | -22.0 | -26.2 |
| 29 | 6 | yellow 2 | 7.5 | 4.7 | -23.1 | -27.2 | -22.1 | -26.3 |
| 30 | 9 | yellow 3 | 7.8 | 4.5 | -23.4 | -27.0 | -22.8 | -26.7 |
| Comp. ex.1 | - | blue 10 | 7.0 | 4.9 | -17.5 | -25.6 | -13.4 | -19.9 |
| 2 | - | red 4 | 7.3 | 5.1 | -21.5 | -26.6 | -19. 5 | -24.5 |
| 3 | - | yellow4 | 7.2 | 5.3 | -14.0 | -19.6 | -11.2 | -16.5 |

<Examples 31 to 45 and comparative examples 4 to 6>

At first there will be explained an image forming apparatus employed in the image forming method of the examples 31 to 45 and the comparative examples 4 to 6. Fig. 1 is a schematic cross-sectional view of an image forming apparatus for executing the image forming method of the examples of the present invention and the comparative examples. Referring to Fig. 1, a photosensitive drum 1 is provided with a photosensitive layer 1a containing organic photoconductor on a substrate 1b, is rendered rotatable in the direction of arrow, and is surfacially charged at a surface potential of about -600 V by a charging roller 2 constituting a charging member opposed to the photosensitive drum 1 and rotated by contact therewith. As shown in Fig. 1, the charging roller 2 is composed of a conductive elastic layer 2a

provided on a core metal 2b.

Then exposure 3 is made toward the surfacially charged photosensitive drum 1 in on-off mode according to the digital image information by a polygon mirror to form an electrostatic charge image of an exposed potential of -100 V and a dark potential of -600 v. Then the electrostatic charge image on the photosensitive drum 1 is reversal developed and rendered visible with plural developing devices 4-1, 4-2, 4-3, 4-4 to form a toner image on the photosensitive drum 1. In this operation, there were respectively employed the two-component developers obtained in the examples 16 to 30 and comparative examples 1 to 3 to form toner images with yellow, magenta and cyan toner. Fig. 2 is a magnified partial cross-sectional view of each developing device 4 for two-component developer, employed in the development. Then the toner image on the photosensitive drum 1 is transferred onto an intermediate transfer member 5 rotated in contact with the photosensitive drum 1. As a result, superposed visible images of four colors are formed on the intermediate transfer member 5. The remaining toner, not transferred and remaining on the photosensitive drum 1, is recovered by a cleaner member 8 into a remaining toner container 9.

As shown in Fig. 1, the intermediate transfer member 5 is composed of a metal core 5b constituting a support member, and an elastic layer 5a laminated thereon. In the present example, there was employed an intermediate transfer member 5 formed by coating a pipe-shaped core metal 5b with an elastic layer 5b composed of carbon black as conductivity providing material sufficiently dispersed in nitrile-butadiene rubber (NBR). The elastic layer 5b had a hardness of 30 degrees measured according to JIS K 6301, and a volume resistivity of $10^9\Omega$cm. The transfer current required for transfer from the photosensitive drum 1 to the intermediate transfer member 5 was about 5 $\mu$A and was obtained by providing the core metal 5b with a voltage of +500 V from a power source.

The superposed toner images of four colors formed on the intermediate transfer member 5 were transferred by a transfer roller 7 to a transfer material such as paper, and then fixed by a heat fixing device H. The transfer roller 7 was formed by coating, on a core metal 7b of an external diameter of 10 mm, an elastic layer 7b composed of carbon as conductivity providing material sufficiently dispersed in ethylene-propylene-diene three-dimensional foamed copolymer (EPDM). It had a volume resistivity of $10^6\Omega$cm and a hardness of 35 degrees measured according to JIS K 6301. The transfer roller 7 was given a voltage to obtain a transfer current of 15 $\mu$A.

In the apparatus shown in Fig. 1, the heat fixing device H was of heated roller type without oil coating mechanism as shown in Figs. 5 and 6. Both the upper and lower rollers were provided with surface layers of fluorinated resin. The rollers had a diameter of 60 mm. The fixing temperature was selected as 160˚C, and the nip width was selected as 7 mm. The toner remaining on the photosensitive drum 1 and recovered by cleaning was conveyed by a reuse mechanism to the developing device and was reused.

<Evaluation>

Printout test was conducted with the above-described configuration, under the environments of normal temperature and normal humidity (25˚C, 60 %RH) and high temperature and high humidity (30˚C, 80 %RH) and at a printout speed of 8 (A4 size) sheet/minute, respectively utilizing the two-component developers prepared with the toners of examples 16 to 30 and those prepared with the toners of comparative examples 1 to 3 under successive replenishment, in a monocolor intermittent mode (developing device being stopped for 10 seconds after each printout to accelerate the deterioration of the toner by the preparatory operation at the re-start), and the obtained printout image was evaluated for the following items. The results of evaluation are summarized in Table 4.

(Printout image evaluation)

1. Image density

Printouts of a predetermined number were made on ordinary copying plain paper (75 g/$m^2$), and there was evaluated the level of image density maintained in the image at the end of printing with respect to the initial image. The image density was measured with a Macbeth reflective densitometer (supplied by Macbeth Inc.), and the evaluation was made on the relative density of the printout image corresponding to a white portion of the original having a density of 0.00 by the following criteria:

◎: excellent (image density at end at least equal to 1.4)
O: good (image density at end at least equal to 1.35 but less than 1.40)
Δ: fair (image density at end at least equal to 1.00 but less than 1.35)
×: poor (image density at end less than 1.00).

2. Image fog

Printouts of a predetermined number were made on ordinary copying plain paper (75 g/$m^2$), and the evaluation was made by a solid white image at the end of the printouts. More specifically, the worst reflective density Ds of the white portion after printout, measured with a reflective densitometer (Reflectometer Model TC-6DS supplied by Tokyo Denshoku Co., Ltd.) and the average reflective density Dr of the sheet before printing were used to calculate (Ds - Dr) as the fog amount, which was evaluated according to the following criteria:

◎: excellent (fog amount at least equal to 0 % but less than 1.5 %)
○: good (fog amount at least equal to 1.5 % but less than 3.0 %)
Δ: fair (fog amount at least equal to 3.0 % but less than 5.0 %)
×: poor (fog amount at least equal to 5.0 %).

3. Transfer ability

A solid-black image was printed for a predetermined number on ordinary copying plain paper (75 g/$m^2$), and the amount of image lacking amount at the end of printouts was observed visually and evaluated according to the following criteria:

◎: excellent (almost none)
○: good (slight)
Δ practically acceptable
×: practically unacceptable

Also after image outputs of 5000 sheets in the examples 31 to 45 and comparative examples 4 to 6, the surfacial scars on the photosensitive drum and the intermediate transfer member, generation of fixing of the remaining toner and influence on the printout image (matching with image forming apparatus) were visually inspected. In the systems utilizing the two-component developers of both the examples 31 to 45 and the comparative example 4 to 6, the surfacial scars on the photosensitive drum and the intermediate transfer member and generation of fixing of the remaining toner were not at all observed, and the matching with image forming apparatus was very satisfactory.

Table 4

| Example/ Comp. Ex. | 2-comp developer | Normal temp/normal humidity | | | High temp/high humidity | | |
|---|---|---|---|---|---|---|---|
| | | Image density | Image fog | Transfer | Image density | Image fog | Transfer |
| Ex. 31 | blue 1 | ◎ | ◎ | ◎ | ◎ | ◎ | ◎ |
| 32 | blue 2 | ◎ | ◎ | ◎ | ◎ | ◎ | ◎ |
| 33 | blue 3 | ◎ | ◎ | ◎ | ◎ | ◎ | ◎ |
| 34 | blue 4 | ◎ | ◎ | ◎ | ◎ | ◎ | ○ |
| 35 | blue 5 | ◎ | ◎ | ◎ | ◎ | ◎ | ◎ |
| 36 | blue 6 | ◎ | ◎ | ◎ | ◎ | ◎ | ◎ |
| 37 | blue 7 | ◎ | ◎ | ◎ | ◎ | ◎ | ○ |
| 38 | blue 8 | ◎ | ◎ | ◎ | ◎ | ◎ | ◎ |
| 39 | blue 9 | ◎ | ◎ | ◎ | ◎ | ◎ | ◎ |
| 40 | red 1 | ◎ | ◎ | ◎ | ◎ | ◎ | ◎ |
| 41 | red 2 | ◎ | ◎ | ◎ | ◎ | ◎ | ◎ |
| 42 | red 3 | ◎ | ◎ | ◎ | ◎ | ◎ | ◎ |
| 43 | yellow 1 | ◎ | ◎ | ◎ | ◎ | ◎ | ◎ |
| 44 | yellow 2 | ◎ | ◎ | ◎ | ◎ | ◎ | ◎ |
| 45 | yellow 3 | ◎ | ◎ | ◎ | ◎ | ◎ | ◎ |

(continued)

| Example/ Comp. Ex. | 2-comp developer | Normal temp/normal humidity | | | High temp/high humidity | | |
|---|---|---|---|---|---|---|---|
| | | Image density | Image fog | Transfer | Image density | Image fog | Transfer |
| Comp. Ex. 4 | blue 10 | ◎ | ◎ | ◎ | ◎ | ○ | ○ |
| 5 | red 4 | ◎ | ◎ | ◎ | ◎ | ◎ | ◎ |
| 6 | yellow 4 | ◎ | ○ | ○ | ◎ | ○ | ○ |

<Examples 46 to 48 and comparative examples 7 to 9>

In the execution of the image forming method of the examples 46 to 48 and the comparative examples 7 to 9, there were respectively employed toners obtained in the examples 16, 25, 28 and the comparative examples 1 to 3 as the developer. Also as image forming means, there was employed an image forming apparatus obtained by modifying and resetting a commercially available laser beam printer LBP-EX (Canon Inc.) by mounting a reuse mechanism as shown in Fig. 3. More specifically, in the image forming apparatus shown in Fig. 3, there is provided a system for reusing the recovered toner, in which the untransferred toner remaining on the photosensitive drum 20 after the transfer is scraped off by an elastic blade 22 of a cleaner 21 maintained in contact with the photosensitive drum 20, then fed into the cleaner 21 by a cleaner roller, further conveyed by a cleaner reuse mechanism 23 and returned by a supply pipe 24 having a feed screw to a developing device 26 through a hopper 25.

In the image forming apparatus shown in Fig. 3, the photosensitive drum 20 is surfacially charged by a primary charging roller 27. The primary charging roller 27 was composed of a rubber roller (diameter 12 mm, contact pressure 50 g/cm) containing conductive carbon dispersed therein and covered with nylon resin. By laser exposure (600 dpi, not shown), an electrostatic latent image with a dark potential VD = -700 V and a light potential VL = -200 V was formed on the electrostatic latent image bearing member (photosensitive drum 20). The toner bearing member was composed of a developing sleeve 28 having a surface coarseness Ra of 1.1 and surfacially coated with resin containing carbon black dispersed therein.

Fig. 4 is a partial magnified cross-sectional view of a developing device for one-component developer, employed in the examples 46 to 48 and the comparative examples 7 to 9. As the developing conditions for the electrostatic latent image, the speed of the developing sleeve was selected as 1.1 times of the surface moving speed of the opposed photosensitive drum 20, and the gap $\alpha$ (S-D) between the photosensitive drum 20 and the developing sleeve 28 was selected as 270 $\mu$m. For regulating the toner layer thickness, an urethane rubber blade 29 was employed in contact state. Also the heat fixing device for fixing the toner image was set at a temperature of 160˚C. The employed fixing device was as shown in Figs. 5 and 6.

Printout test was conducted up to 30,000 prints with the above-described configuration, under the environments of normal temperature and normal humidity (25˚C, 60 %RH), at a printout speed of 8 (A4 size) sheet/minute and under successive toner replenishment, in a continuous mode (developing device being operated without stopping to accelerate the consumption of the toner), and the image density was measured on the obtained printout image and the durability of the image density was evaluated by the following criteria. Also the image of the 10,000th print was observed and the image fog was evaluated by the following criteria. Also, observation was made on the state of the components constituting the image forming apparatus after the durability test, and evaluation was made also on the matching between each component and each toner. The results of evaluation are summarized in Tab. 5.

(Image density change in durability test)

Printouts of a predetermined number were made on ordinary copying plain paper (75 g/m$^2$), and there was evaluated the level of image density maintained in the image at the end of printing with respect to the initial image. The image density was measured with a Macbeth reflective densitometer (supplied by Macbeth Inc.), and the evaluation was made on the relative density of the printout image corresponding to a white portion of the original having a density of 0.00 by the following criteria:

◎ : excellent (image density at end at least equal to 1.4)
○: good (image density at end at least equal to 1.35 but less than 1.40)
Δ: fair (image density at end at least equal to 1.00 but less than 1.35)
×: poor (image density at end less than 1.00).

2. Image fog

Printouts of a predetermined number were made on ordinary copying plain paper (75 g/$m^2$), and the evaluation was made by a solid white image at the end of the printouts. More specifically, the worst reflective density Ds of the white portion after printout, measured with a reflective densitometer (Reflectometer Model TC-6DS supplied by Tokyo Denshoku Co., Ltd.) and the average reflective density Dr of the sheet before printing were used to calculate (Ds - Dr) as the fog amount, which was evaluated according to the following criteria:

◎: excellent (fog amount at least equal to 0 % but less than 1.5 %)
○: good (fog amount at least equal to 1.5 % but less than 3.0 %)
Δ: fair (fog amount at least equal to 3.0 % but less than 5.0 %)
×: poor (fog amount at least equal to 5.0 %).

(Evaluation of matching with image forming apparatus)

1. Matching with developing sleeve

After the printout test, the state of fixation of the remaining toner to the developing sleeve surface and the influence thereof on the printout image were evaluated visually:

◎: excellent (none)
○ : good (almost none)
Δ : practically acceptable (toner fixation present but little influence on the image)
×: practically unacceptable (toner fixation present in a large amount to cause unevenness in the image).

2. Matching with photosensitive drum

The scars on the photosensitive drum surface, the state of fixation of the remaining toner thereto and the influence thereof on the printout image were evaluated visually:

◎: excellent (none)
○: good (slight scar generated but no influence on the image)
Δ: practically acceptable (toner fixation and scars present but little influence on the image)
×: practically unacceptable (toner fixation present in a large amount to cause image defects in streaks).

3. Matching with fixing device

The state of the surface of the fixing film was observed, and the durability thereof was evaluated in consideration of the surface state and the fixation of the remaining toner.

(1) Surface state

After the printout test, scars and peeling on the surface of the fixing film were visually observed and evaluated:

◎: excellent (none)
○: good (almost none)
Δ: practically acceptable
×: practically unacceptable.

(2) Fixation of remaining toner

After the printout test, the state of fixation of the remaining toner on the surface of the fixing film was visually observed and evaluated:

◎: excellent (none)
○: good (almost none)
Δ: practically acceptable
×: practically unacceptable.

Table 5

| Example | Toner | Printout image evaluation | | | | | Apparatus matching evaluation | | | |
|---|---|---|---|---|---|---|---|---|---|---|
| | | Image density change in durability test | | | | Image fog 10000 sheets | Developing sleeve | Photo-sensitive drum | Fixing device | |
| | | Initial | 1000 sheets | 10000 sheets | 30000 sheets | | | | Surface | Toner fix |
| Ex.46 | blue 1 | ◎ | ◎ | ◎ | ◎ | ◎ | ◎ | ◎ | ◎ | ◎ |
| 47 | red 1 | ◎ | ◎ | ◎ | ◎ | ◎ | ◎ | ◎ | ◎ | ◎ |
| 48 | yellow 1 | ◎ | ◎ | ◎ | ◎ | ◎ | ◎ | ◎ | ◎ | ◎ |
| Comp. ex.7 | blue 10 | ◎ | ◎ | ◎ | ○ | △ | ◎ | ○ | ◎ | ◎ |
| 8 | red 4 | ◎ | ◎ | ○ | ○ | ○ | ◎ | ○ | ◎ | ◎ |
| 9 | yellow 4 | ◎ | ◎ | ◎ | ○ | △ | ○ | ○ | ◎ | ◎ |

<Example 49>

Printout test was executed in the same manner as in the example 46, except that the toner reuse device was detached from the image forming apparatus shown in Fig. 3 and the printout speed was changed to 16 (A4 size) sheet/minute, under successive replenishment of the blue toner 1 of the example 16, in a continuous mode (developing device being operated without stopping to accelerate the consumption of the toner). The obtained printout image and the matching with the image forming apparatus were evaluated on the items same as those in the examples 46 to 48 and the comparative examples 7 to 9. Satisfactory results could be obtained in all the items.

## Claims

1. Electrostatic charge image developing toner comprising at least a colorant of which at least a part thereof is covered with polyhydroxyalkanoate constituting a first resin component, and binder resin constituting a second resin component.

2. Electrostatic charge image developing toner according to claim 1, wherein said colorant contains a pigment.

**3.** Electrostatic charge image developing toner according to claim 1 or 2, wherein said polyhydroxyalkanoate includes at least one selected from the group consisting of monomer units represented by the following formulas (1) to (10):

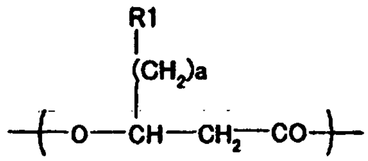

[1]

wherein the monomer unit is at least one selected from the group consisting of monomer units in which the combination of R1 and a is any of the following:

a monomer unit in which R1 is a hydrogen atom (H) and a is an integer from 0 to 10;
a monomer unit in which R1 is a halogen atom and a is an integer from 1 to 10;
a monomer unit in which R1 is a chromophore and a is an integer from 1 to 10;
a monomer unit in which R1 is a carboxyl group or a salt thereof and a is an integer from 1 to 10; and
a monomer unit in which R1 is a group represented by the following formula:

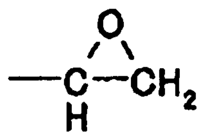

and a is an integer from 1 to 7;

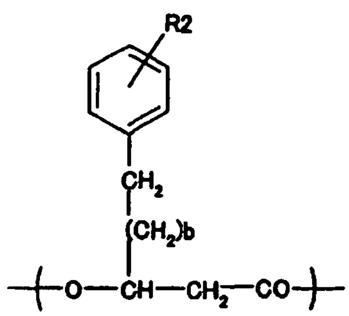

[2]

wherein b is an integer from 0 to 7, and R2 is a substitution selected from the group consisting of a hydrogen atom (H), a halogen atom, -CN, $-NO_2$, $-CF_3$, $-C_2F_5$ and $-C_3F_7$;

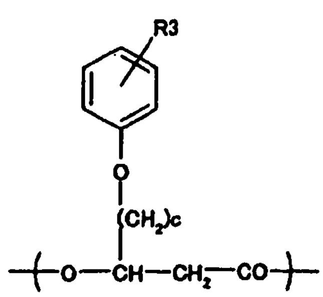

[3]

wherein c is an integer from 1 to 8, and R3 is a substitution selected from the group consisting of a hydrogen atom (H), a halogen atom, -CN, $-NO_2$, $-CF_3$, $-C_2F_5$ and $-C_3F_7$;

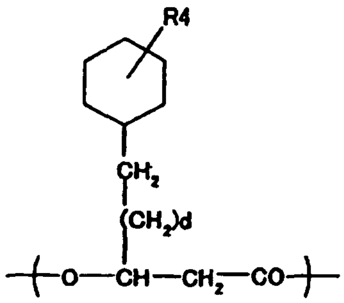

[4]

wherein d is an integer from 0 to 7, and R4 is a substitution selected from the group consisting of a hydrogen atom (H), a halogen atom, -CN, $-NO_2$, $-CF_3$, $-C_2F_5$ and $-C_3F_7$;

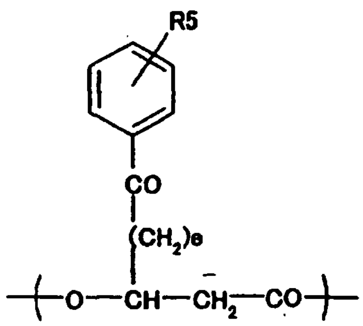

[5]

wherein e is an integer from 1 to 8, and R5 is a substitution selected from the group consisting of a hydrogen atom (H), a halogen atom, -CN, $-NO_2$, $-CF_3$, $-C_2F_5$, $-C_3F_7$, $-CH_3$, $-C_2H_5$ and $-C_3H_7$;

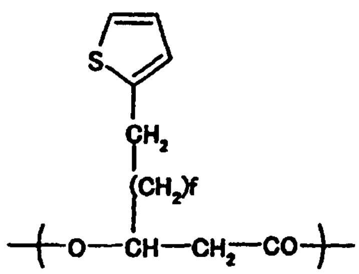

[6]

wherein f is an integer from 0 to 7;

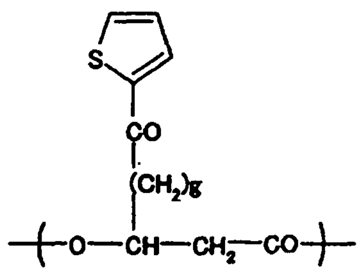

[7]

wherein g is an integer from 1 to 8;

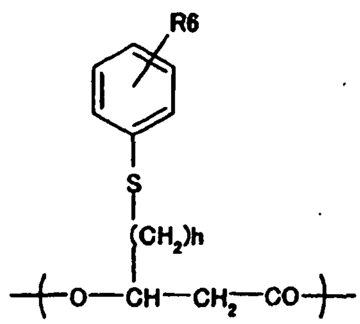

[8]

wherein h is an integer from 1 to 7, R6 is a substitution selected from the group consisting of a hydrogen atom (H), a halogen atom, -CN, $-NO_2$, -COOR', $-SO_2R''$, $-CH_3$, $-C_2H_5$, $-C_3H_7$, $-CH(CH_3)_2$ and $-C(CH_3)_3$, R' is a hydrogen atom, Na, K, $-CH_3$ or $-C_2H_5$, and R'' is -OH, -ONa, -OK, a halogen atom, $-OCH_3$ or $-OC_2H_5$;

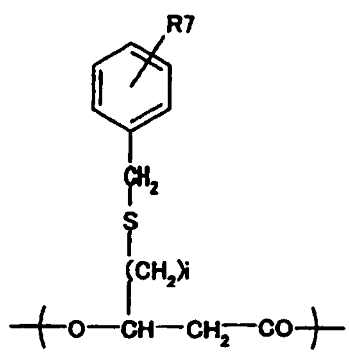

[9]

wherein i is an integer from 1 to 7, R7 is a substitution selected from the group consisting of a hydrogen atom (H), a halogen atom, -CN, $-NO_2$, -COOR' and $-SO_2R''$, R' is a hydrogen atom, Na, K, $-CH_3$ or $-C_2H_5$, and R'' is -OH, -ONa, -OK, a halogen atom, $-OCH_3$ or $-OC_2H_5$; and

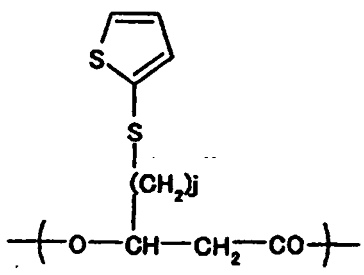

[10]

wherein j is an integer from 1 to 9.

**4.** Electrostatic charge image developing toner according to any of claims 1 to 3, wherein said polyhydroxyalkanoate has a number average molecular weight within a range from 1,000 to 10,000,000.

**5.** Electrostatic charge image developing toner according to any of claims 1 to 4, wherein the monomer unit composition of said polyhydroxyalkanoate changes in a direction from the inner side to the outer side of said colorant.

**6.** Electrostatic charge image developing toner according to any of claims 1 to 5, wherein at least a part of said polyhydroxyalkanoate is chemically modified polyhydroxyalkanoate.

**7.** Electrostatic charge image developing toner according to claim 6, wherein said chemically modified polyhydroxyalkanoate includes at least a graft chain.

**8.** Electrostatic charge image developing toner according to claim 7, wherein said graft chain is a graft chain formed by chemical modification of polyhydroxyalkanoate at least including a monomer unit having an epoxy group.

**9.** Electrostatic charge image developing toner according to claim 7 or 8, wherein said graft chain is a graft chain of a compound having an amino group.

**10.** Electrostatic charge image developing toner according to claim 9, wherein said compound having amino group is a terminal amino-modified compound.

**11.** Electrostatic charge image developing toner according to claim 6, wherein at least a part of said polyhydroxyalkanoate is crosslinked polyhydroxyalkanoate.

**12.** Electrostatic charge image developing toner according to claim 11, wherein said crosslinked polyhydroxyalkanoate is crosslinked from polyhydroxy alkanoate at least including a monomer unit having an epoxy group.

**13.** An image forming method including at least a step of externally applying a voltage to a charging member thereby charging an electrostatic latent image bearing member, a step of forming an electrostatic charge image on the charged electrostatic latent image bearing member, a development step of developing the electrostatic charge image with electrostatic charge image developing toner thereby forming a toner image on the electrostatic latent image bearing member, a transfer step of transferring the toner image on the electrostatic latent image bearing member onto a recording material, and a fixation step of heat fixing the toner image on the recording material, the method comprising the use of the electrostatic charge image developing toner according to any of claims 1 to 12.

**14.** An image forming method according to claim 13, including at least a step of externally applying a voltage to a charging member thereby charging an electrostatic latent image bearing member, a step of forming an electrostatic charge image on the charged electrostatic latent image bearing member, a development step of developing the electrostatic charge image with electrostatic charge image developing toner thereby forming a toner image on the electrostatic latent image bearing member, a first transfer step of transferring the toner image on the electrostatic latent image bearing member onto an intermediate transfer member, a second transfer step of transferring the toner image on the intermediate transfer member onto a recording material, and a fixation step of heat fixing the toner image on the recording material, the method comprising the use of the electrostatic charge image developing toner according to any of claims 1 to 12.

15. An image forming apparatus at least including means for externally applying a voltage to a charging member thereby charging an electrostatic latent image bearing member, means for forming an electrostatic charge image on the charged electrostatic latent image bearing member, development means containing the electrostatic charge image developing toner according to any of claims 1 to 12 for developing the electrostatic charge image with electrostatic charge image developing toner thereby forming a toner image on the electrostatic latent image bearing member, transfer means for transferring the toner image on the electrostatic latent image bearing member onto a recording material, and fixation means for heat fixing the toner image on the recording material.

16. An image forming apparatus according to claim 15, at least including means for externally applying a voltage to a charging member thereby charging an electrostatic latent image bearing member, means for forming an electrostatic charge image on the charged electrostatic latent image bearing member, development means containing the electrostatic charge image developing toner according to any of claims 1 to 12 for developing the electrostatic charge image with electrostatic charge image developing toner thereby forming a toner image on the electrostatic latent image bearing member, first transfer means for transferring the toner image on the electrostatic latent image bearing member onto an intermediate transfer member, second transfer means for transferring the toner image on the intermediate transfer member onto a recording material, and fixation means for heat fixing the toner image on the recording material.

17. A method for producing electrostatic charge image developing toner including a colorant obtained by covering at least a part of the surface of a coloring agent with polyhydroxyalkanoate constituting a first resin component, the method comprising execution of a polyhydroxyalkanoate synthesizing reaction utilizing 3-hydroxyacyl CoA as the substrate in the presence of a polyhydroxyalkanoate synthesizing enzyme fixed on the surface of a coloring agent dispersed in aqueous medium to cover at least a part of the surface of said coloring agent with polyhydroxyalkanoate thereby producing said colorant.

18. A method for producing electrostatic charge image developing toner according to claim 17, wherein said polyhydroxyalkanoate includes at least one selected from the group consisting of monomer units represented by the following formulas (1) to (10), and the respectively corresponding 3-hydroxyacyl coenzyme A is any of those represented by the chemical formulas (11) to (20):

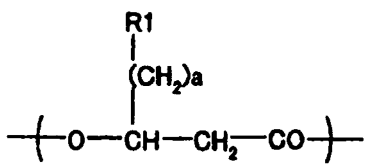

[1]

wherein the monomer unit is at least one selected from the group consisting of monomer units in which the combination of R1 and a is any of the following:

a monomer unit in which R1 is a hydrogen atom (H) and a is an integer from 0 to 10;
a monomer unit in which R1 is a halogen atom and a is an integer from 1 to 10;
a monomer unit in which R1 is a chromophore and a is an integer from 1 to 10;
a monomer unit in which R1 is a carboxyl group or a salt thereof and a is an integer from 1 to 10; and
a monomer unit in which R1 is a group represented by the following formula:

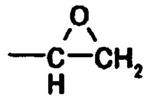

and a is an integer from 1 to 7;

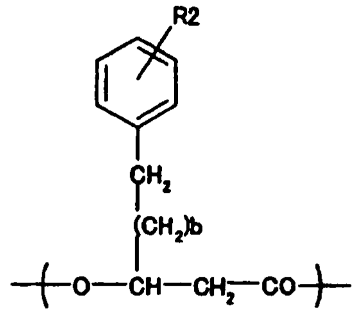

[2]

wherein b is an integer from 0 to 7, and R2 is a substitution selected from the group consisting of a hydrogen atom (H), a halogen atom, -CN, $-NO_2$, $-CF_3$, $-C_2F_5$ and $-C_3F_7$;

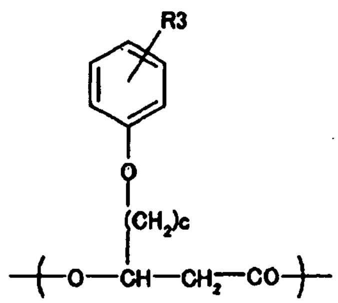

[3]

wherein c is an integer from 1 to 8, and R3 is a substitution selected from the group consisting of a hydrogen atom (H), a halogen atom, -CN, $-NO_2$, $-CF_3$, $-C_2F_5$ and $-C_3F_7$;

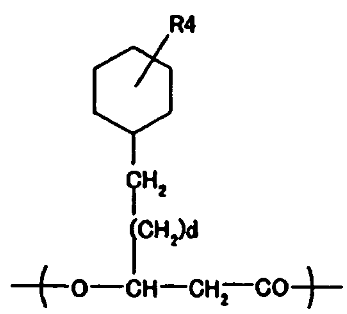

[4]

wherein d is an integer from 0 to 7, and R4 is a substituted selected from the group consisting of a hydrogen atom (H), a halogen atom, -CN, $-NO_2$, $-CF_3$, $-C_2F_5$ and $-C_3F_7$;

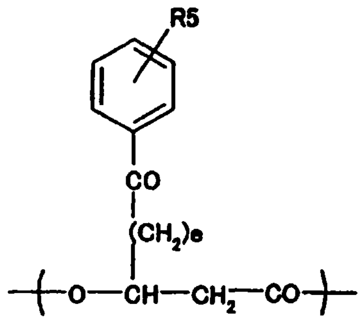

[5]

wherein e is an integer from 1 to 8, and R5 is a substitution selected from the group consisting of a hydrogen atom (H), a halogen atom, -CN, $-NO_2$, $-CF_3$, $-C_2F_5$, $-C_3F_7$, $-CH_3$, $-C_2H_5$ and $-C_3H_7$;

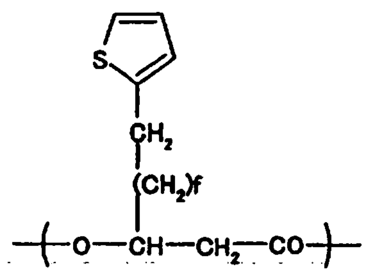

[6]

wherein f is an integer from 0 to 7;

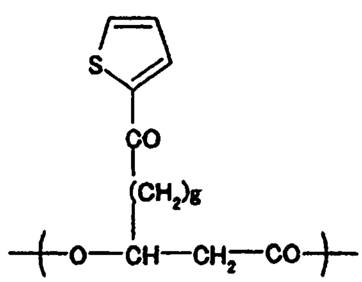

[7]

wherein g is an integer from 1 to 8;

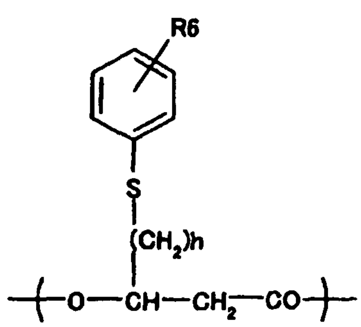

[8]

wherein h is an integer from 1 to 7, R6 is a substitution selected from the group consisting of a hydrogen atom (H), a halogen atom, -CN, $-NO_2$, -COOR', $-SO_2R''$, $-CH_3$, $-C_2H_5$, $-C_3H_7$, $-CH(CH_3)_2$ and $-C(CH_3)_3$, R' is a hydrogen atom, Na, K, $-CH_3$ or $-C_2H_5$, and R" is -OH, -ONa, -OK, a halogen atom, $-OCH_3$ or $-OC_2H_5$;

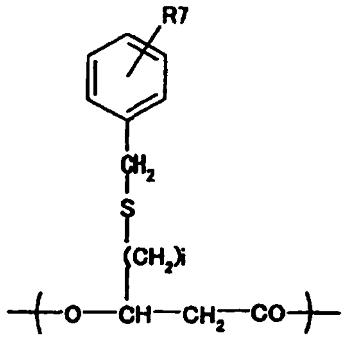

[9]

wherein i is an integer from 1 to 7, R7 is a substitution selected from the group consisting of a hydrogen atom (H), a halogen atom, -CN, $-NO_2$, -COOR' and $-SO_2R''$, R' is a hydrogen atom, Na, K, $-CH_3$ or $-C_2H_5$, and R" is -OH, -ONa, -OK, a halogen atom, $-OCH_3$ or $-OC_2H_5$;

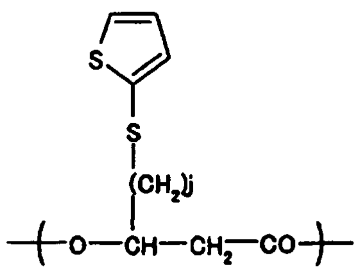

[10]

wherein j is an integer from 1 to 9;

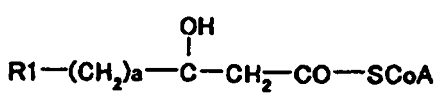

[11]

wherein -SCoA indicates coenzyme A bonded to alkanoic acid, and the combination of R1 and a is any of the following and corresponds to the combination of R1 and a in the foregoing chemical formula (1):

a monomer unit in which R1 is a hydrogen atom (H) and a is an integer 0 to 10;
a monomer unit in which R1 is a halogen atom and a is an integer from 1 to 10;
a monomer unit in which R1 is a chromophore and a is an integer from 1 to 10;
a monomer unit in which R1 is a carboxyl group or a salt thereof and a is an integer from 1 to 10; and
a monomer unit in which R1 is a group represented by the following formula;

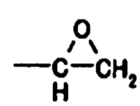

and a is an integer from 1 to 7;

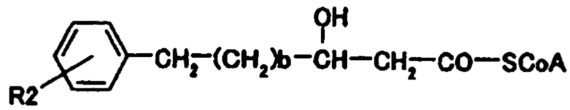

[12]

wherein -SCoA indicates coenzyme A bonded to alkanoic acid, b is an integer from 0 to 7 corresponding to b in the foregoing chemical formula (2), and R2 is a substitution selected from the group consisting of a hydrogen atom (H), a halogen atom, -CN, $-NO_2$, $-CF_3$, $-C_2F_5$ and $-C_3F_7$, corresponding to R2 in the foregoing chemical formula (2);

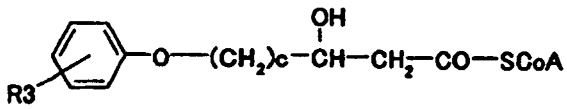

[13]

wherein -SCoA indicates coenzyme A bonded to alkanoic acid, c is an integer from 1 to 8 corresponding to c in the foregoing chemical formula (3), and R3 is a substitution selected from the group consisting of a hydrogen atom (H), a halogen atom, -CN, $-NO_2$, $-CF_3$, $-C_2F_5$ and $-C_3F_7$, corresponding to R3 in the foregoing chemical formula (3);

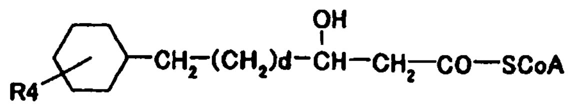

[14]

wherein -SCoA indicates coenzyme A bonded to alkanoic acid, d is an integer from 0 to 7 corresponding to d in the foregoing chemical formula (4), and R4 is a substitution selected from the group consisting of a hydrogen atom (H), a halogen atom, -CN, $-NO_2$, $-CF_3$, $-C_2F_5$ and $-C_3F_7$, corresponding to R4 in the foregoing chemical formula (4);

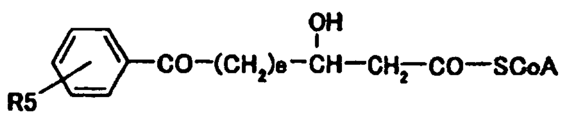

[15]

wherein -SCoA indicates coenzyme A bonded to alkanoic acid, e is an integer from 1 to 8 corresponding to e in the foregoing chemical formula (5), and R5 is a substitution selected from the group consisting of a hydrogen atom (H), a halogen atom, -CN, $-NO_2$, $-CF_3$, $-C_2F_5$, $-C_3F_7$, $-CH_3$, $-C_2H_5$ and $-C_3H_7$, corresponding to R5 in the foregoing chemical formula (5);

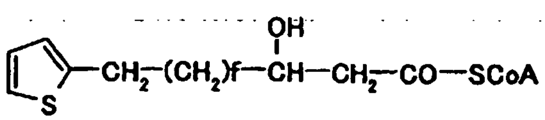

[16]

wherein -SCoA indicates coenzyme A bonded to alkanoic acid, and f is an integer from 0 to 7 corresponding to f in the foregoing chemical formula (6);

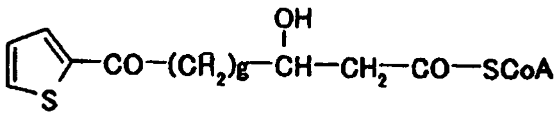

[17]

wherein -SCoA indicates coenzyme A bonded to alkanoic acid, and g is an integer from 1 to 8 corresponding to g in the foregoing chemical formula (7);

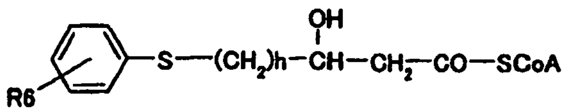

[18]

wherein -SCoA indicates coenzyme A bonded to alkanoic acid, h is an integer from 1 to 7 corresponding to h in the foregoing chemical formula (8), R6 is a substitution selected from the group consisting of a hydrogen atom (H), a halogen atom, -CN, $-NO_2$, -COOR', $-SO_2R''$, $-CH_3$, $-C_2H_5$, $-C_3H_7$, $-CH(CH_3)_2$ and $-C(CH_3)_3$ corresponding to R6 in the foregoing chemical formula (8), R' is a hydrogen atom, Na, K, $-CH_3$ or $-C_2H_5$, and R" is -OH, -ONa, -OK, a halogen atom, $-OCH_3$ or $-OC_2H_5$;

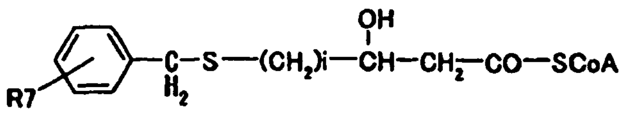

[19]

wherein -SCoA indicates coenzyme A bonded to alkanoic acid, i is an integer from 1 to 7 corresponding to i in the foregoing chemical formula (9), R7 is a substitution selected from the group consisting of a hydrogen atom (H), a halogen atom, -CN, $-NO_2$, -COOR' and $-SO_2R''$ corresponding to R7 in the foregoing chemical formula (9), R' is a hydrogen atom, Na, K, $-CH_3$ or $-C_2H_5$, and R" is -OH, -ONa, -OK, a halogen atom, $-OCH_3$ or $-OC_2H_5$; and

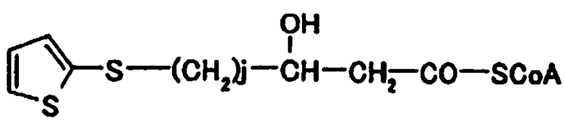

[20]

wherein -SCoA indicates coenzyme A bonded to alkanoic acid, and j is an integer of 1 to 9 corresponding to the foregoing chemical formula (10).

**19.** A method for producing electrostatic charge image developing toner according to claim 17 or 18, wherein the composition of said 3-hydroxyacyl coenzyme A is changed in time to change the 3-hydroxyalkanoic acid unit composition of said polyhydroxyalkanoate in a direction from the inner side to the outer side of said colorant.

**20.** A method for producing electrostatic charge image developing toner according to any of claims 17 to 19, further comprising a step of applying chemical modification in at least a part of polyhydroxyalkanoate covering said colorant.

**21.** A method for producing electrostatic charge image developing toner according to claim 20, wherein said step of applying chemical modification is a step of adding a graft chain in at least a part of polyhydroxyalkanoate.

**22.** A method for producing electrostatic charge image developing toner according to claim 21, wherein the step of adding said graft chain is a step of reacting at least a part of polyhydroxyalkanoate with a compound having a

reactive functional group at the end.

**23.** A method for producing electrostatic charge image developing toner according to claim 20, wherein the step of applying said chemical modification is a step of crosslinking at least a part of polyhydroxyalkanoate.

**24.** A method for producing electrostatic charge image developing toner according to claim 23, wherein said crosslinking step is a step of irradiating polyhydroxyalkanoate with an electron beam.

**25.** A method for producing electrostatic charge image developing toner according to any of claims 17 to 19, wherein said polyhydroxyalkanoate synthesizing enzyme is a polyhydroxyalkanoate synthesizing enzyme produced by microorganisms having ability of producing said enzyme, or a transformant obtained by introducing a gene relating to said producing ability into host microorganisms.

**26.** A method for producing electrostatic charge image developing toner according to claim 25, wherein said microorganisms having the ability of producing said polyhydroxyalkanoate synthesizing enzyme are those of Pseudomonas sp.

**27.** A method for producing electrostatic charge image developing toner according to claim 25, wherein said microorganisms having the ability of producing said polyhydroxyalkanoate synthesizing enzyme are those of Burkholderia sp.

**28.** A method for producing electrostatic charge image developing toner according to claim 25, wherein said microorganisms having the ability of producing said polyhydroxyalkanoate synthesizing enzyme are those of Alcaligenes sp.

**29.** A method for producing electrostatic charge image developing toner according to claim 25, wherein said microorganisms having the ability of producing said polyhydroxyalkanoate synthesizing enzyme are those of Ralstonia sp.

**30.** A method for producing electrostatic charge image developing toner according to claim 25, wherein the host microorganisms for the transformant having the ability for producing said polyhydroxyalkanoate synthesizing enzyme are Escherichia coli.

**Patentansprüche**

**1.** Ein elektrostatisches Ladungsbild entwickelnder Toner, umfassend zumindest ein färbendes Mittel, von dem zumindest ein Teil überzogen ist mit Polyhydroxyalkanoat, das eine erste Harzkomponente bildet, und ein Bindeharz, das eine zweite Harzkomponente bildet.

**2.** Toner nach Anspruch 1, wobei
das färbende Mittel ein Pigment enthält.

**3.** Toner nach Anspruch 1 oder 2, wobei
das Polyhydroxyalkanoat zumindest eines einschließt, das ausgewählt ist aus der Gruppe, bestehend aus Monomereinheiten der folgenden Formeln (1) bis (10):

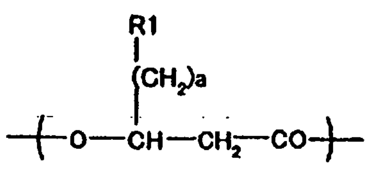

[1]

wobei die Monomereinheit zumindest eine ist, die ausgewählt ist aus der Gruppe, bestehend aus Monomereinheiten, bei denen die Kombination von R1 und a eine der folgenden ist:

eine Monomereinheit, bei der R1 ein Wasserstoffatom (H) ist, und a eine ganze Zahl von 0 bis 10;
eine Monomereinheit, bei der R1 ein Halogenatom ist, und a eine ganze Zahl von 0 bis 10;

eine Monomereinheit, bei der R1 ein Chromophor ist, und a eine ganze Zahl von 0 bis 10;
eine Monomereinheit, bei der R1 eine Carboxylgruppe oder ein Salz davon ist, und a eine ganze Zahl von 0 bis 10; und
eine Monomereinheit, bei der R1 eine Gruppe der folgenden Formel:

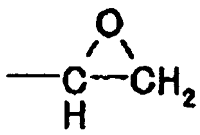

ist, und a eine ganze Zahl von 1 bis 7;

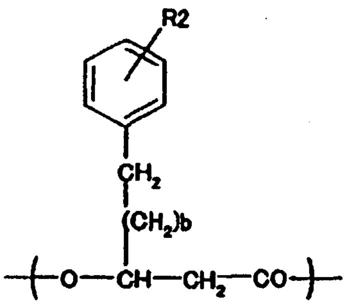

[2]

worin b eine ganze Zahl von 0 bis 7 ist und R2 ein Substituent ist, der ausgewählt ist aus der Gruppe, bestehend aus einem Wasserstoffatom (H), einem Halogenatom, -CN, $-NO_2$, $-CF_3$, $-C_2F_5$ und $-C_3F_7$;

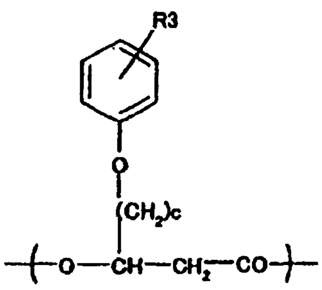

[3]

worin c eine ganze Zahl von 1 bis 8 ist und R3 ein Substituent ist, der ausgewählt ist aus der Gruppe, bestehend aus einem Wasserstoffatom (H), einem Halogenatom, -CN, $-NO_2$, $-CF_3$, $-C_2F_5$ und $-C_3F_7$;

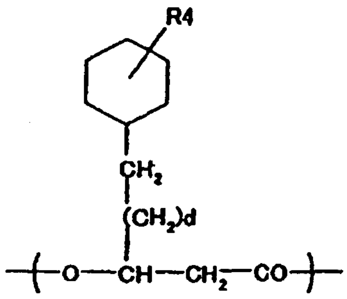

[4]

worin d eine ganze Zahl von 0 bis 7 ist und R4 ein Substituent ist, der ausgewählt ist aus der Gruppe, bestehend aus einem Wasserstoffatom (H), einem Halogenatom, -CN, $-NO_2$, $-CF_3$, $-C_2F_5$ und $-C_3F_7$;

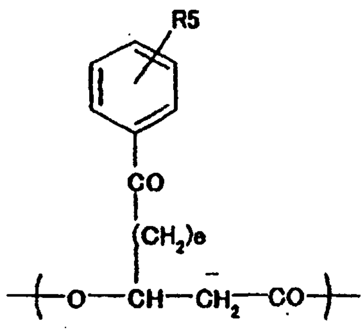

[5]

worin e eine ganze Zahl von 1 bis 8 ist und R5 ein Substituent ist, der ausgewählt ist aus der Gruppe, bestehend aus einem Wasserstoffatom (H), einem Halogenatom, -CN, $-NO_2$, $-CF_3$, $-C_2F_5$,$-C_3F_7$ , $-CH_3$, $-C_2H_5$ und $-C_3H_7$;

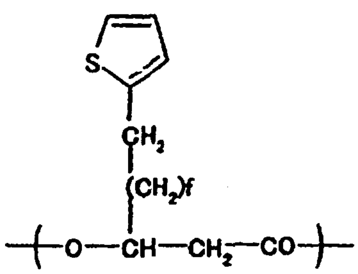

[6]

worin f eine ganze Zahl von 0 bis 7 ist;

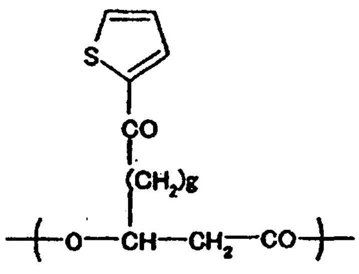

[7]

worin g eine ganze Zahl von 1 bis 8 ist;

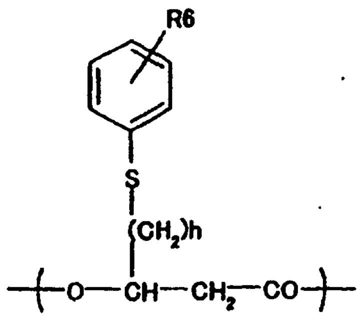

[8]

worin h eine ganze Zahl von 1 bis 7 ist und R6 ein Substituent ist, der ausgewählt ist aus der Gruppe, bestehend aus einem Wasserstoffatom (H), einem Halogenatom, -CN, $-NO_2$, -COOR', $-SO_2R''$, $-CH_3$, $-C_2H_5$, $-C_3H_7$, $-CH(CH_3)_2$ und $-C(CH_3)_3$, worin ferner R' ein Wasserstoffatom, Na, K, $-CH_3$ oder $-C_2H_5$ ist und worin R'' gleich -OH, -ONa, -OK, ein Halogenatom, $-OCH_3$ oder $-OC_2H_5$ ist;

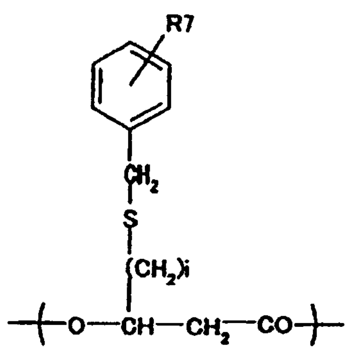

[9]

worin i eine ganze Zahl von 1 bis 7 ist und R7 ein Substituent ist, der ausgewählt ist aus der Gruppe, bestehend aus einem Wasserstoffatom (H), einem Halogenatom, -CN, $-NO_2$, -COOR' und $-SO_2R''$, worin ferner R' ein Wasserstoffatom, Na, K, $-CH_3$ oder $-C_2H_5$ ist und worin R'' gleich -OH, -ONa, -OK, ein Halogenatom, $-OCH_3$ oder $-OC_2H_5$ ist; und

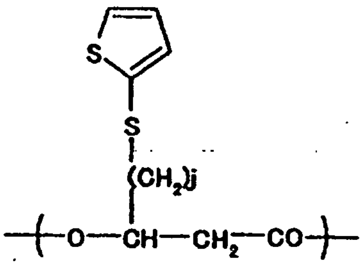

[10]

worin j eine ganze Zahl von 1 bis 9 ist.

**4.** Toner nach einem der Ansprüche 1 bis 3, wobei das Polyhydroxyalkanoat ein Zahlenmittel des Molekulargewichts im Bereich von 1.000 bis 10.000.000 hat.

**5.** Toner nach einem der Ansprüche 1 bis 4, wobei sich die Zusammensetzung der Monomereinheiten des Polyhydroxyalkanoats in einer Richtung von der Innenseite zur Außenseite des färbenden Mittels ändert.

**6.** Toner nach einem der Ansprüche 1 bis 5, wobei zumindest ein Teil des Polyhydroxyalkanoats ein chemisch modifiziertes Polyhydroxyalkanoat ist.

**7.** Toner nach Anspruch 6, wobei das chemisch modifizierte Polyhydroxyalkanoat zumindest eine Pfropfkette einschließt.

**8.** Toner nach Anspruch 7, wobei die Pfropfkette eine solche ist, die erzeugt ist durch chemisches Modifizieren eines Polyhydroxyalkanoats, das zumindest eine Monomereinheit mit einer Epoxygruppe einschließt.

**9.** Toner nach einem der Ansprüche 7 oder 8, wobei die Pfropfkette eine Pfropfkette einer Verbindung mit einer Aminogruppe ist.

**10.** Toner nach Anspruch 9, wobei die Verbindung mit einer Aminogruppe eine mit einer endständigen Aminogruppe modifizierte Verbindung ist.

**11.** Toner nach Anspruch 6, wobei zumindest ein Teil des Polyhydroxyalkanoats ein vernetztes Polyhydroxyalkanoat ist.

**12.** Toner nach Anspruch 1 1, wobei das vernetzte Polyhydroxyalkanoat vorliegt als Vernetzungsprodukt eines Polyhydroxyalkanoats, das zumindest eine Monomereinheit mit einer Epoxygruppe einschließt.

**13.** Bilderzeugungsverfahren, umfassend zumindest
einen Schritt, bei dem an ein Aufladeteil von außen eine Spannung angelegt wird, wodurch ein Tragteil für ein latentes elektrostatisches Bild aufgeladen wird,
einen Schritt, bei dem auf dem aufgeladenen Tragteil ein elektrostatisches Ladungsbild erzeugt wird,
einen Entwicklungsschritt, bei dem das elektrostatische Ladungsbild mit einem Toner entwickelt wird, wodurch auf dem Tragteil ein Tonerbild entsteht,
einen Übertragungsschritt, bei dem das Tonerbild auf dem Tragteil auf ein Aufzeichnungsmaterial übertragen wird, und
einen Fixierschritt, bei dem das Tonerbild auf dem Aufzeichnungsmaterial mittels Wärme fixiert wird,
wobei das Bilderzeugungsverfahren die Verwendung eines ein elektrostatisches Ladungsbild entwickelnden Toners nach einem der Ansprüche 1 bis 12 umfaßt.

**14.** Verfahren nach Anspruch 13, umfassend zumindest

einen Schritt, bei dem an ein Aufladeteil von außen eine Spannung angelegt wird, wodurch ein Tragteil für ein latentes elektrostatisches Bild aufgeladen wird,
einen Schritt, bei dem auf dem aufgeladenen Tragteil ein elektrostatisches Ladungsbild erzeugt wird,
einen Entwicklungsschritt, bei dem das elektrostatische Ladungsbild mit einem Toner entwickelt wird, wodurch auf dem Tragteil ein Tonerbild entsteht,
einen ersten Übertragungsschritt, bei dem das Tonerbild auf dem Tragteil auf ein Zwischenübertragungsteil übertragen wird,
einen zweiten Übertragungsschritt, bei dem das Tonerbild auf dem Zwischenübertragungsteil auf ein Aufzeichnungsmaterial übertragen wird, und
einen Fixierschritt, bei dem das Tonerbild auf dem Aufzeichnungsmaterial mittels Wärme fixiert wird,
wobei das Verfahren die Verwendung eines ein elektrostatisches Ladungsbild entwickelnden Toners nach einem der Ansprüche 1 bis 12 umfaßt.

**15.** Bilderzeugungsvorrichtung, umfassend zumindest
eine Einrichtung, um an ein Aufladeteil von außen eine Spannung anzulegen, wodurch ein Tragteil für ein latentes elektrostatisches Bild aufgeladen wird,
eine Einrichtung, um auf dem aufgeladenen Tragteil ein elektrostatisches Ladungsbild zu erzeugen,
eine Entwicklungseinrichtung, die den ein elektrostatisches Ladungsbild entwickelnden Toner nach einem der Ansprüche 1 bis 12 enthält, zum Entwickeln des elektrostatischen Ladungsbildes mit diesem Toner, wodurch auf dem Tragteil ein Tonerbild erzeugt wird,
eine Übertragungseinrichtung zum Übertragen des Tonerbildes auf dem Tragteil auf ein Aufzeichnungsmaterial und
eine Fixiereinrichtung zum Fixieren des Tonerbildes auf dem Aufzeichnungsmaterial mittels Wärme.

**16.** Vorrichtung nach Anspruch 15, umfassend zumindest
eine Einrichtung, um an ein Aufladeteil von außen eine Spannung anzulegen, wodurch ein Tragteil für ein latentes elektrostatisches Bild aufgeladen wird,
eine Einrichtung, um auf dem aufgeladenen Tragteil ein elektrostatisches Ladungsbild zu erzeugen,
eine Entwicklungseinrichtung, die den ein elektrostatisches Ladungsbild entwickelnden Toner nach einem der Ansprüche 1 bis 12 enthält, zum Entwickeln des elektrostatischen Ladungsbildes mit diesem Toner, wodurch auf dem Tragteil ein Tonerbild entsteht,
eine erste Übertragungseinrichtung zum Übertragen des Tonerbildes auf dem Tragteil auf ein Zwischenübertragungsteil,
eine zweite Übertragungseinrichtung zum Übertragen des Tonerbildes auf dem Zwischenübertragungsteil auf ein Aufzeichnungsmaterial, und
eine Fixiereinrichtung zum Fixieren des Tonerbildes auf dem Aufzeichnungsmaterial mittels Wärme.

**17.** Verfahren zur Herstellung eines ein elektrostatisches Ladungsbild entwickelnden Toners, der ein färbendes Mittel einschließt, das erhalten wird, indem zumindest ein Teil der Oberfläche eines färbenden Mittels überzogen wird mit einem Polyhydroxyalkanoat, das eine erste Harzkomponente bildet,
wobei das Verfahren umfasst
die Durchführung einer Polyhydroxyalkanoat-Synthesereaktion unter Verwendung, von 3-Hydroxyacyl-CoA als Substrat in Gegenwart eines Polyhydroxyalkanoat synthetisierenden Enzyms, das an der Oberfläche eines in einem wässrigen Medium dispergierten färbenden Mittels fixiert wird, so daß zumindest ein Teil der Oberfläche des färbenden Mittels mit Polyhydroxyalkanoat überzogen wird, wodurch das färbende Mittel erzeugt wird.

**18.** Verfahren nach Anspruch 17,
wobei das Polyhydroxyalkanoat zumindest eines einschließt, das ausgewählt ist aus der Gruppe, bestehend aus Monomereinheiten der folgenden Formel (1) bis (10), und
wobei das entsprechende 3-Hydroxyacyl-Coenzym A eines mit den chemischen Formeln (11) bis (20) ist:

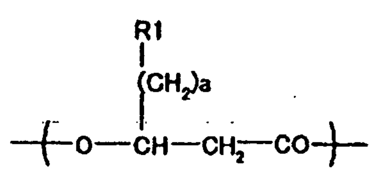

[1]

wobei die Monomereinheit zumindest eine ist, die ausgewählt ist aus der Gruppe, bestehend aus Monomereinheiten, bei denen die Kombination von R1 und a eine der folgenden ist:

eine Monomereinheit, bei der R1 ein Wasserstoffatom (H) ist, und a eine ganze Zahl von 0 bis 10;
eine Monomereinheit, bei der R1 ein Halogenatom ist, und a eine ganze Zahl von 0 bis 10;
eine Monomereinheit, bei der R1 ein Chromophor ist, und a eine ganze Zahl von 0 bis 10;
eine Monomereinheit, bei der R1 eine Carboxylgruppe oder ein Salz davon ist, und a eine ganze Zahl von 0 bis 10; und
eine Monomereinheit, bei der R1 eine Gruppe der folgenden Formel:

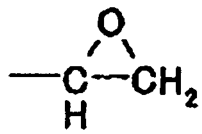

ist, und a eine ganze Zahl von 1 bis 7;

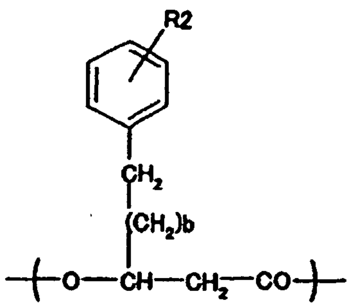

[2]

worin b eine ganze Zahl von 0 bis 7 ist und R2 ein Substituent ist, der ausgewählt ist aus der Gruppe, bestehend aus einem Wasserstoffatom (H), einem Halogenatom, -CN, $-NO_2$, $-CF_3$, $-C_2F_5$ und $-C_3F_7$;

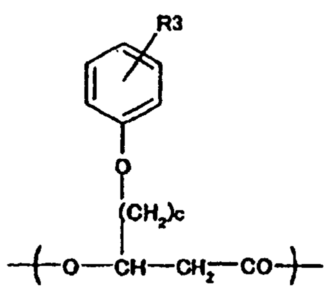

[3]

worin c eine ganze Zahl von 1 bis 8 ist und R3 ein Substituent ist, der ausgewählt ist aus der Gruppe, bestehend aus einem Wasserstoffatom (H), einem Halogenatom, -CN, $-NO_2$, $-CF_3$, $-C_2F_5$ und $-C_3F_7$;

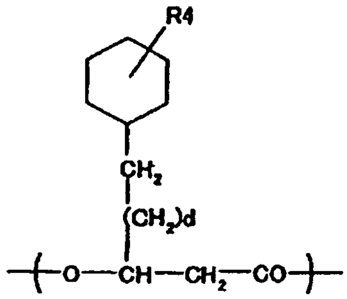

[4]

worin d eine ganze Zahl von 0 bis 7 ist und R4 ein Substituent ist, der ausgewählt ist aus der Gruppe, bestehend aus einem Wasserstoffatom (H), einem Halogenatom, -CN, $-NO_2$, $-CF_3$, $-C_2F_5$ und $-C3F_7$;

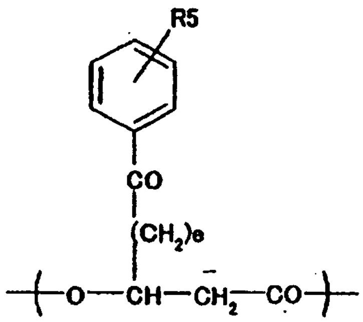

[5]

worin e eine ganze Zahl von 1 bis 8 ist und R5 ein Substituent ist, der ausgewählt ist aus der Gruppe, bestehend aus einem Wasserstoffatom (H), einem Halogenatom, -CN, $-NO_2$, $-CF_3$, $-C_2F_5$, $-C_3F_7$, $-CH_3$, $-C_2H_5$ und $-C_3H_7$;

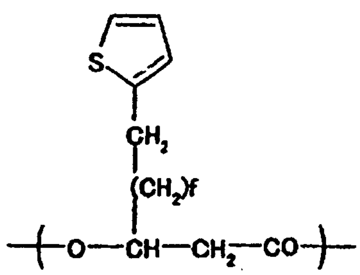

[6]

worin f eine ganze Zahl von 0 bis 7 ist;

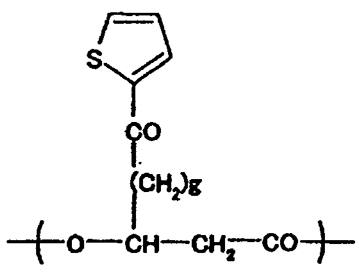

[7]

worin g eine ganze Zahl von 1 bis 8 ist;

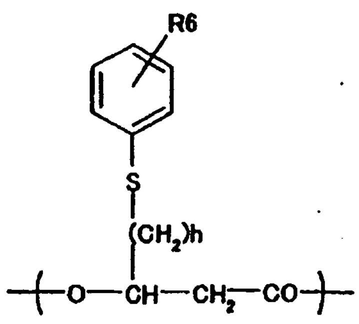

[8]

worin h eine ganze Zahl von 1 bis 7 ist und R6 ein Substituent ist, der ausgewählt ist aus der Gruppe, bestehend aus einem Wasserstoffatom (H), einem Halogenatom, -CN, $-NO_2$, -COOR', $-SO_2R''$, $-CH_3$, $-C_2H_5$, $-C_3H_7$, -CH $(CH3)_2$ und $-C(CH_3)_3$, worin ferner R' ein Wasserstoffatom, Na, K, $-CH_3$ oder $-C_2H_5$ ist und worin R" gleich -OH, -ONa, -OK, ein Halogenatom, $-OCH_3$ oder $-OC_2H_5$ ist;

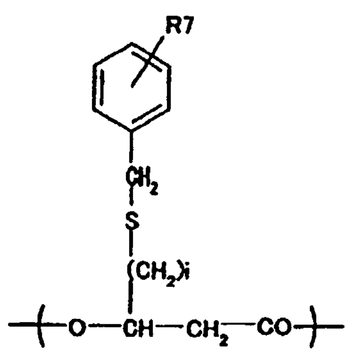

[9]

worin i eine ganze Zahl von 1 bis 7 ist und R7 ein Substituent ist, der ausgewählt ist aus der Gruppe, bestehend aus einem Wasserstoffatom (H), einem Halogenatom, -CN, $-NO_2$, -COOR' und $-SO_2R''$, worin ferner R' ein Wasserstoffatom, Na, K, $-CH_3$ oder $-C_2H_5$ ist und worin R" gleich -OH, -ONa, -OK, ein Halogenatom, $-OCH_3$ oder $-OC_2H_5$ ist;

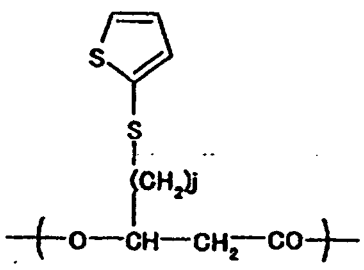 [10]

worin j eine ganze Zahl von 1 bis 9 ist;

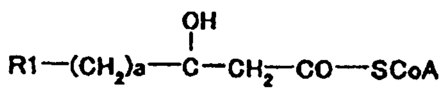 [11]

worin -SCoA für an Alkansäure gebundenes Coenzym A steht und die Kombination aus R1 und a eine der folgenden ist und der Kombination von R1 und a in der vorstehenden chemischen Formel (1) entspricht:

eine Monomereinheit, bei der R1 ein Wasserstoffatom (H) ist, und a eine ganze Zahl von 0 bis 10;
eine Monomereinheit, bei der R1 ein Halogenatom ist, und a eine ganze Zahl von 0 bis 10;
eine Monomereinheit, bei der R1 ein Chromophor ist, und a eine ganze Zahl von 0 bis 10;
eine Monomereinheit, bei der R1 eine Carboxylgruppe oder ein Salz davon ist, und a eine ganze Zahl von 0 bis 10; und
eine Monomereinheit, bei der R1 eine Gruppe der folgenden Formel

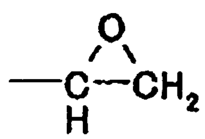

ist, und a eine ganze Zahl von 1 bis 7;

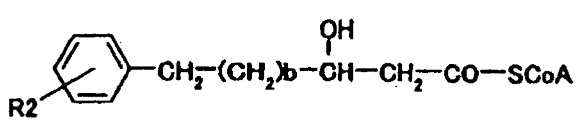 [12]

worin -SCoA für an Alkansäure gebundenes Coenzym A steht, b eine ganze Zahl von 0 bis 7 ist und b in der vorstehenden chemischen Formel (2) entspricht und worin ferner R2 ein Substituent ist, der ausgewählt ist aus der Gruppe, bestehend aus einem Wasserstoffatom (H), einem Halogenatom, -CN, $-NO_2$, $-CF_3$, $-C_2F_5$ und $-C_3F_7$, und R2 in der vorstehenden chemischen Formel (2) entspricht;

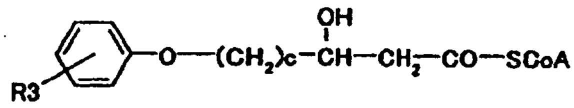

[13]

worin -SCoA für an Alkansäure gebundenes Coenzym A steht, c eine ganze Zahl von 1 bis 8 ist und c in der vorstehenden chemischen Formel (3) entspricht und worin ferner R3 ein Substituent ist, der ausgewählt ist aus der Gruppe, bestehend aus einem Wasserstoffatom (H), einem Halogenatom, -CN, $-NO_2$, $-CF_3$, $-C_2F_5$ und $-C_3F_7$ und R3 in der vorstehenden chemischen Formel (3) entspricht;

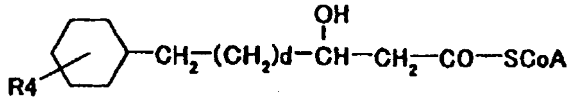

[14]

worin -SCoA für an Alkansäure gebundenes Coenzym A steht, d eine ganze Zahl von 0 bis 7 ist und d in der vorstehenden chemischen Formel (4) entspricht und worin ferner R4 ein Substituent ist, der ausgewählt ist aus der Gruppe, bestehend aus einem Wasserstoffatom (H), einem Halogenatom, -CN, $-NO_2$, $-CF_3$, $-C_2F_5$ und $-C_3F_7$, und R4 in der vorstehenden chemischen Formel (4) entspricht;

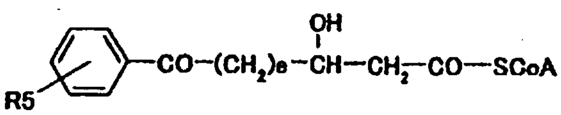

[15]

worin -SCoA für an Alkansäure gebundenes Coenzym A steht, e eine ganze Zahl von 1 bis 8 ist und e in der vorstehenden chemischen Formel (5) entspricht und worin ferner R5 ein Substituent ist, der ausgewählt ist aus der Gruppe, bestehend aus einem Wasserstoffatom (H), einem Halogenatom, -CN, $-NO_2$, $-CF_3$, $-C_2F_5$, $-C_3F_7$, $-CH_3$, $-C_2H_5$ und $-C_3H_7$, und R5 in der vorstehenden chemischen Formel (5) entspricht;

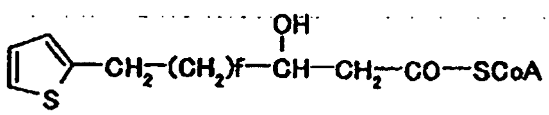

[16]

worin -SCoA für an Alkansäure gebundenes Coenzym A steht und f eine ganze Zahl von 0 bis 7 ist und f in der vorstehenden chemischen Formel (6) entspricht;

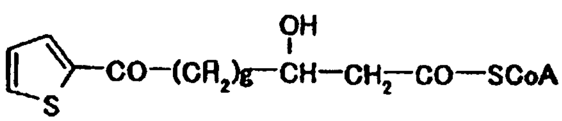

[17]

worin -SCoA für an Alkansäure gebundenes Coenzym A steht und g eine ganze Zahl von 1 bis 8 ist und g in der vorstehenden chemischen Formel (7) entspricht;

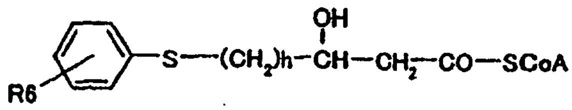

[18]

worin -SCoA für an Alkansäure gebundenes Coenzym A steht, h eine ganze Zahl von 1 bis 7 ist und h in der vorstehenden chemischen Formel (8) entspricht, worin ferner R6 ein Substituent ist, der ausgewählt ist aus der Gruppe, bestehend aus einem Wasserstoffatom (H), einem Halogenatom, -CN, $-NO_2$, -COOR', $-SO_2R''$, $-CH_3$, $-C_2H_5$, $-C_3H_7$, $-CH(CH_3)_2$ und $-C(CH_3)_3$, und R6 in der vorstehenden chemischen Formel (8) entspricht, wobei R' ein Wasserstoffatom, Na, K, $-CH_3$ oder $-C2H_5$ ist und R" gleich -OH, -ONa, -OK, ein Halogenatom, $-OCH_3$ oder $-OC_2H_5$ ist;

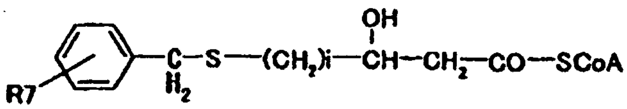

[19]

worin -SCoA für an Alkansäure gebundenes Coenzym A steht, i eine ganze Zahl von 1 bis 7 ist und i in der vorstehenden chemischen Formel (9) entspricht, worin ferner R7 ein Substituent ist, der ausgewählt ist aus der Gruppe, bestehend aus einem Wasserstoffatom (H), einem Halogenatom, -CN, $-NO_2$, -COOR' und $-SO_2R''$, und R7 in der vorstehenden chemischen Formel (9) entspricht, wobei R' ein Wasserstoffatom, Na, K, $-CH_3$ oder $-C_2H_5$ ist und R" gleich -OH, -ONa, -OK, ein Halogenatom, $-OCH_3$ oder $-OC_2H_5$ ist; und

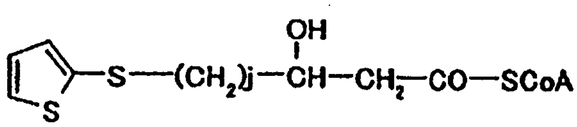

[20]

worin -SCoA für an Alkansäure gebundenes Coenzym A steht und j eine ganze Zahl von 1 bis 9 ist und j in der vorstehenden chemischen Formel (10) entspricht.

**19.** Verfahren nach Anspruch 17 oder 18, bei dem
sich die Zusammensetzung des 3-Hydroxyacyl-Coenzyms A mit der Zeit ändert, so daß sich die Zusammensetzung der 3-Hydroxyalkansäure-Einheit des Polyhydroxyalkanoats in einer Richtung von der Innenseite zur Außenseite des färbenden Mittels ändert.

**20.** Verfahren nach einem der Ansprüche 17 bis 19, weiter umfassend
einen Schritt, bei dem zumindest ein Teil des Polyhydroxyalkanoats, das das färbende Mittel überzieht, chemisch modifiziert wird.

**21.** Verfahren nach Anspruch 20, bei dem
der Schritt des chemischen Modifizierens ein Schritt ist, bei dem zumindest einem Teil des Polyhydroxyalkanoats eine Pfropfkette hinzugefügt wird.

**22.** Verfahren nach Anspruch 21, bei dem
der Schritt des Hinzufügens der Pfropfkette ein Schritt ist, bei dem zumindest ein Teil des Polyhydroxyalkanoats mit einer Verbindung reagiert, die am Ende eine reaktive funktionelle Gruppe aufweist.

**23.** Verfahren nach Anspruch 20, bei dem

der Schritt des chemischen Modifizierens ein Schritt ist, bei dem zumindest ein Teil des Polyhydroxyalkanoats vernetzt wird.

**24.** Verfahren nach Anspruch 23, bei dem
der Vernetzungsschritt ein Schritt ist, bei dem das Polyhydroxyalkanoat mit einem Elektronenstrahlenbündel bestrahlt wird.

**25.** Verfahren nach einem der Ansprüche 17 bis 19, bei dem
das Polyhydroxyalkanoat synthetisierende Enzym ein solches ist, das produziert wird von Mikroorganismen mit der Fähigkeit zur Produktion dieses Enzyms oder einer Transformante, die erhalten wird, indem ein dieses Produktionsvermögen betreffendes Gen in Wirtsorganismen eingeführt wird.

**26.** Verfahren nach Anspruch 25, bei dem
die Mikroorganismen mit der Fähigkeit zur Produktion des Polyhydroxyalkanoat synthetisierenden Enzyms jene von Pseudomonas sp. sind.

**27.** Verfahren nach Anspruch 25, bei dem
die Mikroorganismen mit der Fähigkeit zur Produktion des Polyhydroxyalkanoat synthetisierenden Enzyms jene von Burkholderia sp. sind.

**28.** Verfahren nach Anspruch 25, bei dem
die Mikroorganismen mit der Fähigkeit zur Produktion des Polyhydroxyalkanoat synthetisierenden Enzyms jene von Alcaligenes sp. sind.

**29.** Verfahren nach Anspruch 25, bei dem
wobei die Mikroorganismen mit der Fähigkeit zur Produktion des Polyhydroxyalkanoat synthetisierenden Enzyms jene von Ralstonia sp. sind.

**30.** Verfahren nach Anspruch 25, bei dem
die Wirtsorganismen für die Transformante mit der Fähigkeit zur Produktion des Polyhydroxyalkanoat synthetisierenden Enzyms Escherichia coli sind.

## Revendications

**1.** Toner pour le développement d'une image chargée électrostatique, comprenant au moins une matière colorante dont au moins une partie est couverte d'un polyhydroxyalcanoate constituant une première résine, et d'une résine servant de liant constituant une seconde résine.

**2.** Toner pour le développement d'une image chargée électrostatique suivant la revendication 1, dans lequel ladite matière colorante contient un pigment.

**3.** Toner pour le développement d'une image chargée électrostatique suivant la revendication 1 ou 2, dans lequel ledit polyhydroxyalcanoate comprend au moins un polyhydroxyalcanoate choisi dans le groupe consistant en les motifs monomères représentés par les formules 1 à 10 suivantes :

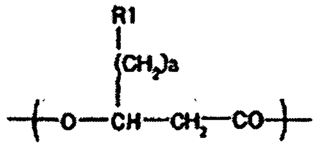

[1]

le motif monomère étant au moins un motif monomère choisi dans le groupe consistant en les motifs monomères dans lesquels l'association de R1 et a est n'importe laquelle des suivantes :

un motif monomère dans lequel R1 représente un atome d'hydrogène (H) et a représente un nombre entier de 0 à 10 ;

un motif monomère dans lequel R1 représente un atome d'halogène et a représente un nombre entier de 1 à 10 ;
un motif monomère dans lequel R1 représente un chromophore et a représente un nombre entier de 1 à 10 ;
un motif monomère dans lequel R1 représente un groupe carboxyle ou un de ses sels et a représente un nombre entier de 1 à 10 ; et
un motif monomère dans lequel R1 représente un groupe représenté par la formule suivante :

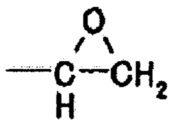

et a représente un nombre entier de 1 à 7 ;

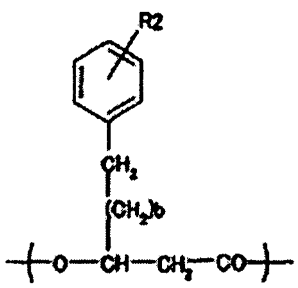

[2]

dans laquelle b représente un nombre entier de 0 à 7 et R2 représente un substituant choisi dans le groupe consistant en un atome d'hydrogène (H), un atome d'halogène, des groupes -CN, $-NO_2$, $-CF_3$, $-C_2F_5$ et $-C_3F_7$ ;

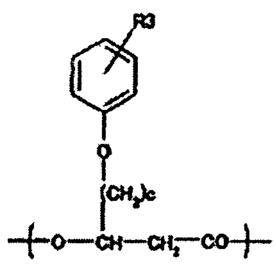

[3]

dans laquelle c représente un nombre entier de 1 à 8 et R3 représente un substituant choisi dans le groupe consistant en un atome d'hydrogène (H), un atome d'halogène, des groupes -CN, $-NO_2$, $-CF_3$, $-C_2F_5$ et $-C_3F_7$ ;

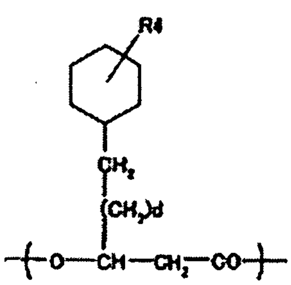

[4]

dans laquelle d représente un nombre entier de 0 à 7 et R4 représente un substituant choisi dans le groupe consistant en un atome d'hydrogène (H), un atome d'halogène, des groupes -CN, $-NO_2$, $-CF_3$, $-C_2F_5$ et

$-C_3F_7$ ;

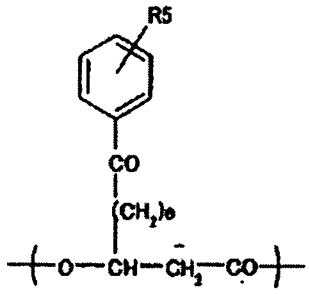

[5]

dans laquelle e représente un nombre entier de 1 à 8 et R5 représente un substituant choisi dans le groupe consistant en un atome d'hydrogène (H), un atome d'halogène, des groupes -CN, $-NO_2$ $-CF_3$, $-C_2F_5$, $-C_3F_7$, $-CH_3$, $-C_2H_5$ et $-C_3H_7$ ;

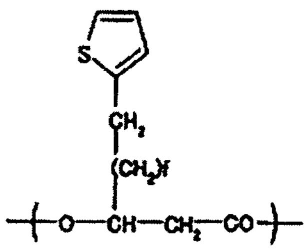

[6]

dans laquelle f représente un nombre entier de 0 à 7 ;

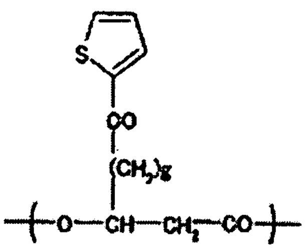

[7]

dans laquelle g représente un nombre entier de 1 à 8 ;

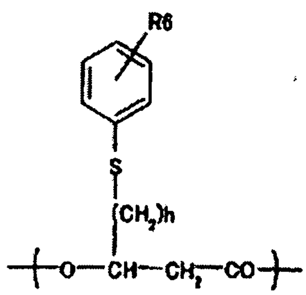

[8]

dans laquelle h représente un nombre entier de 1 à 7, R6 représente un substituant choisi dans le groupe consistant en un atome d'hydrogène (H), un atome d'halogène, des groupes -CN, $-NO_2$ -COOR', $-SO_2R''$ $-CH_3$, $-C_2H_5$, $-C_3H_7$, $-CH(CH_3)_2$ et $-C(CH_3)_3$, R' représente un atome d'hydrogène, un groupe Na, K, $-CH_3$, ou $-C_2H_5$ et R'' représente un groupe -OH, -ONa, -OK, un atome d'halogène, un groupe $-OCH_3$ ou $-OC_2H_5$ ;

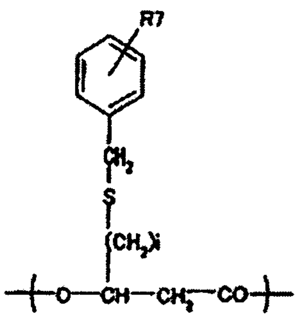

[9]

dans laquelle i représente un nombre entier de 1 à 7, R7 représente un substituant choisi dans le groupe consistant en un atome d'hydrogène (H), un atome d'halogène, des groupes -CN, $-NO_2$, -COOR' et $-SO_2R''$, R' représente un atome d'hydrogène, un groupe Na, K, $-CH_3$, ou $-CH_2H_5$ et R'' représente un groupe -OH, -ONa, -OC, un atome d'halogène, un groupe $-OCH_3$ ou $-OC_2H_5$ ; et

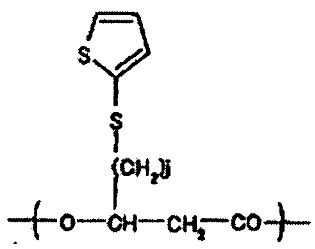

[10]

dans laquelle j représente un nombre entier de 1 à 9.

**4.** Toner pour le développement d'une image chargée électrostatique suivant l'une quelconque des revendications 1 à 3, dans lequel ledit polyhydroxyalcanoate a une moyenne en nombre du poids moléculaire comprise dans l'intervalle de 1000 à 10 000 000.

**5.** Toner pour le développement d'une image chargée électrostatique suivant l'une quelconque des revendications 1 à 4, dans lequel la composition en motifs monomères dudit polyhydroxyalcanoate varie dans une direction de la face intérieure à la face extérieure de ladite matière colorante.

**6.** Toner pour le développement d'une image chargée électrostatique suivant l'une quelconque des revendications 1 à 5, dans lequel au moins une partie dudit polyhydroxyalcanoate est constituée d'un polyhydroxyalcanoate modifié chimiquement.

**7.** Toner pour le développement d'une image chargée électrostatique suivant la revendication 6, dans lequel ledit polyhydroxyalcanoate modifié chimiquement comprend au moins une chaîne greffée.

**8.** Toner pour le développement d'une image chargée électrostatique suivant la revendication 7, dans lequel ladite chaîne greffée est une chaîne greffée formée par modification chimique d'un polyhydroxyalcanoate comprenant au moins un motif monomère ayant un groupe époxy.

**9.** Toner pour le développement d'une image chargée électrostatique suivant la revendication 7 ou 8, dans lequel ladite chaîne greffée est une chaîne greffée d'un composé ayant un groupe amino.

**10.** Toner pour le développement d'une image chargée électrostatique suivant la revendication 9, dans lequel ledit composé ayant un groupe amino est un composé à modification amino terminale.

**11.** Toner pour le développement d'une image chargée électrostatique suivant la revendication 6, dans lequel au moins une partie dudit polyhydroxyalcanoate est un polyhydroxyalcanoate réticulé.

12. Toner pour le développement d'une image chargée électrostatique suivant la revendication 11, dans lequel ledit polyhydroxyalcanoate réticulé est réticulé à partir d'un polyhydroxyalcanoate comprenant au moins un motif monomère ayant un groupe époxy.

13. Procédé de formation d'image comprenant au moins une étape d'application externe d'une tension à un élément de charge en chargeant ainsi un élément de support d'image latente électrostatique, une étape de formation d'une image chargée électrostatique sur l'élément de support d'image chargée latente électrostatique, une étape de développement pour l'image chargée électrostatique avec un toner pour le développement d'une image chargée électrostatique en formant ainsi une image de toner sur l'éluant de support d'image latente électrostatique, une étape de transfert pour transférer l'image de toner sur l'élément de support d'image latente électrostatique sur une matière d'enregistrement, et une étape de fixage pour le fixage à chaud de l'image de toner sur la matière d'enregistrement, procédé comprenant l'utilisation du toner pour le développement d'une image chargée électrostatique suivant l'une quelconque des revendications 1 à 12.

14. Procédé de formation d'image suivant la revendication 13, comprenant au moins une étape d'application externe d'une tension à un élément de charge pour charger ainsi un élément de support d'image latente électrostatique, une étape de formation d'une image chargée électrostatique sur l'élément de support d'image latente électrostatique chargée, une étape de développement pour développer l'image chargée électrostatique avec un toner pour le développement d'une image chargée électrostatique en formant ainsi une image de toner sur l'élément de support d'image latente électrostatique, une première étape de transfert pour transférer l'image de toner sur l'élément de support d'image latente électrostatique sur un élément de transfert intermédiaire, une seconde étape de transfert pour transférer l'image de toner sur l'élément de transfert intermédiaire sur une matière d'enregistrement, et une étape de fixage pour fixer à chaud l'image de toner sur la matière d'enregistrement, procédé comprenant l'utilisation du toner pour le développement d'une image chargée électrostatique suivant l'une quelconque des revendications 1 à 12.

15. Appareil de formation d'image comprenant au moins un moyen pour l'application externe d'une tension à un élément de charge pour charger ainsi un élément de support d'image latente électrostatique, un moyen pour former une image chargée électrostatique sur l'élément de support d'image latente électrostatique chargée, un moyen de développement contenant le toner pour le développement d'une image chargée électrostatique suivant l'une quelconque des revendications 1 à 12 afin de développer l'image chargée électrostatique avec le toner pour le développement d'une image chargée électrostatique en formant ainsi une image de toner sur l'élément de support d'image latente électrostatique, un moyen de transfert pour transférer l'image de toner sur l'élément de support d'image latente électrostatique sur une matière d'enregistrement et un moyen de fixage pour le fixage à chaud de l'image de toner sur la matière d'enregistrement.

16. Appareil de formation d'image suivant la revendication 15, comprenant au moins un moyen pour l'application externe d'une tension à un élément de charge pour charger ainsi un élément de support d'image latente électrostatique, un moyen pour former une image chargée électrostatique sur l'élément de support d'image latente électrostatique chargé, un moyen de développement contenant le toner pour le développement d'une image chargée électrostatique suivant l'une quelconque des revendications 1 à 12 afin de développer l'image chargée électrostatique avec un toner pour le développement d'une image chargée électrostatique en formant ainsi une image de toner sur l'élément de support d'image latente électrostatique, un premier moyen de transfert pour transférer l'image de toner sur l'élément de support d'image latente électrostatique sur un élément de transfert intermédiaire, un second moyen de transfert pour transférer l'image de toner sur l'élément de transfert intermédiaire sur une matière d'enregistrement et un moyen de fixage pour le fixage à chaud de l'image de toner sur la matière d'enregistrement.

17. Procédé pour la production d'un toner pour le développement d'une image chargée électrostatique, comprenant une matière colorante obtenue en couvrant au moins une partie de la surface d'un agent colorant avec un polyhydroxyalcanoate constituant une première résine, procédé comprenant la conduite d'une réaction de synthèse de polyhydroxyalcanoate en utilisant du 3-hydroxyacyle CoA comme substrat en présence d'une enzyme de synthèse de polyhydroxyalcanoate fixée sur la surface d'un agent colorant dispersé dans un milieu aqueux pour couvrir au moins une partie de la surface dudit agent colorant avec un polyhydroxyalcanoate en produisant ainsi ladite matière colorante.

18. Procédé pour la production d'un toner pour le développement d'une image chargée électrostatique suivant la revendication 17, dans lequel ledit polyhydroxyalcanoate comprend au moins un polyhydroxyalcanoate choisi dans le groupe consistant en les motifs monomères représentés par les formules (1) à (10) suivantes, et le 3-hydroxyacyl-coenzyme A correspondant respectivement est n'importe lequel de ceux représentés par les formules chimiques (11) à (20):

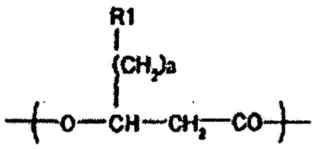

[1]

le motif monomère étant au moins un motif monomère choisi dans le groupe consistant en les motifs monomères dans lesquels l'association de R1 et a est n'importe laquelle des suivantes :

un motif monomère dans lequel R1 représente un atome d'hydrogène (H) et a représente un nombre entier de 0 à 10 ;
un motif monomère dans lequel R1 représente un atome d'halogène et a représente un nombre entier de 1 à 10 ;
un motif monomère dans lequel R1 représente un chromophore et a représente un nombre entier de 1 à 10 ;
un motif monomère dans lequel R1 représente un groupe carboxyle ou un de ses sels et a représente un nombre entier de 1 à 10 ; et
un motif monomère dans lequel R1 représente un groupe représenté par la formule suivante :

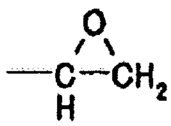

et a représente un nombre entier de 1 à 7 ;

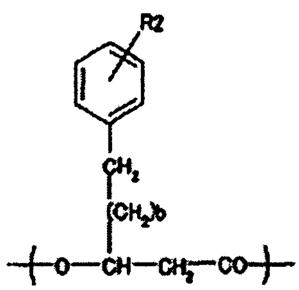

[2]

dans laquelle b représente un nombre entier de 0 à 7 et R2 représente un substituant choisi dans le groupe consistant en un atome d'hydrogène (H), un atome d'halogène, des groupes -CN, $-NO_2$, $-CF_3$, $-C_2F_5$ et $-C_3F_7$ ;

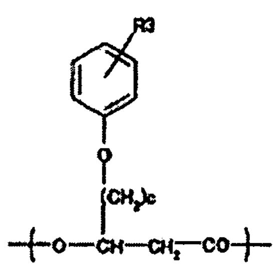

[3]

dans laquelle c représente un nombre entier de 1 à 8 et R3 représente un substituant choisi dans le groupe

consistant en un atome d'hydrogène (H), un atome d'halogène, des groupes -CN, $-NO_2$, $-CF_3$, $-C_2F_5$ et $-C_3F_7$ ;

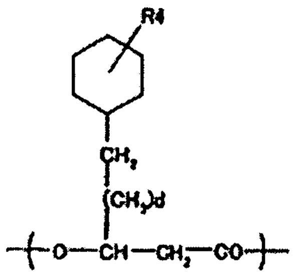

[4]

dans laquelle d représente un nombre entier de 0 à 7 et R4 représente un substituant choisi dans le groupe consistant en un atome d'hydrogène (H), un atome d'halogène, des groupes -CN, $-NO_2$, $-CF_3$, $-C_2F_5$ et $-C_3F_7$ ;

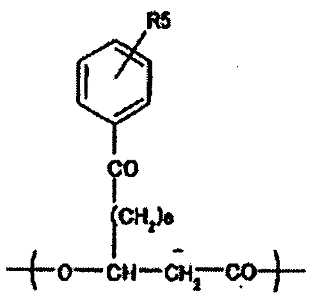

[5]

dans laquelle e représente un nombre entier de 1 à 8 et R5 représente un substituant choisi dans le groupe consistant en un atome d'hydrogène (H), un atome d'halogène, des groupes -CN, $-NO_2$, $-CF_3$, $-C_2F_5$, $-C_3F_7$, $-CH_3$, $-C_2H_5$ et $-C_3H_7$ ;

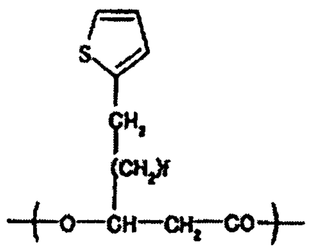

[6]

dans laquelle f représente un nombre entier de 0 à 7 ;

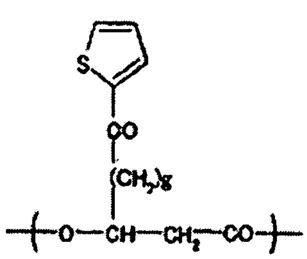

[7]

dans laquelle g représente un nombre entier de 1 à 8 ;

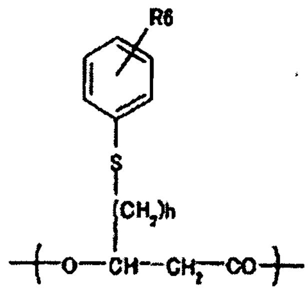

[8]

dans laquelle h représente un nombre entier de 1 à 7, R6 représente un substituant choisi dans le groupe consistant en un atome d'hydrogène (H), un atome d'halogène, des groupes -CN, $-NO_2$, -COOR', $-SO_2R''$, $-CH_3$, $-C_2H_5$, $-C_3H_7$, $-CH(CH_3)_2$ et $-C(CH_3)_3$, R' représente un atome d'hydrogène, un groupe Na, K, $-CH_3$, ou $-C_2H_5$ et R'' représente un groupe -OH, -ONa, -OK, un atome d'halogène, un groupe $-OCH_3$ ou $-OC_2H_5$ ;

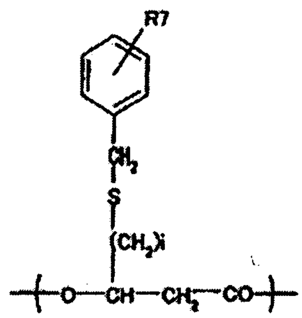

[9]

dans laquelle i représente un nombre entier de 1 à 7, R7 représente un substituant choisi dans le groupe consistant en un atome d'hydrogène (H), un atome d'halogène, des groupes -CN, $-NO_2$, -COOR' et $-SO_2R''$, R' représente un atome d'hydrogène, un groupe Na, K, $-CH_3$, ou $-C_2H_5$ et R'' représente un groupe -OH, -ONa, -OK, un atome d'halogène, un groupe $-OCH_3$ ou $-OC_2H_5$ ;

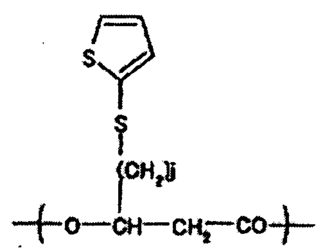

[10]

dans laquelle j représente un nombre entier de 1 à 9 ;

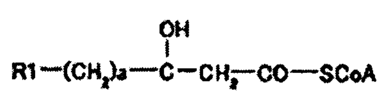

[11]

dans laquelle -SCoA représente le coenzyme A lié à un acide alcanoïque, et l'association de R1 et a est n'importe laquelle des suivantes et correspond à l'association de R1 et a dans la formule chimique (1) précédente :

un motif monomère dans lequel R1 représente un atome d'hydrogène (H) et a représente un nombre entier de 0 à 10 ;
un motif monomère dans lequel R1 représente un atome d'halogène et a représente un nombre entier de 1 à 10 ;

un motif monomère dans lequel R1 représente un chromophore et a représente un nombre entier de 1 à 10 ;
un motif monomère dans lequel R1 représente un groupe carboxyle ou un de ses sels et a représente un nombre entier de 1 à 10 ; et
un motif monomère dans lequel R1 représente un groupe représenté par la formule suivante :

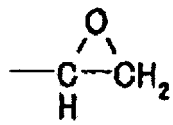

et a représente un nombre entier de 1 à 7 ;

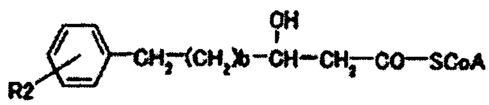

[12]

dans laquelle -SCoA représente le coenzyme A lié à un acide alcanoïque, b représente un nombre entier de 0 à 7 correspondant à b dans la formule chimique (2) précédente et R2 représente un substituant choisi dans le groupe consistant en un atome d'hydrogène (H), un atome d'halogène, des groupes -CN, $-NO_2$, $-CF_3$, $-C_2F_5$ et $-C_3F_7$, correspondant à R2 dans la formule chimique (2) précédente ;

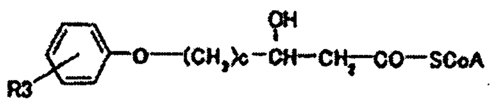

[13]

dans laquelle -SCoA représente le coenzyme A lié à un acide alcanoïque, c représente un nombre entier de 1 à 8 correspondant à c dans la formule chimique (3) précédente et R3 représente un substituant choisi dans le groupe consistant en un atome d'hydrogène (H), un atome d'halogène, des groupes -CN, $-NO_2$ $-CF_3$, $-C_2F_5$ et $-C_3F_7$, correspondant à R3 dans la formule chimique (3) précédente ;

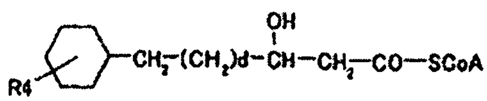

[14]

dans laquelle -SCoA représente le coenzyme A lié à un acide alcanoïque, d représente un nombre entier de 0 à 7 correspondant à d dans la formule chimique (4) précédente et R4 représente un substituant choisi dans le groupe consistant en un atome d'hydrogène (H), un atome d'halogène, des groupes -CN, $-NO_2$, $-CF_3$, $-C_2F_5$ et $-C_3F_7$, correspondant à R4 dans la formule chimique (4) précédente ;

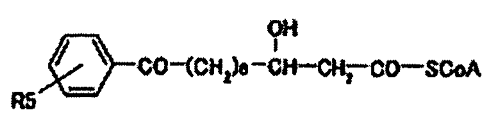

[15]

dans laquelle -SCoA représente le coenzyme A lié à un acide alcanoïque, e représente un nombre entier de 1 à 8 correspondant à e dans la formule chimique (5) précédente et R5 représente un substituant choisi dans le groupe consistant en un atome d'hydrogène (H), un atome d'halogène, des groupes -CN, $-NO_2$, $-CF_3$, $-C_2F_5$, $-C_3F_7$, $-CH_3$, $-C_2H_5$ et $-C_3H_7$ correspondant à R5 dans la formule chimique (5) précédente ;

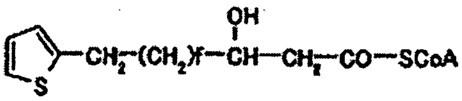

[16]

dans laquelle -SCoA représente le coenzyme A lié à un acide alcanoïque et f représente un nombre entier de 0 à 7 correspondant à f dans la formule chimique (6) précédente ;

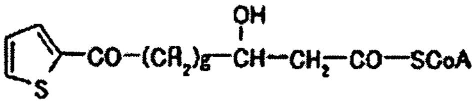

[17]

dans laquelle -SCoA représente le coenzyme A lié à un acide alcanoïque et g représente un nombre entier de 1 à 8 correspondant à f dans la formule chimique (7) précédente ;

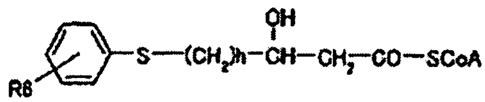

[18]

dans laquelle -SCoA représente le coenzyme A lié à un acide alcanoïque, h représente un nombre entier de 1 à 7 correspondant à h dans la formule chimique (8) précédente ; R6 représente un substituant choisi dans le groupe consistant en un atome d'hydrogène (H), un atome d'halogène, des groupes -CN, $-NO_2$, -COOR', $-SO_2R''$, $-CH_3$, $-C_2H_5$, $-C_3H_7$, $-CH(CH_3)_2$ et $-C(CH_3)_3$ correspondant à R6 dans la formule chimique (8) précédente, R' représente un atome d'hydrogène, un groupe Na, K, $-CH_3$, ou $-C_2H_5$ et R'' représente un groupe -OH, -ONa, -OK, un atome d'halogène, un groupe $-OCH_3$ ou $-OC_2H_S$ ;

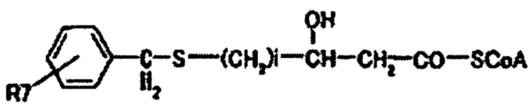

[19]

dans laquelle -SCoA représente le coenzyme A lié à un acide alcanoïque et i représente un nombre entier de 1 à 7 correspondant à i dans la formule chimique (9) précédente ; R7 représente un substituant choisi dans le groupe consistant en un atome d'hydrogène (H), un atome d'halogène, des groupes -CN, $-NO_2$, -COOR' et $-SO_2R''$ correspondant à R7 dans la formule chimique (9) précédente, R' représente un atome d'hydrogène, un groupe Na, K, $-CH_3$, ou $-C_2H_5$ et R'' représente un groupe -OH, -ONa, -OC, un atome d'halogène, un groupe $-OCH_3$ ou $-OC_2H_5$ ; et

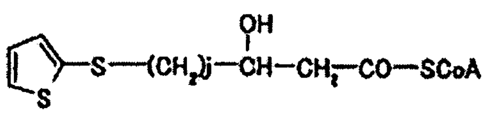

[20]

dans laquelle -SCoA représente le coenzyme A lié à un acide alcanoïque et j représente un nombre entier de 1 à 9 correspondant à la formule chimique (10) précédente.

**19.** Procédé pour la production d'un toner pour le développement d'une image chargée électrostatique suivant la revendication 17 ou 18, dans lequel la composition dudit 3-hydroxyacyl-coenzyme A est modifiée au cours du temps pour faire varier la composition en motif acide 3-hydroxy-alcanoïque dudit polyhydroxyalcanoate dans une direction de la face intérieure à la face extérieure de ladite matière colorante.

**20.** Procédé pour la production d'un toner pour le développement d'une image chargée électrostatique suivant l'une

quelconque des revendications 17 à 19, comprenant en outre une étape d'application d'une modification chimique à au moins une partie du polyhydroxyalcanoate couvrant ladite matière colorante.

**21.** Procédé pour la production d'un toner pour le développement d'une image chargée électrostatique suivant la revendication 20, dans lequel ladite étape d'application d'une modification chimique est une étape d'addition d'une chaîne greffée à au moins une partie d'un polyhydroxyalcanoate.

**22.** Procédé pour la production d'un toner pour le développement d'une image chargée électrostatique suivant la revendication 21, dans lequel l'étape d'addition de ladite chaîne greffée est une étape de réaction d'au moins une partie d'un polyhydroxyalcanoate avec un composé ayant un groupe fonctionnel réactif à l'extrémité.

**23.** Procédé pour la production d'un toner pour le développement d'une image chargée électrostatique suivant la revendication 20, dans lequel d'application de ladite modification chimique est une étape de réticulation d'au moins une partie d'un polyhydroxyalcanoate.

**24.** Procédé pour la production d'un toner pour le développement d'une image chargée électrostatique suivant la revendication 23, dans lequel ladite étape de réticulation est une étape d'irradiation d'un polyhydroxyalcanoate avec un faisceau d'électrons.

**25.** Procédé pour la production d'un toner pour le développement d'une image chargée électrostatique suivant l'une quelconque des revendications 17 à 19, dans lequel ladite enzyme de synthèse d'un polyhydroxyalcanoate est une enzyme de synthèse d'un polyhydroxyalcanoate produite par des micro-organismes présentant l'aptitude à produire ladite enzyme, ou un transformant obtenu en introduisant un gène en rapport avec ladite capacité de production dans des micro-organismes hôtes.

**26.** Procédé pour la production d'un toner pour le développement d'une image chargée électrostatique suivant la revendication 25, dans lequel lesdits micro-organismes présentant l'aptitude à produire ladite enzyme de synthèse de polyhydroxyalcanoate sont des micro-organismes Pseudomonas sp.

**27.** Procédé pour la production d'un toner pour le développement d'une image chargée électrostatique suivant la revendication 25, dans lequel lesdits micro-organismes présentant l'aptitude à produire ladite enzyme de synthèse de polyhydroxyalcanoate sont des micro-organismes Burkholderia sp.

**28.** Procédé pour la production d'un toner pour le développement d'une image chargée électrostatique suivant la revendication 25, dans lequel lesdits micro-organismes présentant l'aptitude à produire ladite enzyme de synthèse de polyhydroxyalcanoate sont des micro-organismes Alcaligenes sp.

**29.** Procédé pour la production d'un toner pour le développement d'une image chargée électrostatique suivant la revendication 25, dans lequel lesdits micro-organismes présentant l'aptitude à produire ladite enzyme de synthèse de polyhydroxyalcanoate sont des micro-organismes Ralstonia sp.

**30.** Procédé pour la production d'un toner pour le développement d'une image chargée électrostatique suivant la revendication 25, dans lequel les micro-organismes hôtes pour le transformant présentant l'aptitude à produire ladite enzyme de synthèse de polyhydroxyalcanoate sont des micro-organismes Escherichia coli.

FIG. 1

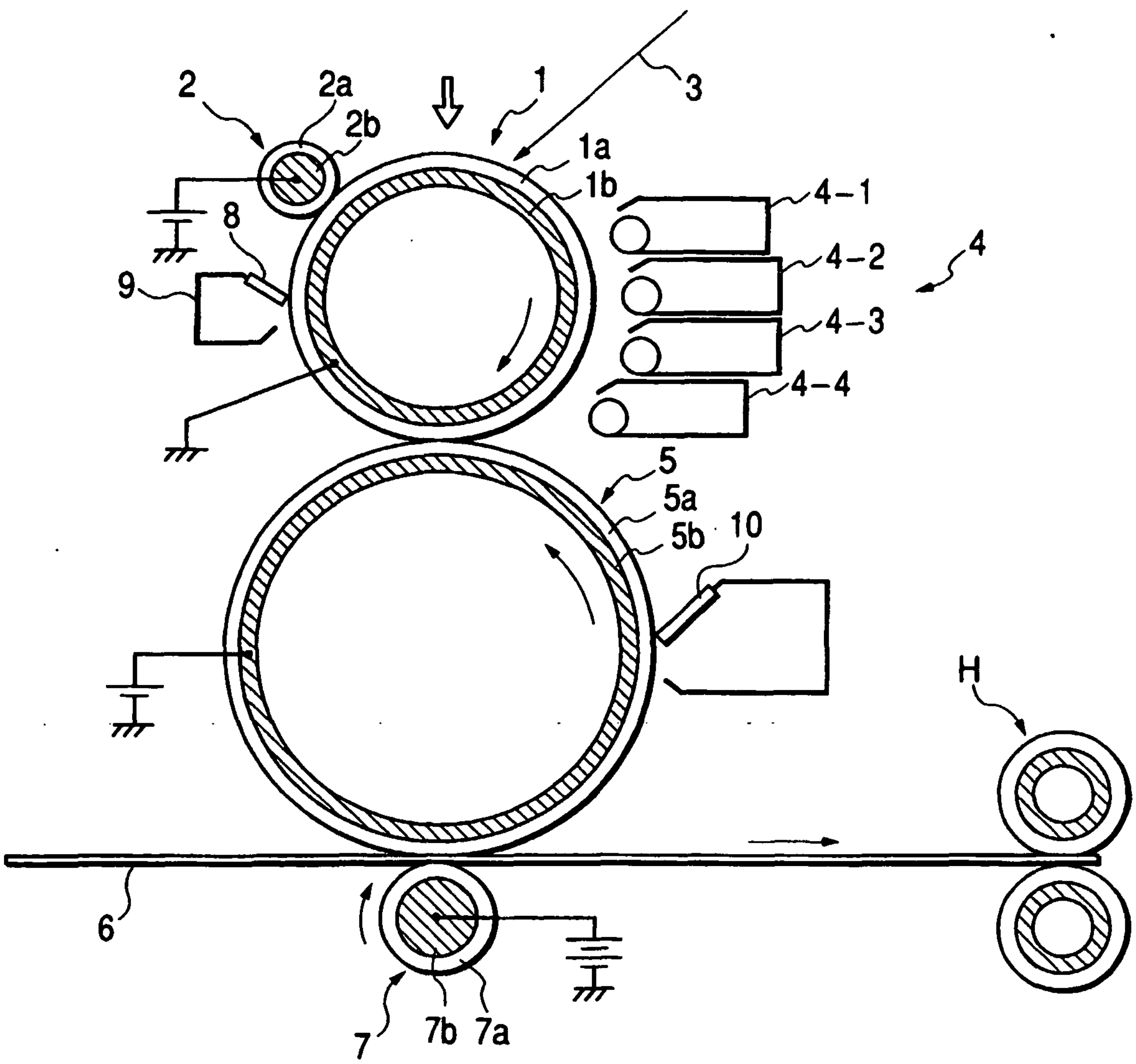

FIG. 2

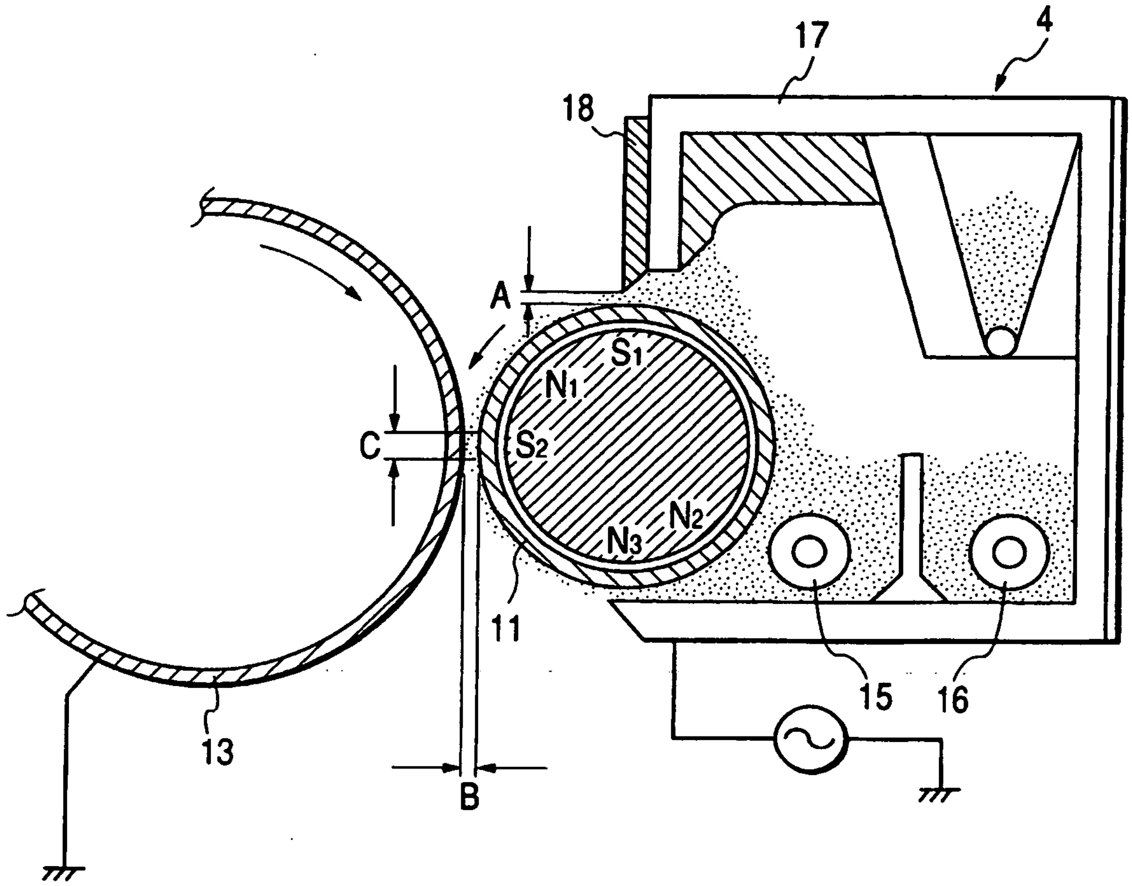

FIG. 3

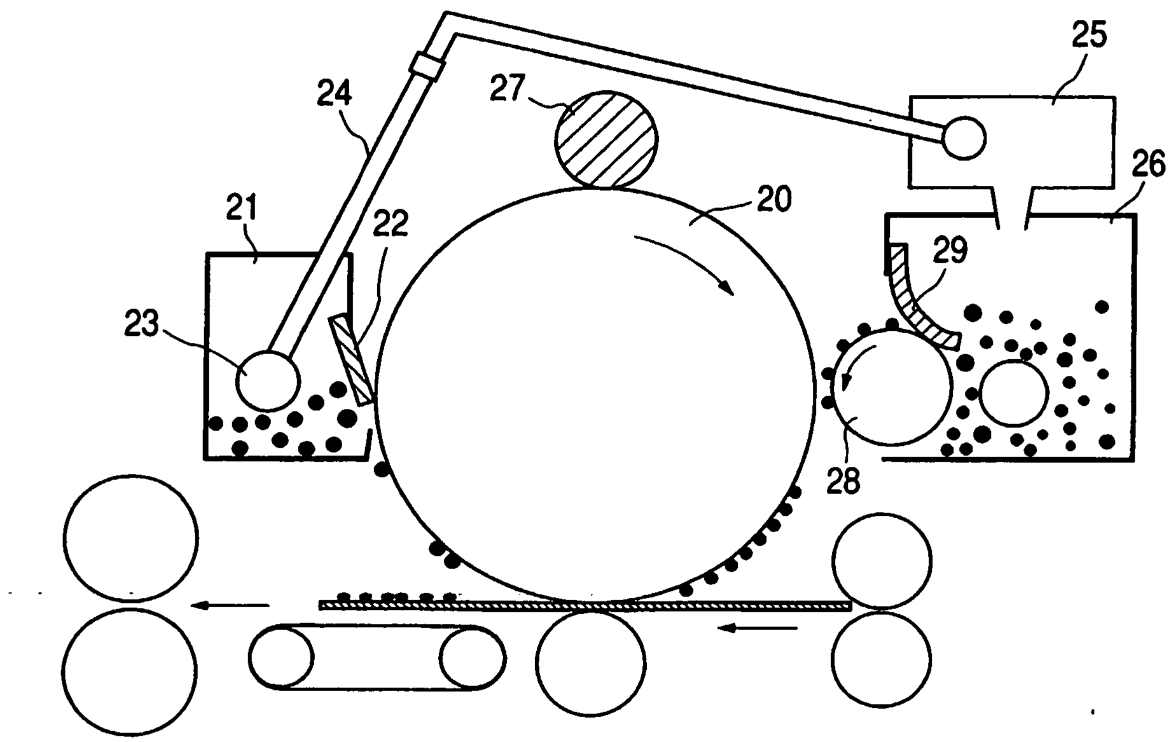

FIG. 4

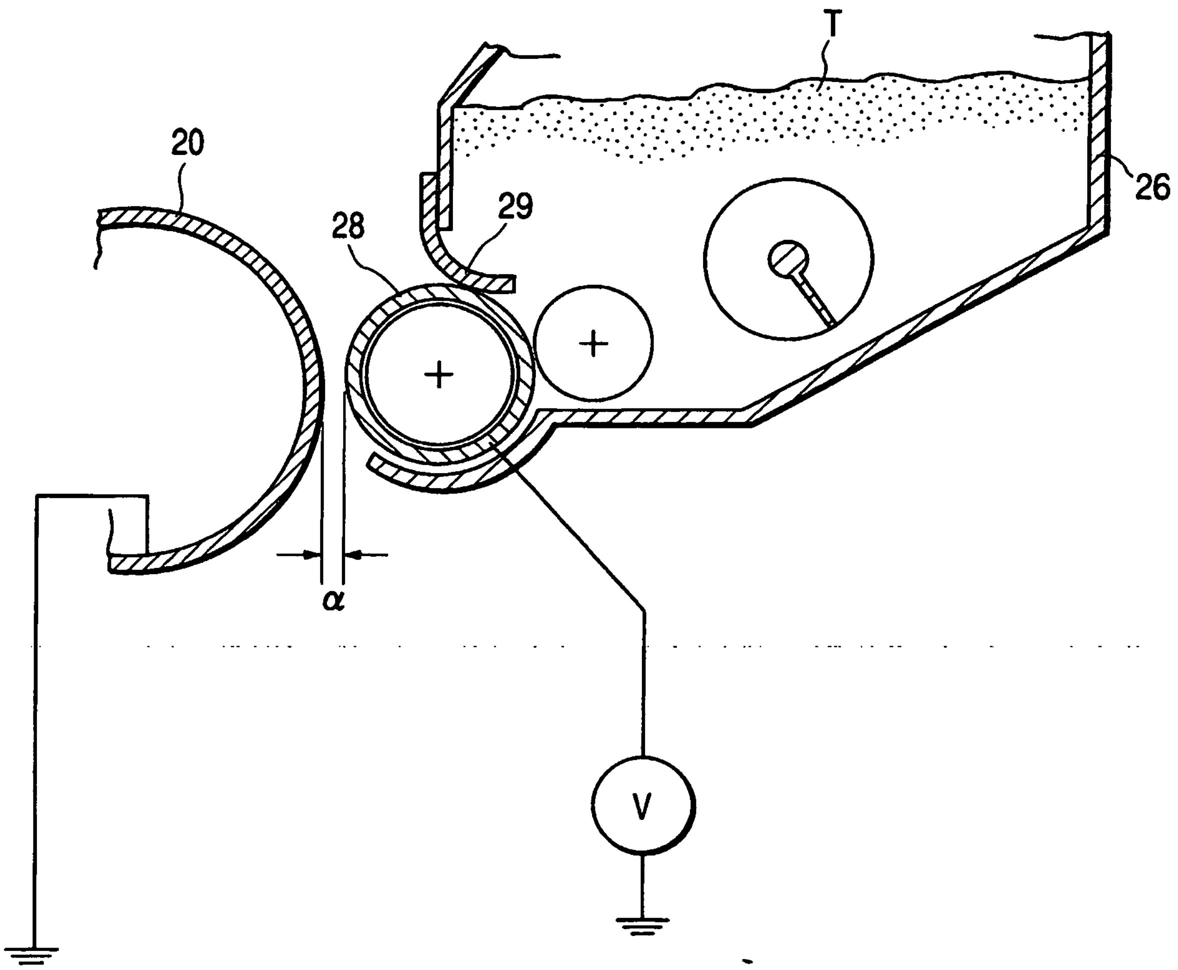

FIG. 5

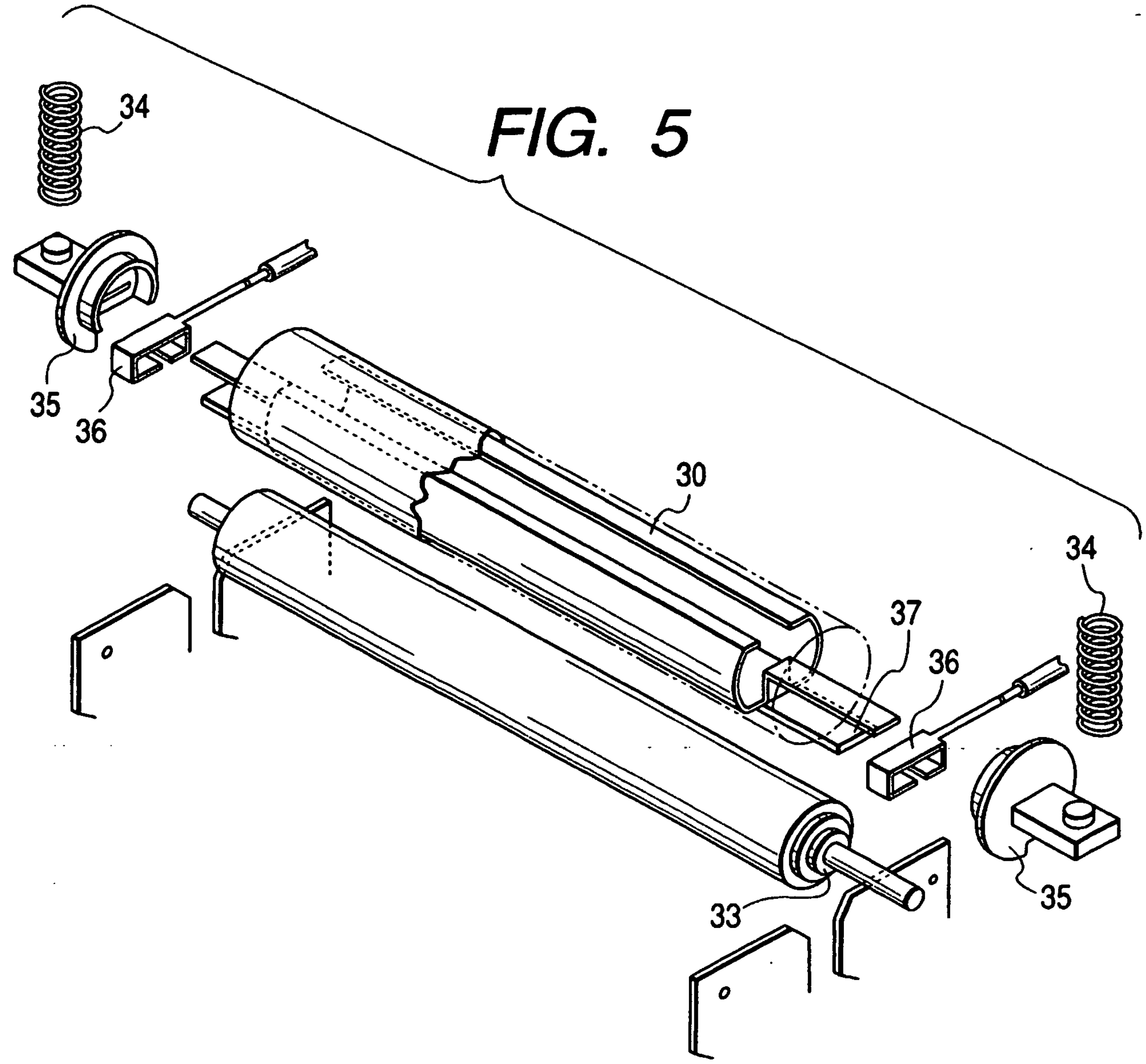

FIG. 6

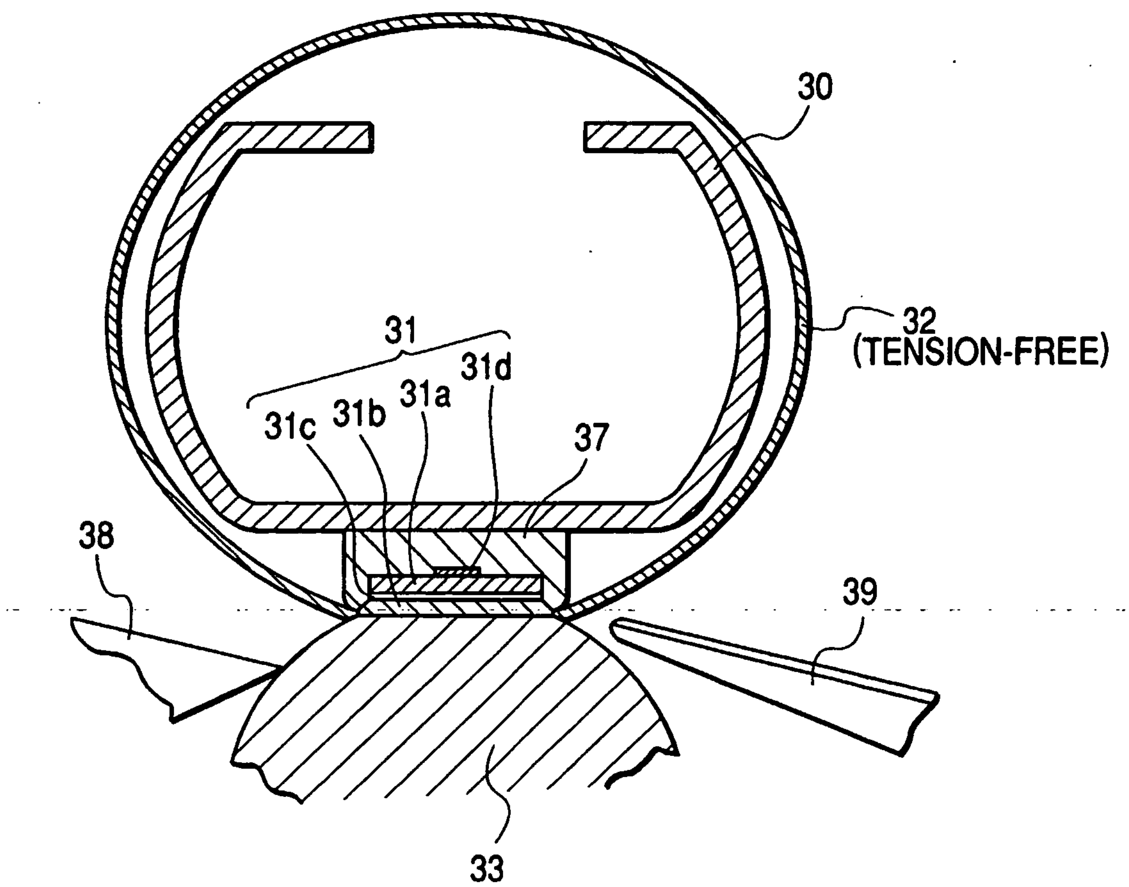

# FIG. 7

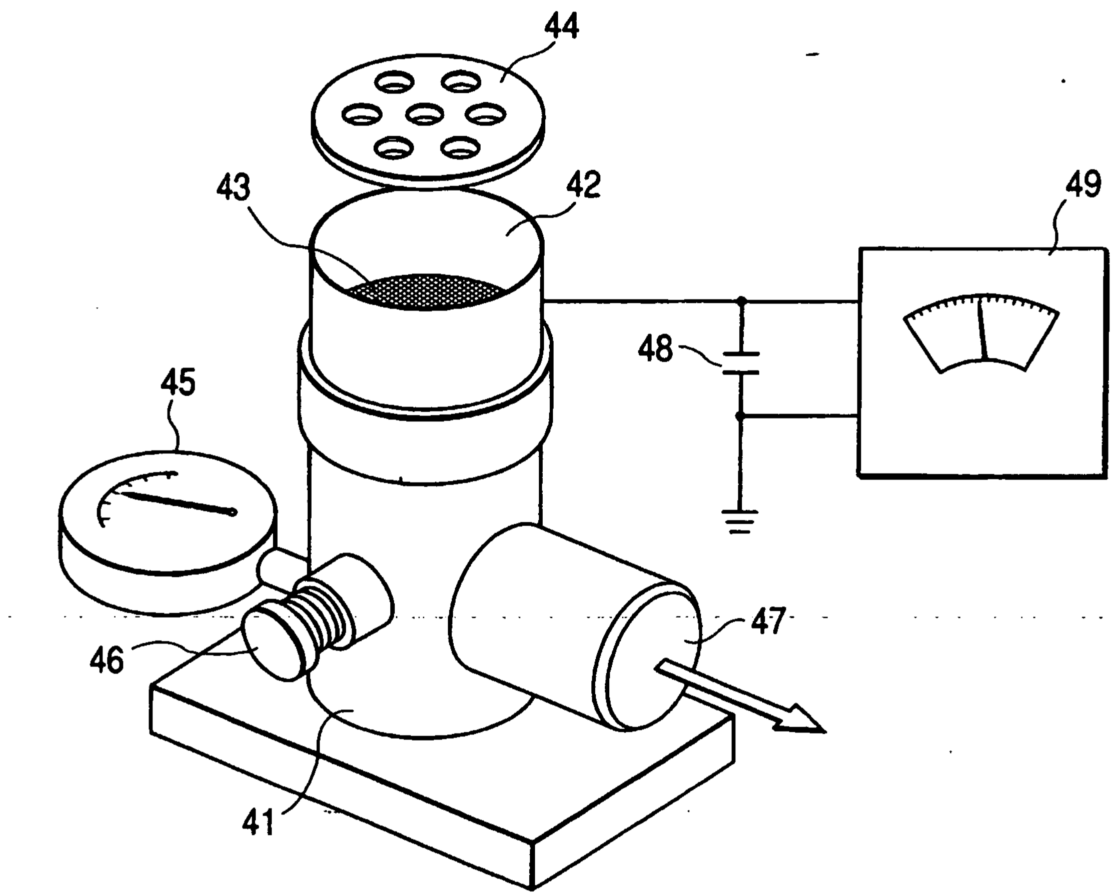

FIG. 8

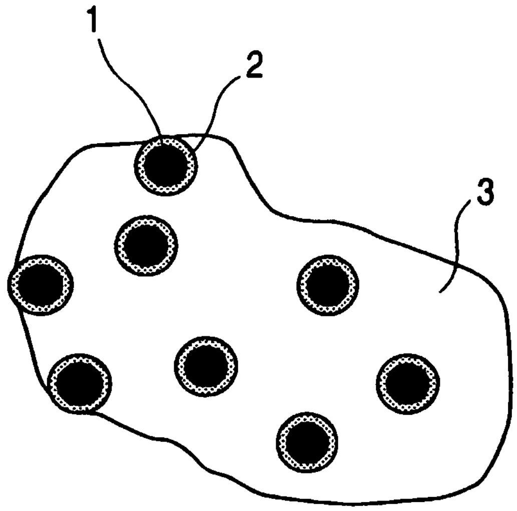


FIG. 9

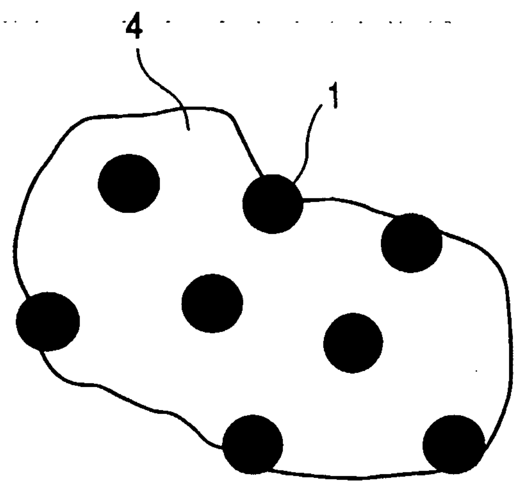

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 62017753 A
- JP 5088406 A
- JP 5289396 A
- JP 5341574 A
- JP 63305367 A
- JP 5074492 A
- JP 6015604 A
- JP 7014352 A
- JP 8019227 A
- JP 9191893 A
- JP 5093049 A
- JP 7265065 A
- JP 2642937 B
- JP 2989175 B
- US 5004664 A
- JP 2000078753 A
- JP 2001069968 A
- JP P2001133728 B
- JP P2001210021 B


### Non-patent literature cited in the description


- *Int. J. Biol. Macromol.,* 1994, vol. 16 (3), 119
- *Macromolecules,* 1991, vol. 24, 5256-5260
- *Macromol. Chem.,* 1990, vol. 191, 1957-1965
- *Chirality,* 1991, vol. 3, 492-494
- *Macromolecules,* 1996, vol. 29, 1762-1766
- *Macromolecules,* 1999, vol. 32, 2889-2895
- *Macromol. Chem. Phys.,* 1994, vol. 195, 1665-1672
- *Macromolecules,* 1996, vol. 29, 3432-3435
- *Can. J. Microbiol.,* 1995, vol. 41, 32-43
- *Macromolecules,* 1997, vol. 30, 1611-1615
- *Proc. Natl. Acad. Sci. USA,* 1995, vol. 92, 6279-6283
- *Int. J. Biol. Macromol.,* 1999, vol. 25, 55-60
- *Macromol. Rapid Commun.,* 2000, vol. 21, 77-84
- *FEMS Microbiol. Lett.,* 1998, vol. 168, 319-324
- *Appl. Microbiol. Biotechnol.,* 2000, vol. 54, 37-43
- *Eur. J. Biochem.,* 1997, vol. 250, 432-439
- *J. Biol. Chem.,* 1956, vol. 218, 97-106
- *J. Amer. Chem. Soc.,* 1956, vol. 78, 2278
- Molecular Cloning. Cold Spring Harbor Laboratory, 1989, vol. 1, 572
- Molecular Cloning. Cold Spring Harbor Laboratory, 1989, vol. 1, 5.7.2
- *J. Org. Chem.,* 1990, vol. 55, 1490-1492
- *Int. J. Biol. Macromol.,* 1990, vol. 12, 85-91

SEQUENCE LISTING

<110> CANON KABUSHIKI KAISHA

<120> Electrostatic charge image developing toner, producing method therefor, and image forming method and image forming apparatus utilizing the toner

<130> CF016375

<150> JP P2001-133728
<151> 2001-04-27

<150> JP P2001-210021
<151> 2001-07-10

<160> 15

<170> Microsoft Word

<210> 1
<211> 30
<212> DNA
<213> Artificial Sequence

<220>
<223> Primer for PCR multiplication

<400> 1

cgggatccag taacaagagt aacgatgagt 30

<210> 2
<211> 30
<212> DNA
<213> Artificial Sequence

<220>
<223> Primer for PCR multiplication

<400> 2

cgatctcgag ttaccgttcg tgcacgtacg 30

<210> 3
<211> 20
<212> DNA
<213> Artificial Sequence

<400> 3

tgctggaact gatccagtac 20

<210> 4
<211> 23
<212> DNA
<213> Artificial Sequence

<400> 4

gggttgagga tgctctggat gtg 23

<210> 5
<211> 1680
<212> DNA
<213> Pseudomonas cichorii YN2 ; FERM BP-7375

<400> 5

atgagtaaca agagtaacga tgagttgaag tatcaagcct ctgaaaacac 50
cttggggctt aatcctgtcg ttgggctgcg tggaaaggat ctactggctt 100
ctgctcgaat ggtgcttagg caggccatca agcaaccggt gcacagcgtc 150
aaacatgtcg cgcactttgg tcttgaactc aagaacgtac tgctgggtaa 200
atccgggctg caaccgacca gcgatgaccg tcgcttcgcc gatccggcct 250
ggagccagaa cccgctctat aaacgttatt tgcaaaccta cctggcgtgg 300
cgcaaggaac tccacgactg gatcgatgaa agtaacctcg cccccaagga 350
tgtggcgcgt gggcacttcg tgatcaacct catgaccgaa gccatggcgc 400
cgaccaacac cgcggccaac ccggcggcag tcaaacgctt tttcgaaacc 450
ggtggcaaaa gcctgctcga cggcctctcg cacctggcca aggatctggt 500
acacaacggc ggcatgccga gccaggtcaa catgggtgca ttcgaggtcg 550
gcaagagcct gggcgtgacc gaaggcgcgg tggtgtttcg caacgatgtg 600
ctggaactga tccagtacaa gccgaccacc gagcaggtat acgaacgccc 650
gctgctggtg gtgccgccgc agatcaacaa gttctacgtt ttcgacctga 700
gcccggacaa gagcctggcg cggttctgcc tgcgcaacaa cgtgcaaacg 750
ttcatcgtca gctggcgaaa tcccaccaag gaacagcgag agtggggcct 800
gtcgacctac atcgaagccc tcaaggaagc ggttgatgtc gttaccgcga 850
tcaccggcag caaagacgtg aacatgctcg gcgcctgctc cggcggcatc 900
acttgcaccg cgctgctggg ccattacgcg gcgattggcg aaaacaaggt 950
caacgccctg accttgctgg tgagcgtgct tgataccacc ctcgacagcg 1000
atgttgccct gttcgtcaat gaacagaccc ttgaagccgc caagcgccac 1050

tcgtaccagg ccggcgtact ggaaggccgc gacatggcga aggtcttcgc 1100
ctggatgcgc cccaacgatc tgatctggaa ctactgggtc aacaattacc 1150
tgctaggcaa cgaaccgccg gtgttcgaca tcctgttctg gaacaacgac 1200
accacacggt tgcccgcggc gttccacggc gacctgatcg aactgttcaa 1250
aaataaccca ctgattcgcc cgaatgcact ggaagtgtgc ggcacccccca 1300
tcgacctcaa gcaggtgacg gccgacatct tttccctggc cggcaccaac 1350
gaccacatca ccccgtggaa gtcctgctac aagtcggcgc aactgtttgg 1400
cggcaacgtt gaattcgtgc tgtcgagcag cgggcatatc cagagcatcc 1450
tgaacccgcc gggcaatccg aaatcgcgct acatgaccag caccgaagtg 1500
gcggaaaatg ccgatgaatg gcaagcgaat gccaccaagc ataccgattc 1550
ctggtggctg cactggcagg cctggcaggc ccaacgctcg ggcgagctga 1600
aaaagtcccc gacaaaactg ggcagcaagg cgtatccggc aggtgaagcg 1650
gcgccaggca cgtacgtgca cgaacggtaa 1680

<210> 6
<211> 1683
<212> DNA
<213> Pseudomonas cichorii YN2 ; FERM BP-7375

<400> 6

atgcgcgata aacctgcgag ggagtcacta cccacccccg ccaagttcat 50
caacgcacaa agtgcgatta ccggcctgcg tggccgggat ctggtttcga 100
ctttgcgcag tgtcgccgcc catggcctgc gccaccccgt gcacaccgcg 150
cgacacgcct tgaaactggg tggtcaactg ggacgcgtgt tgctgggcga 200
caccctgcat cccaccaacc cgcaagaccg tcgcttcgac gatccggcgt 250
ggagtctcaa tcccttttat cgtcgcagcc tgcaggcgta cctgagctgg 300
cagaagcagg tcaagagctg gatcgacgaa agcaacatga gcccggatga 350
ccgcgcccgt gcgcacttcg cgttcgccct gctcaacgat gccgtgtcgc 400
cgtccaacag cctgctcaat ccgctggcga tcaaggaaat cttcaactcc 450
ggcggcaaca gcctggtgcg cgggatcggc catctggtcg atgacctctt 500
gcacaacgat ggcttgcccc ggcaagtcac caggcatgca ttcgaggttg 550
gcaagaccgt cgccaccacc accggcgccg tggtgtttcg caacgagctg 600
ctggagctga tccaatacaa gccgatgagc gaaaagcagt attccaaacc 650
gctgctggtg gtgccgccac agatcaacaa gtactacatt tttgacctca 700
gcccccataa cagcttcgtc cagttcgcgc tcaagaacgg cctgcaaacc 750
ttcgtcatca gctggcgcaa tccggatgta cgtcaccgcg aatggggcct 800
gtcgacctac gtcgaagcgg tggaagaagc catgaatgtc tgccgggcaa 850
tcaccggcgc gcgcgaggtc aacctgatgg gcgcctgcgc tggcgggctg 900

accattgctg ccctgcaggg ccacttgcaa gccaagcgac agctgcgccg 950
cgtctccagc gcgacgtacc tggtgagcct gctcgacagc caactggaca 1000
gcccggccac actcttcgcc gacgaacaga ccctggaggc ggccaagcgc 1050
cgctcctacc agaaaggtgt gctggaaggc cgcgacatgg ccaaggtttt 1100
cgcctggatg cgccccaacg atttgatctg gagctacttc gtcaacaatt 1150
acctgatggg caaggagccg ccggcgttcg acattctcta ctggaacaat 1200
gacaacacac gcctgccggc cgccctgcat ggtgacttgc tggacttctt 1250
caagcacaac ccgctgagcc atccgggtgg cctggaagtg tgcggcaccc 1300
cgatcgactt gcaaaaggtc accgtcgaca gtttcagcgt ggccggcatc 1350
aacgatcaca tcacgccgtg ggacgcggtg tatcgctcaa ccctgttgct 1400
cggtggcgag cgtcgctttg tcctggccaa cagcggtcat gtgcagagca 1450
ttctcaaccc gccgaacaat ccgaaagcca actacctcga aggtgcaaaa 1500
ctaagcagcg accccagggc ctggtactac gacgccaagc ccgtcgacgg 1550
tagctggtgg acgcaatggc tgggctggat tcaggagcgc tcgggcgcgc 1600
aaaaagaaac ccacatggcc ctcggcaatc agaattatcc accgatggag 1650
gcggcgcccg ggacttacgt gcgcgtgcgc tga 1683

<210> 7
<211> 29
<212> DNA
<213> Artificial Sequence

<220>
<223> Primer for PCR multiplication

<400> 7

ggaccaagct tctcgtctca gggcaatgg 29

<210> 8
<211> 29
<212> DNA
<213> Artificial Sequence

<220>
<223> Primer for PCR multiplication

<400> 8

cgagcaagct tgctcctaca ggtgaaggc 29

<210> 9
<211> 29
<212> DNA
<213> Artificial Sequence

<220>

<223> Primer for PCR multiplication

<400> 9

gtattaagct tgaagacgaa ggagtgttg 29

<210> 10
<211> 30
<212> DNA
<213> Artificial Sequence

<220>
<223> Primer for PCR multiplication

<400> 10

catccaagct tcttatgatc gggtcatgcc 30

<210> 11
<211> 30
<212> DNA
<213> Artificial Sequence

<220>
<223> Primer for PCR multiplication

<400> 11

cgggatccag taacaagagt aacgatgagt 30

<210> 12
<211> 30
<212> DNA
<213> Artificial Sequence

<220>
<223> Primer for PCR multiplication

<400> 12

cgatctcgag ttaccgttcg tgcacgtacg 30

<210> 13 .
<211> 30
<212> DNA
<213> Artificial Sequence

<220>
<223> Primer for PCR multiplication

<400> 13

cgggatcccg cgataaacct gcgagggagt 30

<210> 14
<211> 30
<212> DNA
<213> Artificial Sequence

<220>
<223> Primer for PCR multiplication

<400> 14

cgatctcgag gcgcacgcgc acgtaagtcc 30

SEQUENCE LISTING

<110> CANON KABUSHIKI KAISHA
<120> Electrostatic charge image developing toner, producing method therefor, and image forming method and image forming apparatus utilizing the toner
<130> CF016375
<150> JP P2001-133728
<151> 2001-04-27
<150> JP P2001-210021
<151> 2001-07-10
<160> 15
<170> Microsoft Word
<210> 1
<211> 30
<212> DNA
<213> Artificial Sequence
<220>
<223> Primer for PCR multiplication
<400> 1

cgggatccag taacaagagt aacgatgagt 30

<210> 2
<211> 30
<212> DNA
<213> Artificial Sequence
<220>
<223> Primer for PCR multiplication
<400> 2

cgatctcgag ttaccgttcg tgcacgtacg 30

<210> 3
<211> 20
<212> DNA
<213> Artificial Sequence
<400> 3

tgctggaact gatccagtac 20

<210> 4
<211> 23
<212> DNA
<213> Artificial Sequence
<400> 4

gggttgagga tgctctggat gtg 23

<210> 5

<211> 1680
<212> DNA
<213> Pseudomonas cichorii YN2 ; FERM BP-7375
<400> 5

```
atgagtaaca agagtaacga tgagttgaag tatcaagcct ctgaaaacac   50
cttggggctt aatcctgtcg ttgggctgcg tggaaaggat ctactggctt  100
ctgctcgaat ggtgcttagg caggccatca agcaaccggt gcacagcgtc  150
aaacatgtcg cgcactttgg tcttgaactc aagaacgtac tgctgggtaa  200
atccgggctg caaccgacca gcgatgaccg tcgcttcgcc gatccggcct  250
ggagccagaa cccgctctat aaacgttatt tgcaaaccta cctggcgtgg  300
cgcaaggaac tccacgactg gatcgatgaa agtaacctcg cccccaagga  350
tgtggcgcgt gggcacttcg tgatcaacct catgaccgaa gccatggcgc  400
cgaccaacac cgcggccaac ccggcggcag tcaaacgctt tttcgaaacc  450
ggtggcaaaa gcctgctcga cggcctctcg cacctggcca aggatctggt  500
acacaacggc ggcatgccga gccaggtcaa catgggtgca ttcgaggtcg  550
gcaagagcct gggcgtgacc gaaggcgcgg tggtgtttcg caacgatgtg  600
ctggaactga tccagtacaa gccgaccacc gagcaggtat acgaacgccc  650
gctgctggtg gtgccgccgc agatcaacaa gttctacgtt ttcgacctga  700
gcccggacaa gagcctggcg cggttctgcc tgcgcaacaa cgtgcaaacg  750
ttcatcgtca gctggcgaaa tcccaccaag gaacagcgag agtggggcct  800
gtcgacctac atcgaagccc tcaaggaagc ggttgatgtc gttaccgcga  850
tcaccggcag caaagacgtg aacatgctcg gcgcctgctc cggcggcatc  900
acttgcaccg cgctgctggg ccattacgcg gcgattggcg aaaacaaggt  950
caacgccctg accttgctgg tgagcgtgct tgataccacc ctcgacagcg 1000
atgttgccct gttcgtcaat gaacagaccc ttgaagccgc caagcgccac 1050
```

tcgtaccagg ccggcgtact ggaaggccgc gacatggcga aggtcttcgc 1100
ctggatgcgc cccaacgatc tgatctggaa ctactgggtc aacaattacc 1150
tgctaggcaa cgaaccgccg gtgttcgaca tcctgttctg gaacaacgac 1200
accacacggt tgcccgcggc gttccacggc gacctgatcg aactgttcaa 1250
aaataaccca ctgattcgcc cgaatgcact ggaagtgtgc ggcacccccca 1300
tcgacctcaa gcaggtgacg gccgacatct ttccctggc cggcaccaac 1350
gaccacatca ccccgtggaa gtcctgctac aagtcggcgc aactgtttgg 1400
cggcaacgtt gaattcgtgc tgtcgagcag cgggcatatc cagagcatcc 1450
tgaacccgcc gggcaatccg aaatcgcgct acatgaccag caccgaagtg 1500
gcggaaaatg ccgatgaatg gcaagcgaat gccaccaagc ataccgattc 1550
ctggtggctg cactggcagg cctggcaggc ccaacgctcg ggcgagctga 1600
aaaagtcccc gacaaaactg ggcagcaagg cgtatccggc aggtgaagcg 1650
gcgccaggca cgtacgtgca cgaacggtaa 1680

<210> 6
<211> 1683
<212> DNA
<213> Pseudomonas cichorii YN2 ; FERM BP-7375
<400> 6

atgcgcgata aacctgcgag ggagtcacta cccacccccg ccaagttcat 50
caacgcacaa agtgcgatta ccggcctgcg tggccgggat ctggtttcga 100
ctttgcgcag tgtcgccgcc catggcctgc gccaccccgt gcacaccgcg 150
cgacacgcct tgaaactggg tggtcaactg ggacgcgtgt tgctgggcga 200
caccctgcat cccaccaacc cgcaagaccg tcgcttcgac gatccggcgt 250
ggagtctcaa tcccttttat cgtcgcagcc tgcaggcgta cctgagctgg 300
cagaagcagg tcaagagctg gatcgacgaa agcaacatga gcccggatga 350
ccgcgcccgt gcgcacttcg cgttcgccct gctcaacgat gccgtgtcgc 400
cgtccaacag cctgctcaat ccgctggcga tcaaggaaat cttcaactcc 450
ggcggcaaca gcctggtgcg cgggatcggc catctggtcg atgacctctt 500
gcacaacgat ggcttgcccc ggcaagtcac caggcatgca ttcgaggttg 550
gcaagaccgt cgccaccacc accggcgccg tggtgtttcg caacgagctg 600
ctggagctga tccaatacaa gccgatgagc gaaaagcagt attccaaacc 650
gctgctggtg gtgccgccac agatcaacaa gtactacatt tttgacctca 700
gcccccataa cagcttcgtc cagttcgcgc tcaagaacgg cctgcaaacc 750
ttcgtcatca gctggcgcaa tccggatgta cgtcaccgcg aatggggcct 800
gtcgacctac gtcgaagcgg tggaagaagc catgaatgtc tgccgggcaa 850
tcaccggcgc gcgcgaggtc aacctgatgg gcgcctgcgc tggcgggctg 900

accattgctg ccctgcaggg ccacttgcaa gccaagcgac agctgcgccg 950
cgtctccagc gcgacgtacc tggtgagcct gctcgacagc caactggaca 1000
gcccggccac actcttcgcc gacgaacaga ccctggaggc ggccaagcgc 1050
cgctcctacc agaaaggtgt gctggaaggc cgcgacatgg ccaaggtttt 1100
cgcctggatg cgccccaacg atttgatctg gagctacttc gtcaacaatt 1150
acctgatggg caaggagccg ccggcgttcg acattctcta ctggaacaat 1200
gacaacacac gcctgccggc cgccctgcat ggtgacttgc tggacttctt 1250
caagcacaac ccgctgagcc atccgggtgg cctggaagtg tgcggcaccc 1300
cgatcgactt gcaaaaggtc accgtcgaca gtttcagcgt ggccggcatc 1350
aacgatcaca tcacgccgtg ggacgcggtg tatcgctcaa ccctgttgct 1400
cggtggcgag cgtcgctttg tcctggccaa cagcggtcat gtgcagagca 1450
ttctcaaccc gccgaacaat ccgaaagcca actacctcga aggtgcaaaa 1500
ctaagcagcg accccagggc ctggtactac gacgccaagc ccgtcgacgg 1550
tagctggtgg acgcaatggc tgggctggat tcaggagcgc tcgggcgcgc 1600
aaaaagaaac ccacatggcc ctcggcaatc agaattatcc accgatggag 1650
gcggcgcccg ggacttacgt gcgcgtgcgc tga 1683

<210> 7
<211> 29
<212> DNA
<213> Artificial Sequence
<220>
<223> Primer for PCR multiplication
<400> 7

ggaccaagct tctcgtctca gggcaatgg 29

<210> 8
<211> 29
<212> DNA
<213> Artificial Sequence
<220>
<223> Primer for PCR multiplication
<400> 8

cgagcaagct tgctcctaca ggtgaaggc 29

<210> 9
<211> 29
<212> DNA
<213> Artificial Sequence
<220>
<223> Primer for PCR multiplication
<400> 9

gtattaagct tgaagacgaa ggagtgttg 29

<210> 10

<211> 30
<212> DNA
<213> Artificial Sequence
<220>
<223> Primer for PCR multiplication
<400> 10

catccaagct tcttatgatc gggtcatgcc 30

<210> 11
<211> 30
<212> DNA
<213> Artificial Sequence
<220>
<223> Primer for PCR multiplication
<400> 11

cgggatccag taacaagagt aacgatgagt 30

<210> 12
<211> 30
<212> DNA
<213> Artificial Sequence
<220>
<223> Primer for PCR multiplication
<400> 12

cgatctcgag ttaccgttcg tgcacgtacg 30

<210> 13
<211> 30
<212> DNA
<213> Artificial Sequence
<220>
<223> Primer for PCR multiplication
<400> 13

cgggatcccg cgataaacct gcgagggagt 30

<210> 14
<211> 30
<212> DNA
<213> Artificial Sequence
<220>
<223> Primer for PCR multiplication
<400> 14

cgatctcgag gcgcacgcgc acgtaagtcc 30